(19) Europäisches Patentamt
European Patent Office
Office européen des brevets

(11) EP 1 599 433 B1

# (12) EUROPÄISCHE PATENTSCHRIFT

(45) Veröffentlichungstag und Bekanntmachung des Hinweises auf die Patenterteilung:
**06.12.2006 Patentblatt 2006/49**

(21) Anmeldenummer: **04712518.2**

(22) Anmeldetag: **19.02.2004**

(51) Int Cl.:
*C07C 29/00* (2006.01) *C07D 263/32* (2006.01)

(86) Internationale Anmeldenummer:
**PCT/EP2004/001580**

(87) Internationale Veröffentlichungsnummer:
**WO 2004/076390 (10.09.2004 Gazette 2004/37)**

(54) **VERFAHREN ZUR HERSTELLUNG DER ENANTIOMEREN FORMEN VON CIS-KONFIGURIERTEN 1,3-CYCLOHEXANDIOL-DERIVATEN**

METHOD FOR PRODUCING THE ENANTIOMERIC FORMS OF CIS 1,3-CYCLOHEXANEDIOL DERIVATIVES

PROCEDE POUR REALISER LES FORMES ENANTIOMERES DE DERIVES DE CIS 1,3-CYCLOHEXANDIOL

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**LT LV MK**

(30) Priorität: **27.02.2003 DE 10308350**

(43) Veröffentlichungstag der Anmeldung:
**30.11.2005 Patentblatt 2005/48**

(73) Patentinhaber: **Sanofi-Aventis Deutschland GmbH**
**65929 Frankfurt am Main (DE)**

(72) Erfinder:
- **HOLLA, Wolfgang**
  **65779 Kelkheim (DE)**
- **KEIL, Stefanie**
  **65719 Hofheim (DE)**

(56) Entgegenhaltungen:
**WO-A1-03/020269**

- **LEVY L M ET AL: "Lipase-catalysed resolution of cyclic cis- and trans-beta-hydroxy esters" TETRAHEDRON: ASYMMETRY, ELSEVIER SCIENCE PUBLISHERS, AMSTERDAM, NL, Bd. 14, Nr. 14, 18. Juli 2003 (2003-07-18), Seiten 2053-2058, XP004435220 ISSN: 0957-4166**
- **SUEMUNE H ET AL: "ENANTIOSELECTIVE SYNTHESIS OF (1S,3S,5R)-1-ACETOXY-5-BENZYLOXYCYCLOHE XAN-3-OL AND ITS APPLICATION TO THE SYNTHESIS OF COMPACTIN LACTONE MOIETY AND QUINIC ACID" TETRAHEDRON: ASYMMETRY, ELSEVIER SCIENCE PUBLISHERS, AMSTERDAM, NL, Bd. 3, Nr. 2, 1992, Seiten 297-306, XP001147874 ISSN: 0957-4166 in der Anmeldung erwähnt**

EP 1 599 433 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft ein Verfahren zur Herstellung chiraler, nicht-racemischer, ciskonfigurierter 1,3-disubstituierter Cyclohexanole der Formel (I)

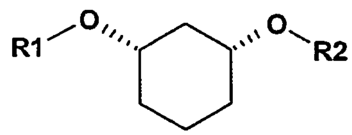

(I)

Unterschiedlich substituierte cis-konfigurierte 1,3-disubstituierte Cyclohexan-Derivate (Verbindungen der Formel (I) mit $R^1 \neq R^2$,) sind zentrale Bausteine bzw. Vorstufen der in der WO 03/020269 beschriebenen Arzneimittelwirkstoffe, die sich generell zur Therapie von Lipidstoffwechselstörungen, von Typ II Diabetes und von Syndrom X u.a. eignen.
Die in der Patentanmeldung 03/020269 beschriebenen Synthesen der nichtracemischen, cis-konfigurierten 1,3-Cyclohexan-Derivate kommen als technische Verfahren nicht in Betracht: So sind beispielsweise Alkylierungen mit NaH/DMF im multi-kg-Maßstab nicht sicher durchführbar (C&EN, September 13, 1982,5). Weiterhin erfordert die Alkylierung nach der $Bu_2SnO$-Methode im Technikums-Maßstab einen unakzeptabel hohen Aufwand; die Abtrennung der Zinn-Verbindungen aus dem gewünschten Produkt ist auch bei Anwendung chromatographischer Trennmethoden sehr schwierig und meist nicht vollständig. Die Entsorgung der Zinn-Verbindungen ist ein weiteres Problem bzw. ein Kostenfaktor. Die Trennung der Enantiomeren (Racematspaltung) per Chromatographie an chiraler Phase ist ebenfalls aufwendig und zu teuer. Darüber hinaus ist es für die chromatographische Enantiomerentrennung erforderlich, dass die racemische Verbindung in guter chemischer Reinheit vorliegt, was sich vielfach nur durch eine zusätzliche, vorgeschaltete Chromatographie erreichen lässt.
Andere, in der Literatur beschriebene, Methoden der Synthese von cis-1,3-Cyclohexandiolbausteinen bzw. Derivaten, wie z.B. die Öffnung von Epoxycyclohexanen (P. Crotti, V. Di Bussolo, L. Favero, M. Pineschi, F. Marianucci, G. Renzi, G. Amici, G. Roselli, Tetrahedron 2000, 56, 7513-7524 und zit. Lit.) oder die Metall-katalysierte Hydroborierung von Cyclohexen-Derivaten (J. A. Brinkmann, T. T. Nguyen, J. R. Sowa, Jr., Org. Lett. **2000**, 2, 981-983; C. E. Garrett, G. C. Fu, J. Org. Chem. 1998, 63, 1370-1371) sind bezüglich der Regio- und der Stereoselektivität überwiegend unbefriedigend. Die Gesamtstufenzahl ist darüberhinaus deutlich höher. Als technische Prozesse kommen sie daher nicht in Frage.
Die Synthese von cis-1,3-Cyclohexandiolderivaten ausgehend von cis, cis-1,3,5-Cyclohexantriol bzw. cis, cis-1,3,5-Cyclohexantriol-Derivaten (L. Dumortier. M. Carda, J. Van der Eycken, G. Snatzke, M. Vandewalle, Tetrahedron: Asymmetry 1991, 2, 789-792; H. Suemune, K. Matsuno, M. Uchida, K. Sakai, Tetrahedron: Asymmetry 1992, 3, 297-306) sind ebenfalls aufgrund der hohen Stufenzahl sehr aufwendig und unökonomisch und daher für eine techniche Anwendung ungeeignet.
Auch die enzymatische Umsetzung des cis/trans-Gemisches von 1,3-Cyclohexandiol mit S-Ethyl-thiooctanoat kommt als technischer Prozess nicht in Betracht. Abgesehen von der kaum vermeidbaren Geruchsbelästigung beim Umgang mit den Schwefelverbindungen und der Tatsache, dass zur Erzielung des erforderlichen Umsatzes das freiwerdende Ethanthiol kontinuierlich entfernt werden muss, führt die beschriebene Reaktion zu einer Mischung von 9 isomeren Formen bzw. Derivaten von Cyclohexandiol, nämlich den nicht umgesetzten Isomeren (S,S)-Diol, (R,R)-Diol und (R, S)-Diol, weiterhin den monoacylierten Produkten (S,S)-Monooctanoat, (R,R)-Monooctanoat und (R,S)-Monooctanoat, und drittens der Gruppe der diacylierten Produkte (S,S)-Dioctanoat, (R,R)- Dioctanoat und (R,S)- Dioctanoat. Das optisch aktive, monoacylierte, cis-konfigurierte (R,S)-Monooctanoat nimmt in der Fraktion der monoacylierten Cyclohexandiole nur einen Anteil von etwa 12 % ein. Eine im präparativen Maßstab durchführbare Herstellung und Isolierung dieses Produktes ist nicht beschrieben, kann aber angesichts der Mengenverhältnisse und des geschilderten Trennproblems nicht ökonomisch sein. Darüber hinaus ist bekannt, dass partiell acylierte Di- bzw. Polyhydroxyverbindungen zu Acylgruppen-Wanderungen neigen. Tritt dieser Fall z. B. im Verlauf der Reinigung des (R,S)-Monooctanoats (z. B. bei der Chromatographie an Kieselgel oder bei wäßrigen Extraktionen) oder im Verlauf einer sich anschließenden Reaktion (z. B. während der Alkylierung der freien Hydroxygruppe) ein, so führt dies zu einer deutlichen Verminderung der optischen Reinheit bzw. zur Racemisierung.
Die cis-konfigurierten (R,S)-Diole bzw. die diacylierten (R,S)-Verbindungen sind optisch nicht aktiv und daher nicht von Interesse.
Ziel der vorliegenden Erfindung war es daher, ein Verfahren zu entwickeln, dass die genannten Nachteile nicht aufweist.
Gegenstand der vorliegenden Erfindung ist ein Verfahren zur Herstellung chiraler, nicht racemischer Verbindungen der Formel I

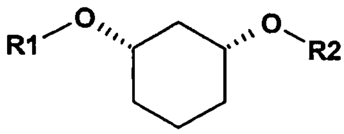

(I) .

mit:

$R^1$

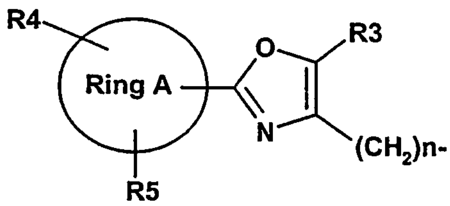

worin bedeuten:

Ring A Phenyl, 5-12 gliedriger heteroaromatischer Ring, der ein bis vier Heteroatome aus der Gruppe N, O oder S enthalten kann, 8 bis 14 gliedriger aromatischer Ring, $(C_3-C_8)$-Cycloalkyl;

$R^3$ H, F, Cl, Br, OH, $NO_2$, $CF_3$, $OCF_3$, $(C_1-C_6)$-Alkyl, $(C_3-C_8)$-Cycloalkyl, Phenyl;

$R^4$, $R^5$ H, F, Cl, Br, OH, $NO_2$, $CF_3$, $OCF_3$, $OCF_2H$, $OCF_2-CF_3$, $OCF_2-CHF_2$, $SCF_3$, O-Phenyl, $(C_1-C_6)$-Alkyl, O-$(C_1-C_6)$-Alkyl, O-$(C_1-C_6)$-Alkyl-O-$(C_1-C_3)$-Alkyl;

n 1 bis 3;

und

$R^2$ $(C_1-C_8)$-Alkyl, wobei in den Alkylgruppen ein oder mehrere $CH_2$-Gruppen durch O, CO, S, SO oder $SO_2$ ersetzt sein können, und Alkyl ein bis dreifach substituiert sein kann durch F, Cl, Br, $CF_3$, CN, $NO_2$, NHAc, NHBoc, NH-CO-$C(CH_3)_3$, Hydroxyl, $OCF_3$, O-$(C_1-C_6)$-Alkyl, COOH, CO-Benzoxy, CO-O$(C_1-C_6)$-Alkyl, Tetrazol, Thiazolidin-2,4-dion, Indol und $(C_6-C_{10})$-Aryl, wobei Thiazolidin-2,4-dion und Aryl wiederum durch F, Cl, Br, $CF_3$, CN, $NO_2$, NHAc, NHTs, NHBoc, NHCbz, NH-CO-$C(CH_3)_3$, Hydroxyl, $OCF_3$, O-$(C_1-C_6)$-Alkyl, COOH, CO-Benzoxy, CO-O $(C_1-C_6)$-Alkyl, $(C_1-C_6)$-Alkyl, O-$(C_1-C_6)$-Alkyl oder Tetrazol substituiert sein können, oder;

$R^2$ eine OH-Schutzgruppe (SG), wie z.B. Benzyloxymethyl, Benzyl, para-Methoxybenzyl oder tert.-Butyl-dimethylsilyl;

dadurch gekennzeichnet, dass man

**A)**

a) Alkylierung (Alk-$R^2$ / Alk-SG)
cis-1,3-Cyclohexandiol der Formel (II)

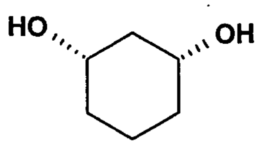

(II)

mit einer Verbindung der Formel (III)

$X^1$-$R^2$ (III)

worin $R^2$ wie oben definiert ist und

$X^1$ für Cl, Br, I, OMs (O-Mesyl), OTs (O-Tosyl), OTf (O-Triflat) steht;

in Gegenwart von Basen in einem geeigneten Lösungsmittel umsetzt zu einer racemischen Verbindung der Formel (IV),

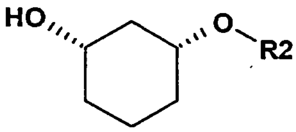

(IV)

worin $R^2$ wie oben definiert ist,

b1) Enzymatische Esterbildung (EB) + Trennung (T)
die erhaltenen Verbindungen der Formel (IV) einer stereoselektiven enzymatischen Esterbildung (EB) unterwirft, wobei die Alkohole in einem organischen Lösungsmitteln wie z. B. Dichlormethan mit einem Acyldonor, wie z.B. einem Vinylester $R^6$-O-CH=CH2 oder einem Säureanhydrid $R^6$-O-$R^6$, worin $R^6$ wie oben definiert ist, und dem Enzym versetzt und die resultierende Mischung bei -20 bis 80 °C gerührt werden und nach Ablauf der Reaktion das eine Stereoisomere als Ester der Formel (V)

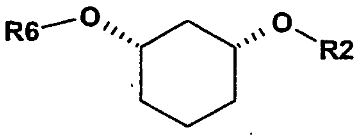

(V)

worin

$R^6$ C(=O)-($C_1$-$C_{16}$)-Alkyl, C(=O)-($C_2$-$C_{16}$)-Alkenyl, C(=O)-($C_3$-$C_{16}$)-Alkinyl, C(=O)-($C_3$-$C_{16}$)-Cycloalkyl, wobei ein oder mehrere Kohlenstoffatome durch Sauerstoffatome ersetzt sein und substituiert sein können mit 1-3 Substituenten aus der Gruppe F, Cl, Br, $CF_3$, CN, $NO_2$, Hydroxy, Methoxy, Ethoxy, Phenyl und CO-O ($C_1$-$C_4$)-Alkyl, CO-O($C_2$-$C_4$)-Alkenyl, die wiederum substituiert sein können mit 1-3 Substituenten aus der Gruppe F, Cl, Br, $CF_3$ bedeutet, und

$R^2$ wie oben definiert ist,

und das andere Stereoisomere unverändert als Alkohol der Formel (IV) vorliegt, und daher durch Ausnutzung ihrer unterschiedlichen chemischen bzw. physikochemischen Eigenschaften (z. B. $R_f$-Werte oder Löslichkeitsunterschiede in Wasser oder anderen Lösungsmitteln) voneinander trennen lassen (Trennung T), z. B. durch einfache Chromatographie an Kieselgel, durch Extraktion (z. B . Heptan / Methanol oder org. Lösungsmittel/Wasser) oder aber durch eine weiterführende chemische Folgereaktion z. B. des Alkohols, an der der Ester nicht teilnimmt,
oder

b2) Enzymatische Esterspaltung [= Chemische Veresterung (CV) + Enzymatische Spaltung (ES)] + Trennung (T)
die erhaltenen Verbindungen der Formel (IV) einer stereoselektiven enzymatischen Esterspaltung unterwirft, wobei die racemischen Alkohole zunächst durch chemische Veresterung (CV), z. B. mittels Säurechloriden $R^6$-Cl oder Säureanhydriden $R^6$-O-$R^6$ in Gegenwart von Basen wie z. B. Triethylamin in die racemischen Ester der Formel (V)

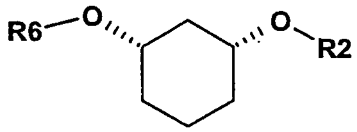

(V)

worin $R^6$ und $R^2$ wie oben definiert sind,
überführt werden, die dann zur Durchführung der stereoselektiven enzymatischen Esterspaltung (ES) in homogenen oder heterogenen, wässrigen, wässrig-organischen oder organischen Medien aufgenommen und in Gegenwart eines Enzyms im Falle der Hydrolyse mit Wasser und im Falle der Alkoholyse mit einem Alkohol wie z. B. n-Butanol bei einer Temperatur von 10 - 80 °C zur Reaktion gebracht werden, nach deren Ablauf das eine Stereoisomere als Alkohol der Formel (IV) und das andere unverändert als Ester der Formel (V) vorliegt und somit wie unter b1) beschreiben voneinander getrennt werden können, wobei
die als Alkohol anfallenden Enantiomere der Formel (IV) wie unter d) beschrieben weiter verarbeitet werden, oder

c) Chemische Hydrolyse (CH)
die als Ester anfallenden Enantiomere der Formel (V) zu chemisch enantiomeren Alkoholen nach bekannten Verfahren verseift und

d) Alkylierung (Alk-$R^1$)
weiter mit Verbindungen der Formel (VI)

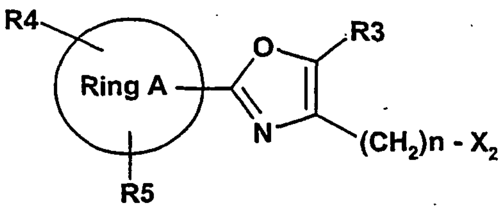

(VI)

worin
Ring A, $R^3$, $R^4$, $R^5$ und n wie oben definiert sind und

$x^2$ Cl, Br, I, OTs, OMs, OTf bedeutet;

in Gegenwart von Basen in einem geeigneten Lösungsmittel zu den Verbindungen der Formel (I) umgesetzt werden, und

e) Abspaltung der Schutzgruppe SG (AbSG)
falls $R^2$ für eine OH-Schutzgruppe (SG) steht wie oben und $R^2$ definiert, die Verbindungen der Formel (Ia)

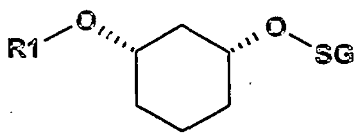

(Ia)

worin $R^1$ und SG wie oben definiert sind,
durch Abspaltung der Schutzgruppe nach bekannten Verfahren, wie z. B. Abspalten von SG = Benzyloxymethyl oder SG = Benzyl durch Hydrierung an Pd/C, oder Abspalten von SG = para-Methoxybenzyl mit beispielsweise DDQ (2,3-Dichloro-5,6-dicyanobenzoquinon), oder Abspalten von SG = tert.-Butyl-dimethylsilyl z. B. mit $Bu_4NF$, in Verbindungen der Formel (VII)

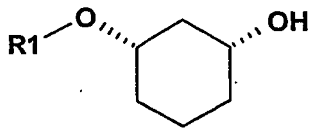

(VII)

worin $R^1$ wie oben definiert ist, überführt,

f) Alkylierung (Alk-$R^2$)
die anschließend mit Verbindungen der Formel (III),

$X^1$-$R^2$ (III)

worin X1 und $R^2$ wie oben definiert sind,
in Gegenwart von Basen in einem geeigneten Lösungsmittel zu Verbindungen der Formel (I), dem Produkt bzw. der enantiomeren Form, umgesetzt werden,
wobei es auch möglich ist die Reihenfolge der einzelnen Reaktionsschritte wie vorstehend unter A) beschrieben:

**A)** Alk-$R^2$ → EB+T / CV + ES + T [→ CH] → Alk-$R^1$ [→ AbSG → Alk-$R^2$] → Produkt/enantiomere Form
zu ändern in:
**B)** Alk-$R^1$ → EB+T / CV+ES+T [→ CH] → Alk-$R^2$ [→ AbSG → Alk-$R^2$] → Produkt/enantiomere Form
oder
**C)** Alk-SG → EB+T / CV+ES+T → CH → Alk-$R^2$ → AbSG → Alk-$R^1$ → Produkt/enantiomere Form
oder
**D)** Alk-SG → EB+T / CV+ES+T → Alk-$R^1$ → AbSG → Alk-$R^2$ → Produkt/enantiomere Form.

Nachfolgend sind mögliche Verfahrensvarianten in den Schemata I bis IV dargestellt:

Schema I

a)

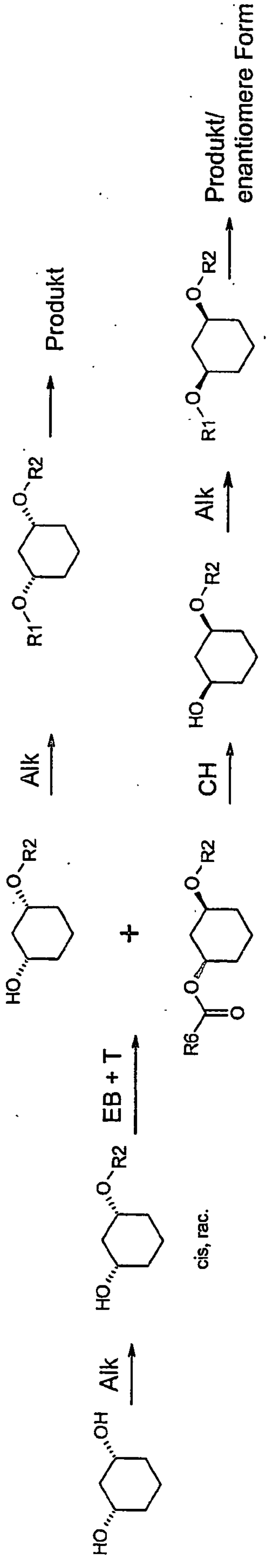
Alk
cis, rac.
EB + T
Alk
CH
Produkt
Alk
Produkt/
enantiomere Form

b)

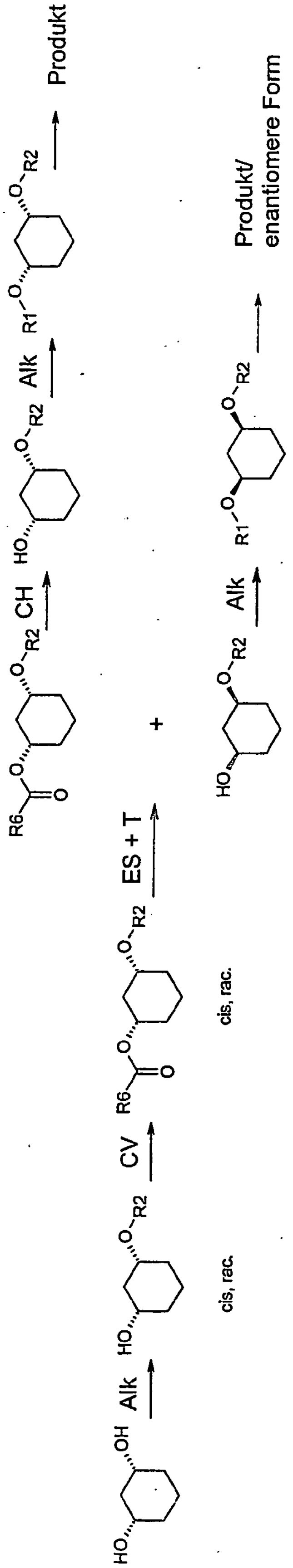
Alk
cis, rac.
CV
cis, rac.
ES + T
CH
Alk
Produkt
Alk
Produkt/
enantiomere Form

Schema II

a)

b)

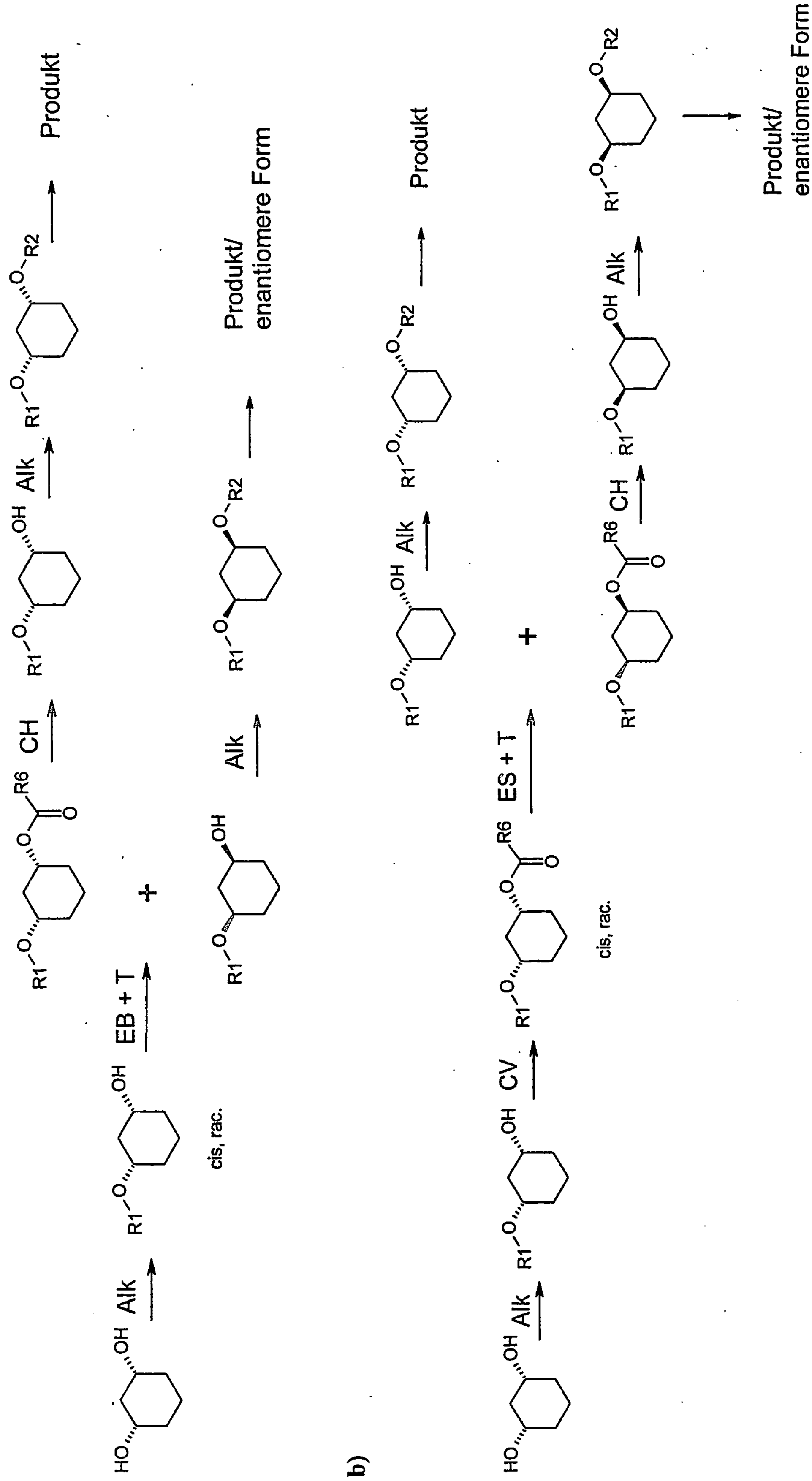
Alk
EB + T
cis, rac.
CH
Alk
Alk
Produkt
Produkt/
enantiomere Form
Alk
CV
ES + T
cis, rac.
Alk
Produkt
CH
Alk
Produkt/
enantiomere Form

Schema III

a)

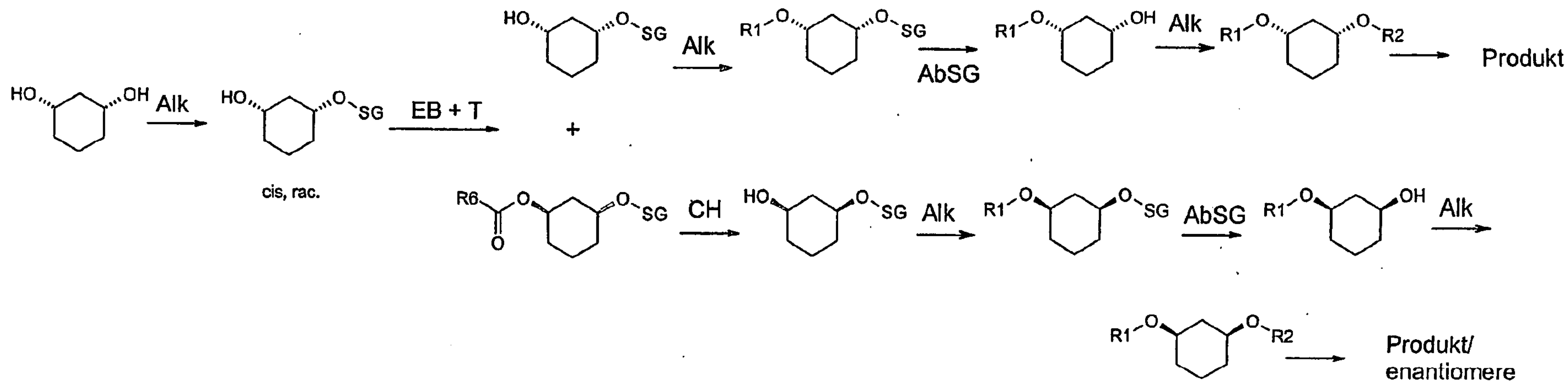
Alk
cis, rac.
EB + T
+
Alk
AbSG
Alk
Produkt
CH
Alk
AbSG
Alk
Produkt/
enantiomere

Schema III

b)

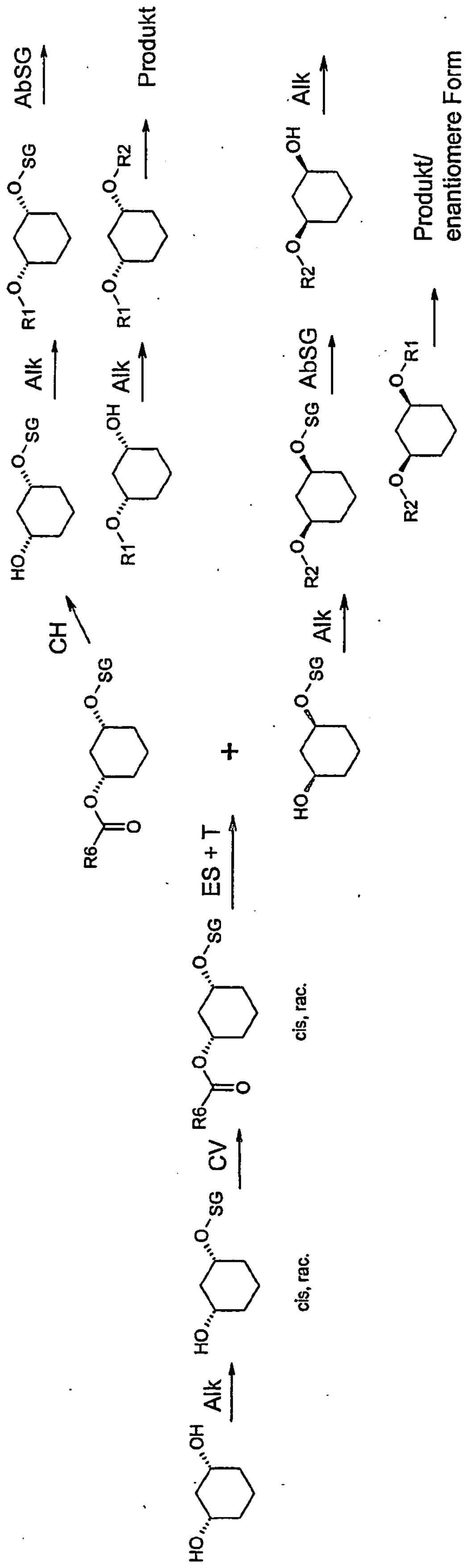
Alk
cis, rac.
CV
cis, rac.
ES + T
CH
Alk
Alk
AbSG
Alk
Produkt
Alk
AbSG
Alk
Produkt/
enantiomere Form

Schema IV

a)

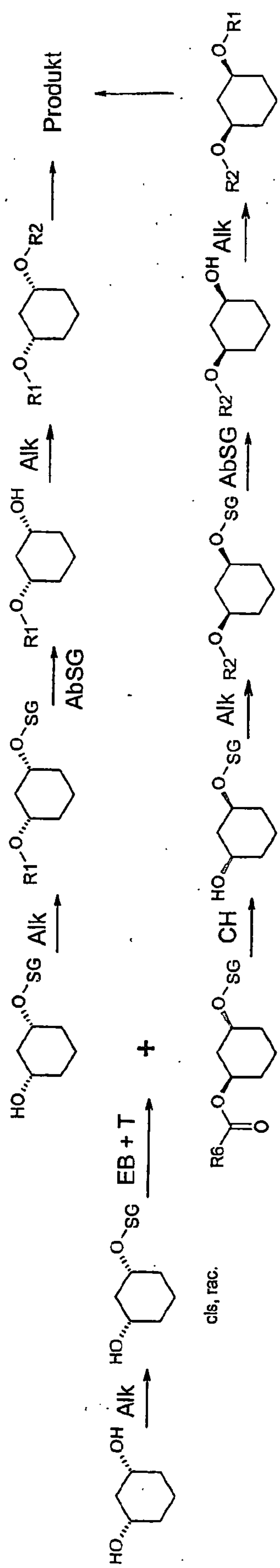

Schema IV

b)

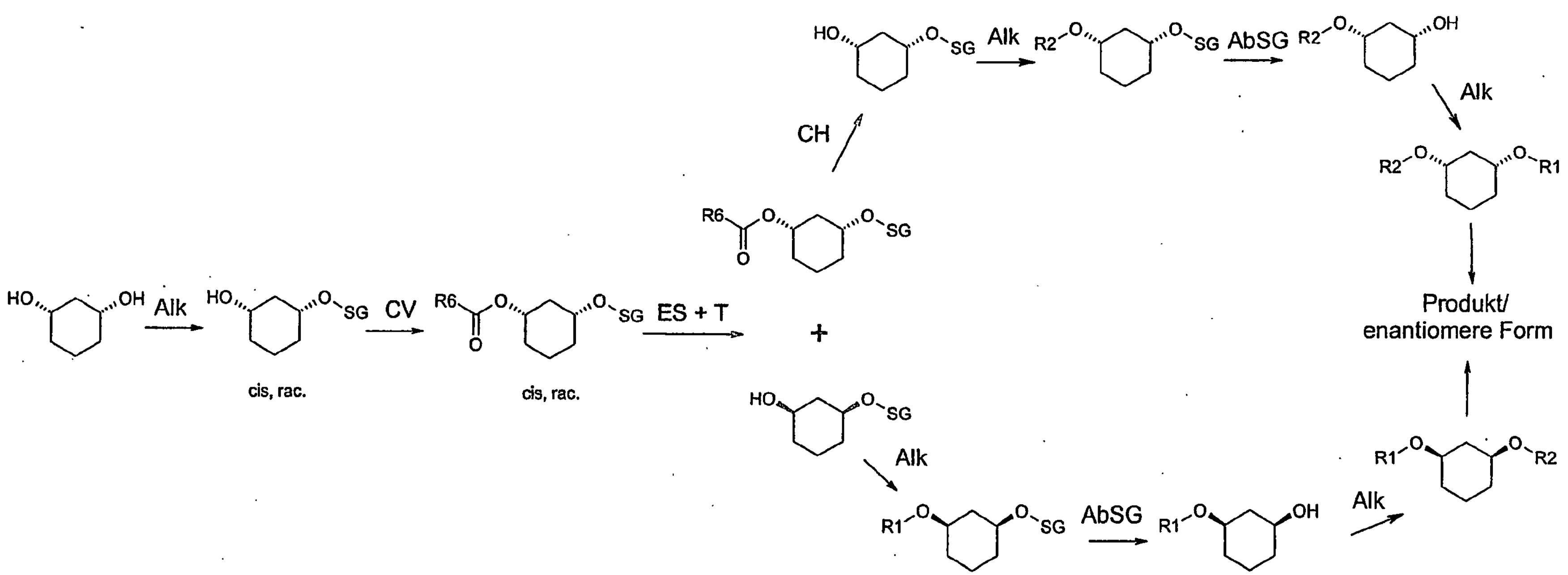
Alk
cis, rac.
CV
cis, rac.
ES + T
+
CH
Alk
AbSG
Alk
Alk
AbSG
Alk
Produkt/
enantiomere Form

Das erfindungsgemäße Verfahren ist ökonomisch, einfach und schnell. Das Verfahren vermeidet das Risiko der Acylgruppenwanderung vollständig, es erfordert keine äquimolaren Mengen optisch reiner Ausgangs- oder Hilfsstoffe, keine teuren Reagenzien, keine Racematspaltung durch Chromatographie an chiralen Phasen, keine unverhältnismäßig großen Lösungsmittelmengen und keine kostenintensiven Arbeitsschritte.

Der für Racematspaltüngen typische Verlust von 50 % kann durch Verwendung beider Enantiomerer und Änderung der Reihenfolge der Alkylierungen vermieden werden. Bevorzugt ist das sogenannte enantiokonvergente Verfahren (siehe Schema IV oder Verfahren C) und D)), bei dem man z. B. wie folgt vorgeht: Alkylierung von cis-1,3-Cyclohexandiol der Formel (II) mit einer Verbindung der Formel (III), wobei $R^2$ als SG so gewählt wird, dass SG im Verlauf der späteren Synthese wieder leicht und selektiv abgespalten werden kann, SG ist also z. B. Benzyl, oder para-Methoxybenzyl oder tert.-Butyl-dimethylsilyl, die erhaltenen Verbindung der Formel (IV) einer stereoselektiven enzymatischen Esterbildung oder Esterspaltung (s. o.) unterwirft und nach der erfolgten Trennung von nicht umgesetztem Alkohol und Ester beide getrennt und auf verschiedenen Wegen in das gleiche optische reine Produkt überführt, indem man den Alkohol (wie im ersten Teil beschrieben) z. B. mit einer Verbindung der Formel (VI) zu einer Verbindung der Formel (Ia) umsetzt, diese dann durch Abspaltung der Gruppe SG zu einer Verbindung der Formel (VII) umsetzt, und diese dann mit einer Verbindung der Formel (III), mit $R^2$ wie im Produkt gewünscht, zu einer Verbindung der Formel (I) umsetzt, dagegen den isomeren Ester durch einfache Esterspaltung in eine Verbindung der Formel (IV) überführt, und diese dann erst mit einer Verbindung der Formel (III), mit $R^2$ wie im Produkt gewünscht, zu einer Verbindung der Formel (VIII) umsetzt,

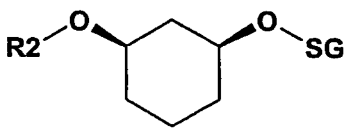

**(VIII)**

diese dann durch Abspaltung der Gruppe SG zu einer Verbindung der Formel (IV)

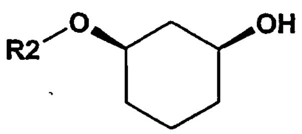

=

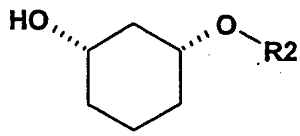

**(IV)**

umsetzt, und diese dann mit einer Verbindung der Formel (VI), zu einer Verbindung der Formel (I) umsetzt.

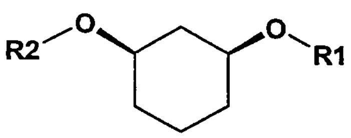

=

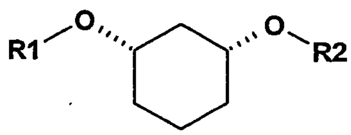

**(I)**

Bevorzugt werden Verbindungen der Formel (III)

$X^1$-$R^2$ (III)

verwendet, worin

$X^1$ Cl, Br, I, OMs oder OTs bedeutet,

besonders bevorzugt solche, mit

$x^1$ Cl, Br oder I.

Bevorzugt ist ein Verfahren zur Herstellung der Verbindungen der Formel (I),

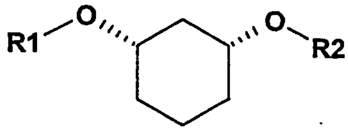

(I)

worin stehen:

$R^1$ für

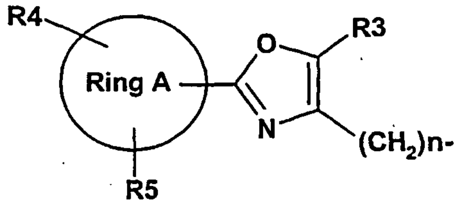

mit

Ring A Phenyl, 5-12 gliedriger heteroaromatischer Ring, der ein oder mehrere Heteroatome aus der Gruppe N, O oder S enthalten kann, annellierter/bicyclischer 8 bis 14 gliedriger aromatischer Ring, $(C_3-C_8)$-Cycloalkyl;

$R^3$ H, $CF_3$, $(C_1-C_6)$-Alkyl, $(C_3-C_8)$-Cycloalkyl, Phenyl;

$R^4$, $R^5$ H, F, Br, $CF_3$, $OCF_3$, $(C_1-C_6)$-Alkyl, O-$(C_1-C_6)$-Alkyl;

n 1 bis 2;

$R^2$ $(C_1-C_8)$-Alkyl, wobei in den Alkylgruppen ein oder mehrere $CH_2$-Gruppen durch O, CO, S, SO oder $SO_2$ ersetzt sein können, und Alkyl ein bis dreifach substituiert sein kann durch F, Cl, Br, $CF_3$, CN, $NO_2$, NHAc, NHBoc, NH-CO-$C(CH_3)_3$, Hydroxyl, $OCF_3$, O-$(C_1-C_6)$-Alkyl, COOH, CO-Benzoxy, CO-O$(C_1-C_6)$-Alkyl, Tetrazol, Thiazolidin-2,4-dion, Indol und $(C_6-C_{10})$-Aryl, wobei Thiazolidin-2,4-dion und Aryl wiederum durch F, Cl, Br, $CF_3$, CN, $NO_2$, NHAc, NHTs, NHBoc, NHCbz, NH-CO-$C(CH_3)_3$, Hydroxyl, $OCF_3$, O-$(C_1-C_6)$-Alkyl, COOH, CO-Benzoxy, CO-O$(C_1-C_6)$-Alkyl, $(C_1-C_6)$-Alkyl, O-$(C_1-C_6)$-Alkyl oder Tetrazol substituiert sein können.

Besonders bevorzugt ist ein Verfahren zur Herstellung der Verbindungen der Formel (I),

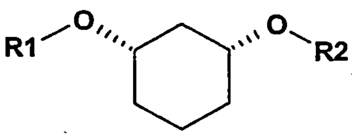

(I)

worin stehen:
$R^1$ für

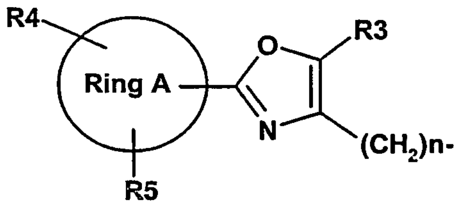

mit

Ring A Phenyl;

$R^3$ $(C_1-C_4)$-Alkyl;

$R^4$, $R^5$ H, $(C_1-C_4)$-Alkyl, O-$(C_1-C_4)$-Alkyl;

n 1;

$R^2$ $(C_1-C_8)$-Alkyl, wobei in den Alkylgruppen ein oder mehrere $CH_2$-Gruppen durch O, CO, S, SO oder $SO_2$ ersetzt sein können, und Alkyl ein bis dreifach substituiert sein kann durch F, Cl, Br, $CF_3$, CN, $NO_2$, NHAc, NHBoc, NH-CO-$C(CH_3)_3$, Hydroxyl, $OCF_3$, O-$(C_1-C_6)$-Alkyl, COOH, CO-Benzoxy, CO-O$(C_1-C_6)$-Alkyl, Tetrazol, Thiazolidin-2,4-dion, Indol und $(C_6-C_{10})$-Aryl, wobei Thiazolidin-2,4-dion und Aryl wiederum durch F, Cl, Br, $CF_3$, CN, $NO_2$, NHAc, NHTs, NHBoc, NHCbz, NH-CO-$C(CH_3)_3$, Hydroxyl, $OCF_3$, O-$(C_1-C_6)$-Alkyl, COOH, CO-Benzoxy, CO-O$(C_1-C_6)$-Alkyl, $(C_1-C_6)$-Alkyl, O-$(C_1-C_6)$-Alkyl oder Tetrazol substituiert sein können.

Die Alkylreste in den Substituenten $R^2$, $R^3$, $R^4$ und $R^5$ können sowohl geradkettig wie verzweigt sein.

Unter einem heteroaromatischen Ring werden sowohl mono- als auch bicyclische Ringe verstanden mitmaximal 4 Heteroatome, insbesondere solche, die bis zu 4 Stickstoffatome und/oder 1 Sauerstoff bzw. 1 Schwefelatom enthalten, wie z.B.: Furan, Thiophen, Thiazol, Oxazol, Thiadiazol, Triazol, Pyridin, Triazin, Chinolin, Isochinolin, Indol, Benzothiophen, Benzofuran, Benzotriazol. Aromatische Ringe können mono- oder bicyclisch und auch anneliert sein, wie z.B. Naphthyl, Benzo[1,3]dioxol, Dihydro-benzo[1,4]-dioxin.

Die racemischen, cis-konfigurierten 1,3-Cyclohexan-Derivate der Formel (IV) und der Formel (VII) werden durch Mono-Alkylierung von cis-Cyclohexandiol (Verbindung der Formel II) hergestellt, können aber auch durch reduktive Öffnung entsprechender Acetale (R. Hunter et al., J. Org. Chem. **1993**, 85, 6756), weiterhin durch sogenannte reduktive Etherbildung, ausgehend von Silylethern und Aldehyden bzw. Ketonen (J. S. Bajwa, X. Jiang, J. Slade, K. Prasad, O. Repic, T. J. Blacklock, Tetrahedron Lett. **2002**, 43, 6709-6713) hergestellt werden.

Die Alkylierungsreagenzien der Formel III sind kommerziell erhältlich oder können nach literaturbekannten Methoden, z. B durch radikalische Seitenkettenhalogenierung (s. Literatur-Übersicht R. C. Larock, Comprehensive Organic Transformations, S. 313, 1989 VCH Publishers, Inc.) oder aus den Alkoholen bzw. daraus herstellbaren Derivaten (s. Literatur-Übersicht R. C. Larock, Comprehensive Organic Transformations, S. 353-363., 1989 VCH Publishers, Inc.) hergestellt werden.

Bekannt ist weiterhin (s. J. Chem. Soc. **1925**, 127, 2275-2297; J. Chem. Soc. **1922**, 121, 2202-2215) die Herstellung verschiedener 2-Bromomethyl-benzoesäurebromide durch radikalische Bromierung, die dann durch weitere Umsetzung mit Alkoholen in die zur Gruppe der Alkylierungsreagenzien der Formel III gehörenden Bromomethylbenzoesäureester überführt werden können.

Die Alkylierungsreagenzien der Formel (VI) bzw. die Alkohole $X^2$ = OH, die als Vorstufen dienen können, sind kommerziell erhältlich oder können nach literaturbekannten Methoden hergestellt werden [a). The Chemistry of Heterocyclic Compounds (Ed.: A. Weissberger, E. C. Taylor): Oxazoles (Ed.: I. J. Turchi); b). Methoden der Organischen Chemie, Houben-Weyl 4. Auflage, Hetarene III, Teilband 1; c) I. Simit, E. Chindris, Arch. Pharm. **1971**, 304, 425; d). Y. Goto, M. Yamazaki, M. Hamana, Chem. Pharm. Bull. **1971**, 19 (10), 2050-2057].

Die Alkylierungsreagenzien der Formel III und VI werden in Gegenwart von Basen mit 1,3-Cyclohexandiol bzw. 1,3-Cyclohexandiolderivaten umgesetzt. Geeignete Basen sind beispielsweise Hydroxide wie KOH, Carbonate wie $Cs_2CO_3$, Alkoholate wie KOtBu sowie Verbindungen wie LDA, BuLi, LiHMDS, KH, NaH und NaHMDS. Geeignete Lösungsmittel sind beispielsweise THF, MTBE, DME, NMP, DMF und Chlorbenzol.

Zur Racematspaltung der Alkohole werden diese in organischen Lösungsmitteln wie z. B. Dimethoxyethan (DME), Methyl-tert.-Butylether (MTBE), Diisopropylether (DIPE), THF, n-Hexan, Cyclohexan, Toluol, Chlorbenzol, Aceton, Dimethylformamid (DMF), Dichlormethan, 1,2-Dichlorethan und tert.-Butanol aufgenommen, Acyldonoren wie

Vinylacetat, Vinylpropionat, Vinylbutyrat, 2,2,2-Trifluorethyl-2H,2H-perfluordecanoat, Ethoxyvinylacetat, p-Nitro- oder p-Chlorphenylacetat, Oximester, Acetanhydrid, Propionsäureanhydrid, Bernsteinsäureanhydrid, Glutarsäureanhydrid, iso-Valeriansäureanhydrid, 2,2,2-Trichlorethylbutyrat, 2,2,2-Trifluorethyl-2H,2H-perfluordecanoat werden zugesetzt und die Reaktionsmischung wird anschließend mit einem geeigneten Enzym versetzt und bei -20 bis 80 °C gerührt. Der Anteil an Cosolvens in der Lösung liegt vorzugsweise bei 10-90 %, ggf. ist es aber auch vorteilhaft die enzymatische Reaktion in reinem Acyldonor, z. B. Vinylacetat, ohne Cosolvens durchzuführen.

Zur Racematspaltung der Esterderivate, z. B. Acetyl-, Propionyl-,Butyryl- oder Glutaryl-, werden diese in homogenen oder heterogenen, wässrigen, wässrig-organischen oder organischen Medien in Gegenwart eines geeigneten Enzyms einer stereoselektiven Hydrolyse oder Alkoholyse (z. B. mit n-Butanol) bei einer Temperatur von 10 - 80 °C gegebenenfalls in Gegenwart von Cosolventien (s. o.) und eines Puffers unterworfen, wobei die Reaktionsmischung vorzugsweise 2 - 50 Gew.-% Ester enthält.

Die Herstellung der oben genannten Esterderivate kann nach literaturbekannten Methoden erfolgen, beispielsweise durch Umsetzung des Alkohols mit Säurechloriden wie Acetylchlorid oder Anhydriden wie Acetanhydrid, in Gegenwart eines Amins, wie z. B. Triethylamin oder Pyridin (s. Literatur-Übersicht R. C. Larock, Comprehensive Organic Transformations, S. 978, 1989 VCH Publishers, Inc.)

Nach Beendigung der Reaktion lassen sich die Produkte bzw. die Enantiomeren auf einfache Weise trennen, z. B. durch Extraktion nach literaturbekannten Methoden [a).T. Yamano, F. Kikumoto, S. Yamamoto, K. Miwa, M. Kawada, T. Ito, T. Ikemoto, K. Tomimatsu, Y. Mizuno, Chem. Lett. **2000**, 448; b). B. Hungerhoff, H. Sonnenschein, F. Theil, J. Org. Chem. **2002**, 67, 1781] oder durch Anwendung chromatographische Methoden.

Eine weitere Methode besteht darin, nach Ablauf der enzymatischen Reaktion die Wasserlöslichkeit des verbleibenden Alkohols durch Derivatisierung, z. B. durch Acylierung mit cyclischen Anhydriden, wie z. B. mit Glutarsäureanhydrid, oder durch Überführung in einen Cholinester [a). H. Kunz, M. Buchholz, Chem. Ber. **1979**, 112, 2145; b.) M. Schelhaas, S. Glomsda, M. Hänsler, H.-D. Jakubke, H. Waldmann, Angew. Chem. **1996,** 108, 82] deutlich zu erhöhen und so eine Trennung von den wasserun- bzw. schlechtlöslichen Estern durch Extraktion zu erreichen. Nach der Trennung lässt sich die Derivatisierung der Alkohole durch chemische oder enzymatische Verseifung wieder rückgängig machen.

Eine besonders interessante Möglichkeit zur Trennung der Enantiomeren besteht darin, im Falle der enzymatischen Acylierung den Acyldonor so zu wählen, dass das acylierte Enantiomere deutlich besser wasserlöslich ist als der nicht umgesetzte Alkohol. Geeignete Acyldonoren sind beispielsweise cyclische Anhydride wie Bernsteinsäureanhydrid. Nach Ablauf der enzymatischen Acylierung trägt das Acylierungsprodukt eine freie Carboxylgruppe, die eine schnelle Abtrennung des Produktes durch wässrige Extraktion im Basischen, zum Beispiel mit ges. wäßriger $NaHCO_3$-Lösung, ermöglicht.

Bei der enzymatischen Racematspaltung durch Esterspaltung geht man vorzugsweise so vor, dass man einen Ester der Formel (I), beispielsweise mit $R^1 = COCH_3$, $COCH_2CH_3$ oder $COCH_2CH_2CH_2COOH$ in einer wasser- oder alkoholhaltigen Lösung mit einer Esterase oder Lipase versetzt und rührt.

Es kann vorteilhaft sein, die genannte Lösung zu puffern, z. B. mit Phosphat- oder TRIS [= Tris-(hydroxymethyl)-methylamin]-Puffer. Der Zusatz kann z. B. 0.01-1.0 molar sein. Ein günstiger Pufferbereich ist pH 5-10.

Als Enzyme werden bevorzugt Hydrolasen aus Säugetierlebern, wie z. B. Lipase aus Schweinepankreas (Fluka) oder aus Mikroorganismen, wie beispielsweise Lipase B aus Candida antarctica (Roche Diagnostics), Lipase OF aus Candida rugosa (Meito Sangyo), Lipase SL aus Pseudomonas cepacia (Meito Sangyo), Lipase L-10 aus Alcaligenes spec. (Roche Diagnostics), und Lipase QL aus Alcaligenes spec. (Meito Sangyo), eingesetzt. Handelt es sich bei den eingesetzten Estern um Glutarsäurederivate, wie z. B Glutarsäure-mono-(3-benzyloxy-cyclohexyl)-ester kann es vorteilhaft sein anstelle der oben genannten Lipasen die Glutaryl-7-ACA-Acylase (Roche Diagnostics) zu verwenden.

Besonders bevorzugt ist Lipase B aus Candida antarctica (Roche Diagnostics), wobei es vorteilhaft sein kann, das freie Enzym oder eine immobilisierte Form des Enzyms, z. B. eines der drei zur Zeit kommerziell erhältlichen Produkte, zu verwenden.

Jedes der genannten Enzyme kann in freier oder in immobilisierter Form (Immobilized Biocatalysts, W. Hartmeier, Springer Verlag Berlin, 1988) eingesetzt werden. Die Enzymmenge wird in Abhängigkeit von der Reaktionsgeschwindigkeit bzw. von der angestrebten Reaktionszeit und von der Art des Enzyms (z. B. frei oder immobilisiert) frei gewählt und ist durch einfache Vorversuche leicht zu bestimmen.

Die Wiedergewinnung des Enzyms kann durch Gefriertrocknung erfolgen. Die Abtrennung (und ggf. spätere Wiederverwendung) des Enzyms kann durch Immobilisierung erleichtert werden.

Durch geeignete Reaktionsführung gelingt es immer, zumindest ein Enantiomeres optisch rein zu erhalten. Strebt man optisch reinen Ester an, sollte der Umsatz im Falle einer enzymatischen Esterbildung unter (oder gleich) 50 % sein, im Falle einer enzymatischen Hydrolyse oder Alkoholyse über (oder gleich) 50 % sein. Strebt man optisch reinen Alkohol an, sollte der Umsatz im Falle einer enzym-katalysierten Esterbildung über (oder gleich) 50 % sein, im Falle einer Hydrolyse oder Alkoholyse unter (oder gleich) 50 % sein.

Die Umsatzbestimmung der enzymatischen Reaktion erfolgte entweder per HPLC direkt aus der Reaktionsmischung oder durch Berechnung aus den optischen Reinheiten der Reaktionsprodukte (Ester und Säure), die ebenfalls direkt

aus der Reaktionsmischung per HPLC an chiraler Phase bestimmt wurden.

Durch die nachfolgenden Beispiele soll die vorliegende Erfindung näher erläutert werden.

Beispiele:

Alle isolierten Produkte bzw. Rohproduktgemische wurden durch $^1H$-NMR- und Massen-Spektren bzw. per HPLC identifiziert.

Die optische Reinheit der Ester und Alkohole wurde durch HPLC, z. B an Chiralpak AD 250 X 4.6 (Daicel) bzw. Chiracel OD 250 x 4,6 bestimmt.

**Zu Schema Ia:**

Beispiel 1

Synthese von racemischem cis-2-(3-Hydroxy-cyclohexyloxymethyl)-6-methyl-benzoesäuremethylester

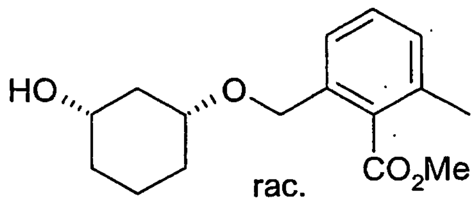

500 g (4.3 mol) cis-1,3-Cyclohexandiol wurden in 5 L NMP gelöst und mit 336 g (3.0 mol) Kalium-tert.-butylat (KOtBu) versetzt. Die Innentemperatur stieg auf 28 °C. Es wurde 30 min gerührt, dann wurde auf - 5 °C abgekühlt und mit 370 g (ca. 94 %ig, ca. 1.4 mol) 2-Bromomethyl-6-methyl-benzoesäuremethylester, den man z. B. durch Methanolyse von 2-Bromomethyl-6-methyl-benzoesäurebromid oder durch Bromierung von 2,6-Dimethylbenzoesäuremethylester herstellen kann, tropfenweise versetzt. Man rührte 30 min und verdünnte dann mit 5 L Wasser. Nach dreimaligem Waschen mit je 3 L n-Heptan und Verwerfen der n-Heptan-Lösungen extrahierte man die verbliebene Wasserphase viermal mit je 2.5 L MTBE. Die vereinigten MTBE-Phasen wurden einmal mit 5 L Wasser gewaschen, mit $Na_2SO_4$ getrocknet und anschließend unter reduziertem Druck eingedampft. Man erhielt 234 g der gewünschten Verbindung als gelbliches Öl, das ohne weitere Reinigung in die nächste Reaktion (z. B. eine Racematspaltung) eingesetzt wurde; $^1H$-NMR ($CDCl_3$), $\delta$ = 1.27 (m, 1 H), 1.45 (m, 1 H), 1.55 (m, 1 H), 1.74 (m, 1 H), 1.83 (m, 1 H), 2.05 (m, 1 H), 2.34 (s, 3 H), 3.47 (m, 1 H), 3.72 (m, 1 H), 3.91 (s, 3 H), 4.58 (dd, 2 H), 7.15 (d, 1 H), 7.20 (d, 2 H), 7.27 (m, 1 H).

Beispiel 2

Racematspaltung von cis-2-(3-Hydroxy-cyclohexyloxymethyl)-6-methyl-benzoesäuremethylester

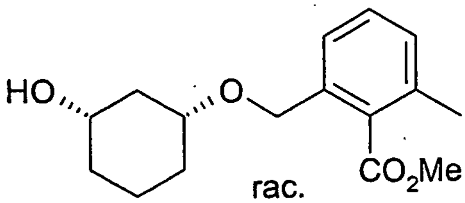

490 g des rohen, racemischen cis 2-(3-Hydroxy-cyclohexyloxymethyl)-6-methylbenzoesäuremethylesters (s. Beispiel 1) wurden in 3.1 L Methylenchlorid und 850 mL Vinylacetat gelöst, mit 18 g Novozym 435 versetzt und bei 21-24 °C gerührt. Nach 28 h wurden weitere 2 g Novozym 435 zugegeben. Nach insgesamt 44 h wurde die Reaktion durch Abfiltrieren des Enzyms beendet und das Filtrat unter reduziertem Druck eingedampft, 540 g wurden erhalten. Chromatographie des Rückstandes an ca. 6 kg Kieselgel (Essigsäureethylester/n-Heptan 1:1) ergab

184 g (1R,3S)-2-(3-Hydroxy-cyclohexyloxymethyl)-6-methyl-benzoesäure-methylester; > 98 % ee (HPLC an Chiralpak

AD-H 250 x 4.6; 1 mL/min, Heptan/EtOH/$CH_3CN$ 25:1:0.5 + 0.1 % TFA); $^1H$-NMR ($CDCl_3$), δ = 1.27 (m, 1 H), 1.45 (m, 1 H), 1.55 (m, 1 H), 1.74 (m, 1 H), 1.83 (m, 1 H), 2.05 (m, 1 H), 2.34 (s, 3 H), 3.47 (m, 1 H), 3.72 (m, 1 H), 3.91 (s, 3 H), 4.58 (dd, 2 H), 7.15 (d, 1 H), 7.20 (d, 2 H), 7.27 (m, 1 H), und 239 g des (1S,3R)-Acetats (93 % ee, HPLC an Chiralcel OD/20 250 x 4.6, 1 mL/min, Heptan/EtOH/$CH_3CN$ 100:1:0.5).

Beispiel 3

Synthese von 4-Iodomethyl-2-(3-methoxy-phenyl)-5-methyl-oxazol

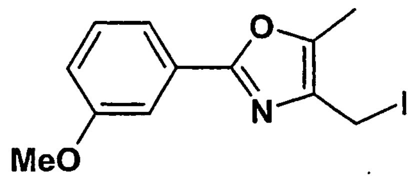

150.0 g (0.63 mol) 4-Chloromethyl-2-(3-methoxy-phenyl)-5-methyl-oxazol wurden in 2.7 L THF gelöst und mit 106 g (0.71 mol) NaI versetzt. Man rührte 4 h und ließ über Nacht stehen, saugte die Salze ab und engte das Filtrat im Vakuum ein. Nach ca.1-2 Stunden wurde das gewünschte Iodid fest, Ausbeute: 216 g, mp 58-59 °C. $^1H$-NMR ($CDCl_3$): δ = 2.30 (s, 3 H), 3.88 (s, 3 H), 4.34 (s, 2 H), 6.97 (dd, 1 H), 7.34 (t, 1 H), 7.52 (d, 1 H), 7.58 (d, 1 H).

Beispiel 4

Synthese von (1R,3S)-2-{3-[2-(3-Methoxy-phenyl)-5-methyl-oxazol-4-ylmethoxy]-cyclohexyl-1-oxymethyl}-6-methyl-benzoesäuremethylester

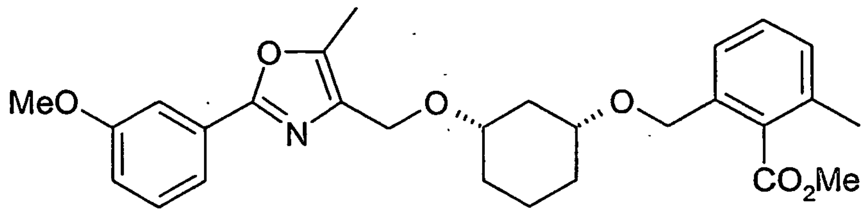

184 g (0.66 mol) (1R,3S)-2-(3-Hydroxy-cyclohexyloxymethyl)-6-methyl-benzoesäuremethylester (s. Beispiel 2) wurden in 2.2 L t-BuOMe gelöst. Man gab 88.0 g (ca. 55 %, 1.8 mmol) NaH hinzu und rührte 45 Minuten bei 20-22 °C. 282 g (83.8 mmol) 4-Iodomethyl-2-(3-methoxy-phenyl)-5-methyl-oxazol (s. Beispiel 3) wurden zugegeben, 8 Stunden bei 22 °C gerührt und über Nacht stehen gelassen. Es wurden weitere 4 h gerührt und dann unter Kühlung vorsichtig zunächst 200 mL, später weitere 1.5 L Wasser zugegeben. Die organische Phase wurde abgetrennt, getrocknet ($Na_2SO_4$) und unter vermindertem Druck eingeengt. Man erhielt 383 g Rohprodukt, das an ca. 6 kg Kieselgel chromatographiert wurde (Dichlormethan/Aceton 19:1), Ausbeute: 199 g eines gelblichen Öls; $^1H$-NMR ($CDCl_3$), δ = 1.15-1.32 (m, 4 H), 1.81 (m, 1 H), 2.00 (m, 1 H), 2.07 (m, 1 H), 2.34 (s, 3 H), 2.40 (s, 3 H), 2.51 (m, 1 H), 3.27 (m, 1 H), 3.37 (m, 1 H), 3.87 (s, 3 H), 3.90 (m, 3 H), 4.48 (s, 2 H), 4.60 (s, 2 H), 6.96 (m, 1 H), 7.12-7.35 (m, 4 H), 7.53 (s, 1 H).7.58 (d, 1H).

Beispiel 5

Synthese von (1 R,3S)-2-{3-[2-(3-Methoxy-phenyl)-5-methyl-oxazol-4-ylmethoxy]-cyclohexyl-1-oxymethyl}-6-methyl-benzoesäure

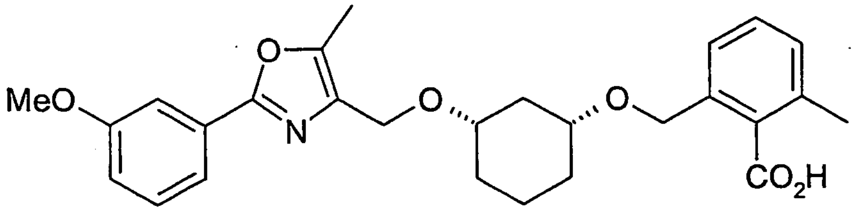

199 g (0.41 mol) (1 R,3S)-2-{3-[2-(3-Methoxy-phenyl)-5-methyl-oxazol-4-ylmethoxy]-cyclohexyl-1-oxymethyl}-6-methyl-benzoesäuremethylester (s. Beispiel 4) wurden in 2 L Ethanol gelöst. Man gab 250 mL 33 %ige NaOH zu und erhitzte 15 Stunden zu Rückfluss. Ethanol wurde im Vakuum abdestilliert, der Rückstand in ca. 2 L Wasser gelöst und viermal mit jeweils 500 mL MTB-Ether gewaschen. Die wässrige Phase wurde unter Kühlung mit konz. Salzsäure auf pH 1 angesäuert und das ölig ausfallende Produkt mit 1.5 L Essigester extrahiert. Die Essigester-Lösung wurde getrocknet und im Vakuum eingeengt. Der Rückstand wurde in 1.2 L DIPE bei ca. 40 °C gelöst. Nach Kristallisation und Trocknen im Vakuum bei 60 °C erhielt man 132.5 g der gewünschten Carbonsäure; mp 103-105 °C; > 98 % ee (HPLC an Chiralpak AD-H 250 x 4.6; 1 mUmin, Heptan/EtOH/$CH_3CN$ 90:7:1 + 0.1 % TFA); $^1$H-NMR ($CDCl_3$), δ = 1.14-1.38 (m, 4 H), 1.80 (m, 1 H), 1.93 (m, 2 H), 2.41 (s, 3 H), 2.44 (s, 3 H), 2.61 (m, 1 H), 3.40 (m, 2 H), 3.86 (s, 3 H), 4.53 (s, 2 H), 4.68 (dd, 2 H), 6.98 (dd, 1 H), 7.17-7.36 (m, 4 H), 7.55 (s, 1 H), 7.61 (d, 1 H).

Beispiel 6

Synthese von 4-lodomethyl-2-(4-methyl-phenyl)-5-methyl-oxazol

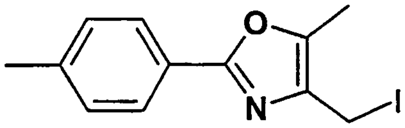

6.0 g 4-Chloromethyl-2-(4-methyl-phenyl)-5-methyl-oxazol wurden in 120 mL THF gelöst und mit 4.18 g (27.9 mmol) Nal versetzt. Man rührte 3.5 h, gab weitere 1.5 g Nal hinzu und erwärmte auf 35 °C. Nach 30 Minuten saugte man die Salze ab und engte das Filtrat im Vakuum ein; Ausbeute: 10.1 g, mp 104-105 °C; $^1$H-NMR ($CDCl_3$): δ = 2.29 (s, 3 H), 2.39 (s, 3 H), 4.34 (s, 2 H), 7.24 (d, 2 H), 7.88 (d, 2 H).

Beispiel 7

Synthese von (1 R,3S)-2-{3-[2-(4-Methyl-phenyl)-5-methyl-oxazol-4-ylmethoxy]-cyclohexyl-1-oxymethyl}-6-methyl-benzoesäuremethylester

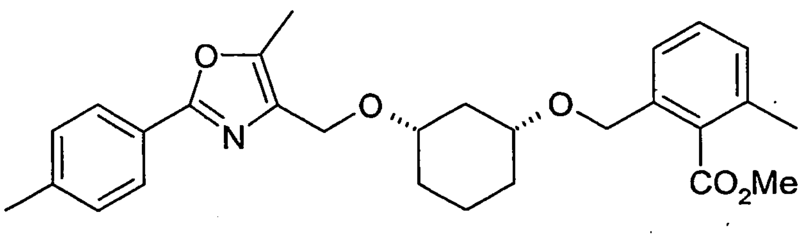

36.0 g (0.129 mol) (1R,3S)-2-(3-Hydroxy-cyclohexyloxymethyl)-6-methyl-benzoesäuremethylester (s. Beispiel 2) wurden in 430 mL tBuOMe gelöst. Man gab 17.2 g (ca. 55 %, 0.35 mol) NaH hinzu und rührte 30 Minuten bei 23 °C. 55.1 g (0.166 mol) 4-Iodomethyl-2-(4-methyl-phenyl)-5-methyl-oxazol (Beispiel 6) wurden zugegeben. Nach 6 Stunden Rühren und Stehenlassen über 2 Tage gab man unter Kühlung 400 mL Wasser hinzu und trennte die organische Phase ab. Nach Trocknen ($Na_2SO_4$) und Einengen wurde das Rohprodukt (75 g) an Kieselgel (ca. 1 kg) chromatographiert (Dichlormethan/ Aceton 19:1), Ausbeute: 42 g des dialkylierten 1,3-Cyclohexandiolderivates als gelbliches Öl; $^1$H-NMR ($CDCl_3$), δ = 1.16-1.31 (m, 4 H), 1.80 (m, 1 H), 1.97-2.1 (m, 2 H), 2.34 (s, 3 H), 2.39 (s, 3 H), 2.40 (s, 3 H), 2.52 (m, 1

H), 3.27 (m, 1 H), 3.37 (m, 1 H), 3.89 (s, 3 H), 4.47 (s, 2 H), 4.59 (s, 2 H), 7.13 (d, 1 H), 7.20-7.28 (m, 4 H), 7.88 (d, 1 H).

Beispiel 8

Synthese von (1 R,3S)-2-{3-[2-(4-Methyl-phenyl)-5-methyl-oxazol-4-ylmethoxy]-cyclohexyl-1-oxymethyl}-6-methyl-benzoesäure

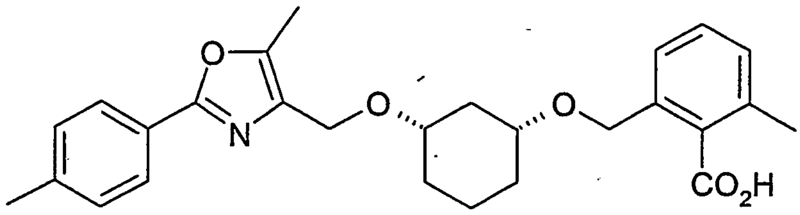

42.0 g (0.09 mol) (1 R,3S)-2-{3-[2-(4-Methyl-phenyl)-5-methyl-oxazol-4-ylmethoxy]-cyclohexyl-1-oxymethyl}-6-methyl-benzoesäuremethylester (s. Beispiel 7) wurden in 420 mL Ethanol gelöst. Man gab 45 mL 33 %ige NaOH zu und erhitzte ca. 20 Stunden zu Rückfluss. Ethanol wurde im Vakuum abdestilliert, der Rückstand in 500 mL Wasser gelöst und die Lösung viermal mit jeweils 100 mL MTB-Ether gewaschen. Die wässrige Phase wurde unter Kühlung mit konz. Salzsäure angesäuert (pH 1) und das ölig ausfallende Produkt mit Essigester extrahiert. Die Essigester-Lösung wurde getrocknet und im Vakuum eingeengt. Der Rückstand wurde in 250 mL DIPE in der Wärme gelöst. Beim Abkühlen setzte die Kristallisation ein. Nach Beendigung der Kristallisation und Trocknen im Vakuum bei 60 °C erhielt man 28.4 g der gewünschten Carbonsäure; mp 117-119 °C; > 98 % ee (HPLC an Chiralpak AD-H 250 x 4.6; 1 mUmin, Heptan/EtOH/$CH_3CN$ 90:7:1 + 0.1 % TFA); $^1$H-NMR ($CDCl_3$), δ = 1.14-1.36 (m, 4 H), 1.80 (m, 1 H), 1.91 (m, 2 H), 2.39 (s, 3 H), 2.40 (s, 3 H), 2.46 (s, 3 H), 2.64 (m, 1 H), 3.40 (m, 2 H), 4.54 (s, 2 H), 4.68 (dd, 2 H), 7.17-7.30 (m, 5 H), 7.91 (d, 2 H).

Beispiel 9

Synthese von 4-Iodomethyl-2-(3-methyl-phenyl)-5-methyl-oxazol

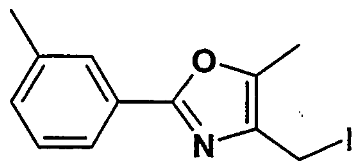

6.0 g 4-Chloromethyl-2-(4-methyl-phenyl)-5-methyl-oxazol wurden in 120 mL THF gelöst und mit 4.5 g (30 mmol) NaI versetzt. Man rührte 5 h und ließ dann über Nacht stehen. Abtrennen des Feststoffes und Einengen des Filtrates im Vakuum ergab 10.2 g des gewünschten Iodids; mp ~ 32 °C; $^1$H-NMR ($CDCl_3$): δ = 2.30 (s, 3 H), 2.40 (s, 3 H), 4.34 (s, 2 H), 7.24 (d, 1 H), 7.32 (t, 1 H), 7.77 (d, 1 H), 7.83 (d, 1 H).

Beispiel 10

Synthese von (1 R,3S)-2-{3-[2-(3-Methyl-phenyl)-5-methyl-oxazol-4-ylmethoxy]-cyclohexyl-1-oxymethyl}-6-methyl-benzoesäuremethylester

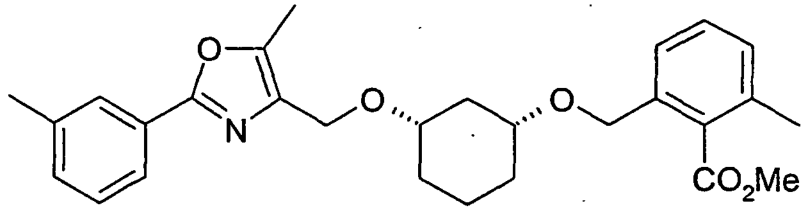

36.0 g (0.129 mol) (1 R,3S)-2-(3-Hydroxy-cyclohexyloxymethyl)-6-methyl-benzoesäuremethylester (s. Beispiel 2), in 430 mL tBuOMe gelöst. Man gab 17.19 g (ca. 55 %, 0.35 mol) NaH hinzu und rührte 30 Minuten bei 20-22 °C. 55.1 g (0.166 mol) 4-Iodomethyl-2-(3-methyl-phenyl)-5-methyl-oxazol (s. Beispiel 9) wurden zugegeben. Nach 6 Stunden Rühren und Stehenlassen über 2 Tage gab man unter Kühlung 400 mL Wasser hinzu und trennte die organische Phase ab. Nach Trocknen ($Na_2SO_4$) und Einengen wurde das Rohprodukt (75 g) an Kieselgel (1.2 kg) chromatographiert (Dichlormethan/ Aceton 19:1), Ausbeute: 49 g (1 R,3S)-2-{3-[2-(3-Methyl-phenyl)-5-methyl-oxazol-4-ylmethoxy]-cyclohexyl-1-oxymethyl}-6-methyl-benzoesäuremethylester; $^1$H-NMR ($CDCl_3$), $\delta$ = 1.13-1.31 (m, 4 H), 1.80 (m, 1 H), 1.97-2.1 (m, 2 H), 2.34 (s, 3 H), 2.40 (s, 3 H), 2.41 (s, 3 H), 2.52 (m, 1 H), 3.27 (m, 1 H), 3.37 (m, 1 H), 3.90 (s, 3 H), 4.48 (s, 2 H), 4.59 (s, 2 H), 7.12-7.33 (m, 4 H), 7.78 (d, 1 H), 7.84 (s, 1 H).

Beispiel 11

Synthese von (1R,3S)-2-{3-[2-(3-Methyl-phenyl)-5-methyl-oxazol-4-ylmethoxy]-cyclohexyl-1-oxymethyl}-6-methyl-benzoesäure

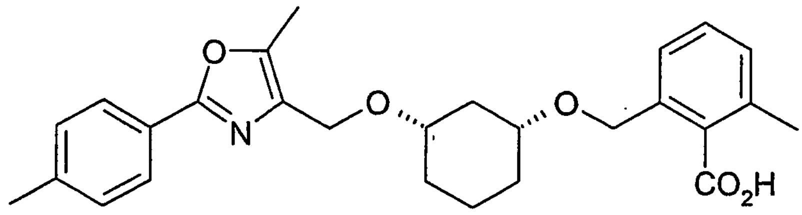

49.0 g (0.09 mol) (1R,3S)-2-{3-[2-(3-Methyl-phenyl)-5-methyl-oxazol-4-ylmethoxy]-cyclohexyl-1-oxymethyl}-6-methyl-benzoesäuremethylester (s. Beispiel 10) wurden in 500 mL Ethanol gelöst. Man gab 50 mL 33 %ige NaOH zu und erhitzte ca. 14 Stunden zu Rückfluss. Ethanol wurde im Vakuum abdestilliert, der Rückstand in 500 mL Wasser gelöst und die Lösung dreimal mit jeweils 150 mL MTB-Ether gewaschen. Die wässrige Phase wurde unter Kühlung mit konz. Salzsäure angesäuert (pH 1) und das ölige Produkt mit Essigester extrahiert. Die Essigester-Lösung wurde getrocknet und im Vakuum eingeengt. Der Rückstand wurde in 250 mL DIPE in der Wärme gelöst. Beim Abkühlen setzte die Kristallisation ein. Nach Beendigung der Kristallisation und Trocknen im Vakuum bei 60°C erhielt man
29.9 g der gewünschten Carbonsäure; mp 109-111 °C; ; > 98 % ee (HPLC an Chiralpak AD-H 250 x 4.6; 1 mL/min, Heptan/EtOH/$CH_3CN$ 90:7:1 + 0.1 % TFA); $^1$H-NMR ($CDCl_3$), $\delta$ = 1.14-1.36 (m, 4 H), 1.80 (m, 1 H), 1.93 (m, 2 H), 2.40 (s, 2 x 3 H), 2.45 (s, 3 H), 2.64 (m, 1 H), 3.40 (m, 2 H), 4.53 (s, 2 H), 4.68 (dd, 2 H), 7.17-7.34 (m, 5 H), 7.81 (d, 1 H), 7.85 (s, 1 H).

**Zu Schema IIa**

Beispiel 12

Racematspaltung von cis-3-[2-(3-Methoxy-phenyl)-5-methyl-oxazol-4-ylmethoxy]-cyclohexan-1-ol

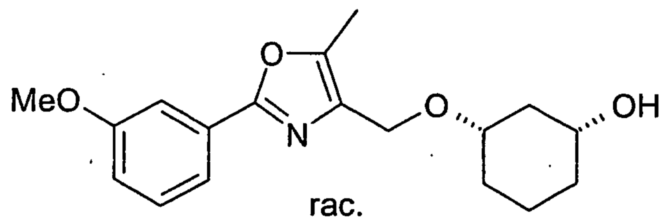

24.9 g des racemischen cis-3-[2-(3-Methoxy-phenyl)-5-methyl-oxazol-4-ylmethoxy]-cyclohexan-1-ols (hergestellt durch Alkylierung von cis-1,3-Cyclohexandiol mit 4-Iodomethyl-2-(3-methoxy-phenyl)-5-methyl-oxazol) wurden in 100 mL Vinylacetat gelöst, mit 1.0 g Chirazyme L-2, lyo., versetzt und bei 20-23 °C gerührt. Nach etwa 30 Minuten wurde das Enzym abfiltriert und die Lösung im Vakuum eingeengt, Rohprodukt: 25.8 g. Nach Chromatographie an Kieselgel (n-Heptan/Essigester 10:1 - 0:1) erhielt man 13.7 g (1S,3R)-3-[2-(3-Methoxy-phenyl)-5-methyl-oxazol-4-ylmethoxy]-cyclohexan-1-ol und 11.3 g der (1 R,3S)-Acetylverbindung.

Beispiel 13

Herstellung von (1R,3S)-3-[2-(3-Methoxy-phenyl)-5-methyl-oxazol-4-ylmethoxy]-cyclohexan-1-ol

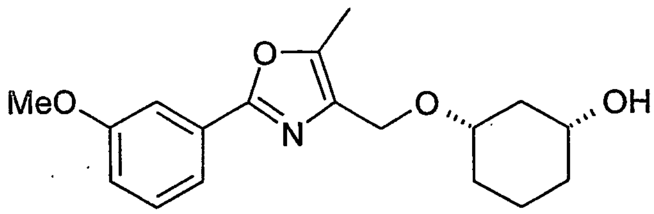

11.2 g des (1R,3S)-Acetats aus Beispiel 12 wurden in ca. 100 mL MeOH gelöst, mit 0.5 mL NaOMe (30 %ig) versetzt und bei 20-23 °C gerührt. Nach 3.5 h wurde mit konzentrierter Essigsäure neutralisiert, mit Essigester aufgenommen, mit $NaHCO_3$ gewaschen, mit $Na_2SO_4$getrocknet und im Vakuum eingeengt. Nach Filtration über Kieselgel (n-Heptan/Essigester 10:1 - 0:1) erhielt man 8.8 g (1R,3S)-3-[2-(3-Methoxy-phenyl)-5-methyl-oxazol-4-ylmethoxy]-cyclohexan-1-ols mit 92 % ee (HPLC an Chiralpak AD-H 250 x 4.6; 1 mL/min, Heptan/EtOH/$CH_3CN$ 90:7:1 + 0.1 % TFA).

Beispiel 14

Synthese von (1 R,3S)-2-{3-[2-(3-Methoxy-phenyl)-5-methyl-oxazol-4-ylmethoxy]-cyclohexyl-1-oxymethyl}-6-methyl-benzoesäuremethylester

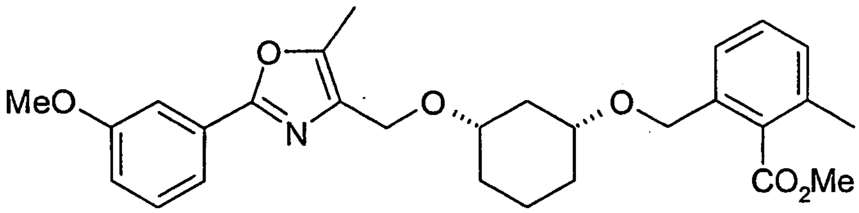

1.4 g (4.4 mmol) (1 R,3S)-3-[2-(3-Methoxy-phenyl)-5-methyl-oxazol-4-ylmethoxy]-cyclohexanol (s. Beispiel 13) wurden in 15 mL tBuOMe aufgenommen, bei 24-27 °C mit 1.20 g (10.7 mmol) KOtBu versetzt und ca. 30 Minuten gerührt. Man kühlte auf 0-5 °C ab, gab 1.89 g (ca. 94 %ig, ca. 7.4 mmol) 2-Bromomethyl-6-methyl-benzoesäuremethylester tropfenweise zu und rührte zunächst 30 Minuten bei 0-5 °C. Ohne weitere Kühlung hatte die Reaktionsmischung nach 1.5 Stunden eine Temperatur von ca. 20 °C. Nach Rühren über Nacht und Zugabe von ca. 200 mg KOtBu war die Reaktion nach einer weiteren Stunde Rühren bei 22 °C beendet. Abdestillieren des Lösungsmittels im Vakuum, Verteilen des Rückstandes zwischen Wasser und tBuOMe und Trocknen der produkthaltigen organischen Phase ergab nach Einengen im Vakuum 1.6 g (1 R,3S)-2-{3-[2-(3-Methoxy-phenyl)-5-methyl-oxazol-4-ylmethoxy]-cyclohexyl-1-oxymethyl}-6-methyl-benzoesäuremethylester als gelbliches Öl; $^1$H-NMR ($CDCl_3$),$\delta$ = 1.15-1.32 (m, 4 H), 1.81 (m, 1 H), 2.00 (m, 1 H), 2.07 (m, 1 H), 2.34 (s, 3 H), 2.40 (s, 3 H), 2.51 (m, 1 H), 3.27 (m, 1 H), 3.37 (m, 1 H), 3.87 (s, 3 H), 3.90 (s, 3 H), 4.48 (s, 2 H), 4.60 (s, 2 H), 6.96 (m, 1 H), 7.12-7.35 (m, 4 H), 7.53-7.60 (m, 2 H).

Beispiel 15

Synthese von (1S,3R)-2-{3-[2-(3-Methoxy-phenyl)-5-methyl-oxazol-4-ylmethoxy]-cyclohexyl-1-oxymethyl}-6-methyl-benzoesäuremethylester

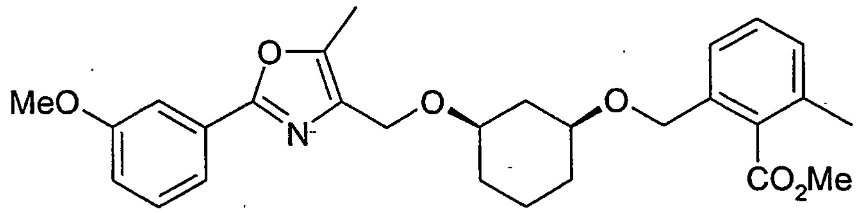

Ausgehend von (1S,3R)-3-[2-(3-Methoxy-phenyl)-5-methyl-oxazol-4-ylmethoxy]-cyclohexan-1-ol aus Beispiel12 gelangt man durch Alkylierung analog Beispiel 14 zu (1S,3R)-2-{3-[2-(3-Methoxy-phenyl)-5-methyl-oxazol-4-ylmethoxy]-cyclohexyl-1-oxymethyl}-6-methyl-benzoesäuremethylester; die $^1$H-NMR-Daten stimmen mit denen in Beispiel 14 überein.

Beispiel 16

Racematspaltung von cis-3-[2-(4-Fluor-phenyl)-oxazol-4-ylmethoxy]-cyclohexan-1-ol, Herstellung von (1S, 3R)- 3-[2-(4-Fluor-phenyl)-oxazol-4-ylmethoxiy]-cydohexan-1-ol

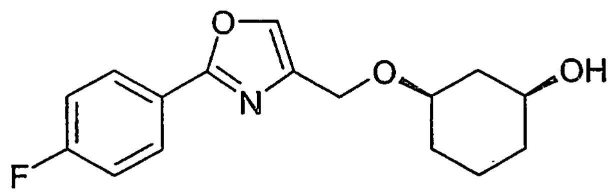

30 mg racemisches cis-3-[2-(4-Fluor-phenyl)-oxazol-4-ylmethoxy]-cydohexan-1-ol wurden in etwa 3 mL Dichlormethan aufgenommen, mit 60 mg p-Nitrophenylacetat versetzt und mit 10 mg Novozyme 435 bei 20-23 °C gerührt. Nach 70 h wurde das immobilisierte Enzym abfiltriert. Die Bestimmung der optischen Reinheit direkt aus dem eingedampften Reaktionsgemisch ergab für (1S,3R)- 3-[2-(4-Fluor-phenyl)-oxazol-4-ylmethoxy]-cyclohexan-1-ol > 95% ee (HPLC an Chiralpak AD 250 x 4.6; 1 mL/min, Acetonitril) und für das (1 R,3S)-Acetat 95 % ee (HPLC an Chiralpak AD 250 x 4.6, 1 mL/min, Acetonitril). Zur Isolierung von (1S,3R)- 3-[2-(4-Fluor-phenyl)-oxazol-4-ylmethoxy]-cyclohexan-l-ol wurde das Rohgemisch an Kieselgel (EE/n-Heptan) chromatographiert; Ausbeute 12 mg, 96 % ee.

Beispiel 17

Synthese von (1S,3R)-2-{3-[2-(4-Fluor-phenyl)-oxazol-4-ylmethoxy]-cyclohexyl-1-oxymethyl}-6-methyl-benzoesäuremethylester

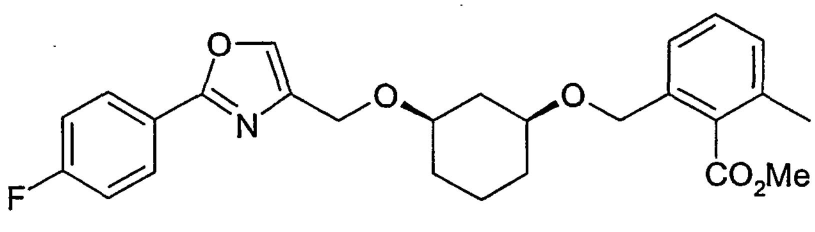

Ausgehend von (1S,3R)- 3-[2-(4-Fluor-phenyl)-oxazol-4-ylmethoxy]-cyclohexan-1-ol aus Beispiel 16 gelangt man durch Alkylierung mit 2-Bromomethyl-6-methyl-benzoesäuremethylester zu (1S,3R)-2-{3-[2-(4-Fluor-phenyl)-oxazol-4-ylmethoxy]-cyclohexyl-1-oxymethyl}-6-methyl-benzoesäuremethylester (s. Beispiel 35).

Beispiel 18

Racematspaltung von 3-[2-(4-Methyl-phenyl)-5-methyl-oxazol-4-ylmethoxy]-cyclohexan-1-ol

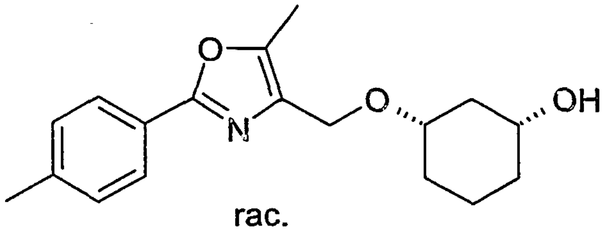

rac.

wobei $R^4$ = p-Me-, $R^5$ = H und $R^3$ = Me)

16.3 g des racemischen 3-[2-(4-Methyl-phenyl)-5-methyl-oxazol-4-ylmethoxy]-cyclohexan-1-ols wurden in 100 mL Vinylacetat gelöst, mit 1.9 g Chirazyme L-2, lyo., versetzt und bei 20-23 °C gerührt. Nach etwa 30 Minuten wurde das Enzym abfiltriert und die Lösung im Vakuum eingeengt, Rohprodukt: 16.6 g. Nach Chromatographie an Kieselgel (n-Heptan/Essigester 10: 1 - 0:1) erhält man 8.6 g (1S,3R)-3-[2-(4-Methylphenyl)-5-methyl-oxazol-4-ylmethoxy]-cyclohexan-1-01 und 6.8 g des (1R,3S)-Acetats.

Beispiel 19

Herstellung von (1R,3S)-3-[2-(4-Methyl-phenyl)-5-methyl-oxazol-4-ylmethoxy]-cyclohexan-1-ol

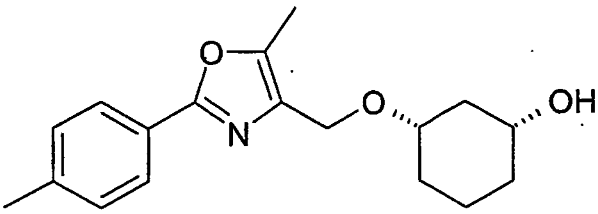

6.8 g der (1R,3S)-Acetylverbindung aus Beispiel 18 wurden in ca. 65 mL MeOH gelöst, mit 0.32 mL NaOMe (30 %ig) versetzt und bei 20-23 °C gerührt. Nach 4 h wurde mit Essigsäure neutralisiert, im Vakuum eingeengt, mit Essigester aufgenommen, mit $NaHCO_3$ gewaschen, getrocknet ($Na_2SO_4$) und im Vakuum eingeengt. Nach Filtration über Kieselgel (n-Heptan/ Essigester 10:1 - 0:1) erhält man 8.8 g des gewünschten (1 R,3S)-3-[2-(4-Methyl-phenyl)-5-methyl-oxazol-4-ylmethoxy]-cyclohexan-1-ols mit 95 % ee (HPLC an Chiralpak AD 250 x 4.6; 1 mL/min, Heptan/EtOH/$CH_3CN$ 90:7:1 + 0.1 % TFA).

Beispiel 20

Racematspaltung von cis-3-[2-phenyl-5-methyl-oxazol-4-ylmethoxy]-cyclohexan-1-ol

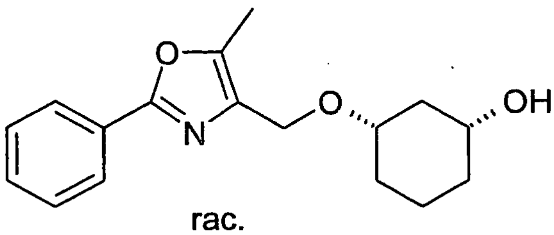

rac.

2.0 g racemisches cis-3-[2-phenyl-5-methyl-oxazol-4-ylmethoxy]-cyclohexan-1-ol wurden in 50 mL Vinylacetat gelöst, mit 0.1 g Chirazyme L-2, lyo., versetzt und bei 20-23 °C gerührt. Nach etwa 5 h wurde das Enzym abfiltriert und die Lösung im Vakuum eingeengt. Nach Chromatographie an Kieselgel (n-Heptan/Essigester 2:1 -1:2) erhält man 1.0 g (1S,3R)-3-[2-phenyl-5-methyl-oxazol-4-ylmethoxy]-cyclohexan-1-ol als hellgelber Feststoff und 0.96 g der acetylierten (1R,3S)-Verbindung als farbloses Öl.

Beispiel 21

Herstellung von (1R,3S)-3-[2-phenyl-5-methyl-oxazol-4-ylmethoxy]-cyclohexan-1-ol

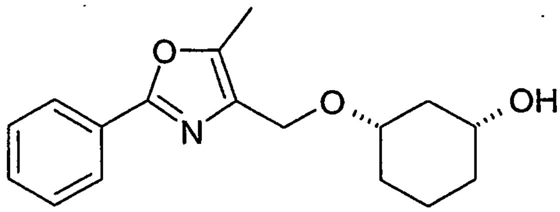

0.96 g der (1R,3S)-Acetylverbindung aus Beispiel 20 wurden in ca. 5-10 mL MeOH gelöst, mit 0.1 mL NaOMe (30 %ig) versetzt und bei 20-23 °C gerührt. Nach 3 h wurde mit Essigsäure neutralisiert und im Vakuum eingeengt, mit Essigester aufgenommen, mit gesättigter $NaHCO_3$ gewaschen, getrocknet ($MgSO_4$) und im Vakuum eingeengt. Nach Filtration über Kieselgel (n-Heptan/ Essigester 10: 1 - 0:1) erhielt man 0.84 g des gewünschten (1 R,3S)-3-[2-phenyl-5-methyl-oxazol-4-ylmethoxy]-cyclohexan-1-ols mit 95 % ee (HPLC an Chiralpak AD 250 x 4.6; 1 mL/min, Heptan/ EtOH/$CH_3CN$ 25:1:0.5 + 0.1 % TFA).

Beispiel 22

Racematspaltung von cis-3-[2-(4-Methoxy-phenyl)-5-methyl-oxazol-4-ylmethoxy]-cyclohexan-1-ol

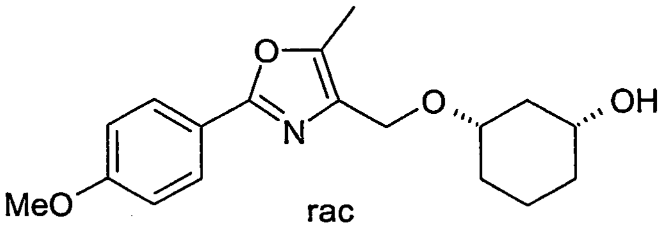

rac

2.0 g des racemischen cis-3-[2-(4-Methoxy-phenyl)-5-methyl-oxazol-4-ylmethoxy]-cyclohexan-1-ols wurden in 50 mL Vinylacetat gelöst, mit 0.1 g Chirazyme L-2, lyo., versetzt und bei 20-23 °C gerührt. Nach etwa 5 h wurde das Enzym abfiltriert und die Lösung im Vakuum eingeengt. Nach Chromatographie an Kieselgel (n-Heptan/Essigester 2:1-1:2) erhielt man 1.16 g (1S,3R)-3-[2-(4-Methoxy-phenyl)-5-methyl-oxazol-4-ylmethoxy]-cyclohexan-1-ol und 0.79 g des (1R, 3S)-Acetats.

Beispiel 23

Herstellung von (1R,3S)-3-[2-(4-Methoxy-phenyl)-5-methyl-oxazol-4-ylmethoxy]-cyclohexan-1-ol

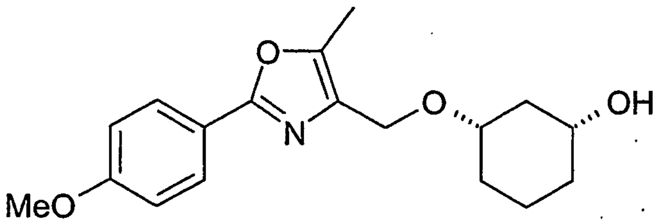

0.79 g Acetat aus Beispiel 22 wurden in ca. 5-10 mL MeOH gelöst, mit 0.1 mL NaOMe (30 %ig) versetzt und bei 20-23 °C gerührt. Nach 3 h wurde mit verdünnter Essigsäure neutralisiert und im Vakuum eingeengt, mit Essigester aufgenommen, mit gesättigter $NaHCO_3$ gewaschen, getrocknet ($MgSO_4$) und im Vakuum eingeengt. Nach Filtration über

Kieselgel (n-Heptan/ Essigester 10:1 - 0:1) erhielt man 0.84 g (1 R,3S)-3-[2-(4-Methoxy-phenyl)-5-methyl-oxazol-4-ylmethoxy]-cydohexan-1-ol als gelbes Öl mit 92 % ee (HPLC an Chiralpak AD 250 x 4.6; 1 mL/min, Heptan/EtOH/$CH_3CN$ 90:7:1 + 0.1 % TFA).

Beispiel 24

Racematspaltung von cis-3-[2-(4-Fluor-phenyl)-5-methyl-oxazol-4-ylmethoxy]-cyclohexan-1-ol

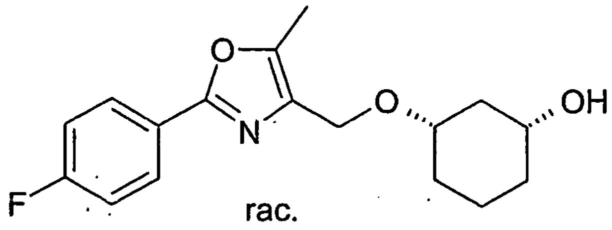

rac.

1.70 g racemisches cis-3-[2-(4-Fluor-phenyl)-5-methyl-oxazol-4-ylmethoxy]-cydohexan-1-ol wurden in 50 mL Vinylacetat gelöst, mit 0.1 g Chirazyme L-2, lyo., versetzt und bei 20-23 °C gerührt. Nach etwa 1.5 h wurde das Enzym abfiltriert und die Lösung im Vakuum eingeengt. Nach Chromatographie an Kieselgel (n-Heptan/Essigester 5:1 -1:1) erhielt man 1.0 g (1S,3R)-3-[2-(4-Fluor-phenyl)-5-methyloxazol-4-ylmethoxy]-cyclohexan-1-ol und 0.75 g des (1R,3S)-Acetats.

Beispiel 25

Herstellung von (1R,3S)-3-[2-(4-Fluor-phenyl)-5-methyl-oxazol-4-ylmethoxy]-cyclohexan-1-ol

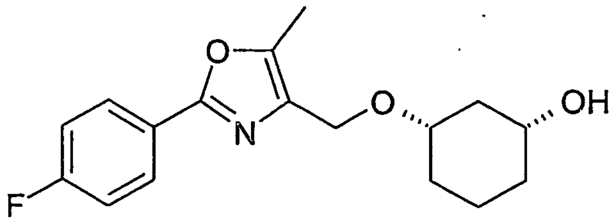

0.75 g Acetat aus Beispiel 24 wurden in ca. 30 mL MeOH gelöst, mit 0.2 mL NaOMe (30 %ig) versetzt und bei 20-23 °C gerührt. Nach 1 h wurde mit verdünnter Essigsäure neutralisiert und im Vakuum eingeengt, mit Essigester aufgenommen, mit gesättigter $NaHCO_3$ gewaschen, getrocknet ($MgSO_4$) und im Vakuum eingeengt, Ausbeute: 0.59 g (1 R, 3S)-3-[2-(4-Fluor-phenyl)-5-methyl-oxazol-4-ylmethoxy]-cyclohexan-1-ol als weißer Feststoff mit 94 % ee (HPLC an Chiralpak OD/19 250 x 4.6; 1 mL/min, Heptan/ EtOH/$CH_3CN$ 110:2:1 + 0.05 % TFA).

**Zu Schema IIb**

Beispiel 26

Stereoselektive Hydrolyse von Essigsäure-3-[2-(4-fluor-phenyl)-oxazol-4-ylmethoxyl]-cyclohexylester, Herstellung von (1R,3S)-3-[2-(4-Fluor-phenyl)-oxazol-4-ylmethoxy]-cyclohexan-1-ol

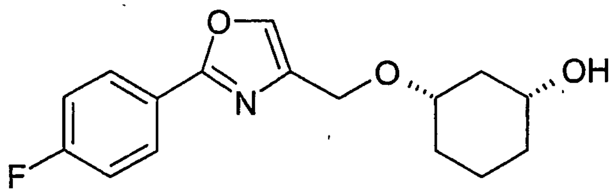

Ca. 10 mg racemische Essigsäure-3-[2-(4-fluor-phenyl)-oxazol-4-ylmethoxyl]-cyclohexylester (hergestellt durch Umsetzung von 3-Benzyloxy-cyclohexan-1-ol mit Essigsäureanhydrid, analog der Synthese von Glutarsäure-mono-(3-benzyloxycyclohexyl)-ester, s. Beispiel 39) wurden in 2 mL Phosphatpuffer (0.1 M, pH = 7.0) und 2 mL DME aufgenommen und mit ca. 5 mg Chirazyme L-2, lyo., bei 20-23 °C ca. 20-24 h gerührt. Die Reaktionsmischung wurde mit Dichlormethan extrahiert. Die organische Phase wurde mit Toluol versetzt und im Vakuum eingedampft. Die Bestimmung der optischen Reinheit ergab für (1 R,3S)-3-[2-(4-Fluor-phenyl)-oxazol-4-ylmethoxy]-cyclohexan-1-ol 99.4 % ee (HPLC an Chiralpak AD 250 x 4.6; 1 mL/min, Acetonitril) und für das (1S,3R)-Acetat 98.9 % ee (HPLC an Chiralcel OD 250 x 4.6, 1 mL/min, Heptan/EtOH/$CH_3CN$ 110:5:1 + 0.1 % TFA).

**Zu Schema IIIa**

Beispiel 27

Synthese von racemischem cis-3-Benzyloxy-cyclohexan-1-ol

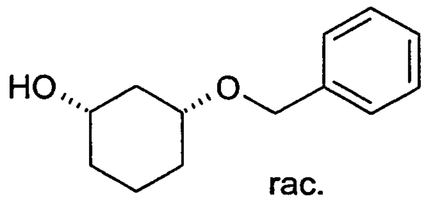

rac.

150.0 g (1.29 mol) cis-1,3-Cyclohexandiol wurden in 1.5 L NMP gelöst, mit 111.6 g (0.99 mol) Kalium-tert.-butylat (KOtBu) versetzt und bei 25-27°C gerührt. Nach etwa 30 Minuten wurde auf 0 °C abgekühlt und mit 78.1 g (0.46 mol) Benzylbromid tropfenweise versetzt. Man rührte 15 min bei ca. 0 °C und gab dann 1.5 L Wasser zu.

Nach dreimaligem Waschen mit 700 mL n-Heptan und Verwerfen der n-Heptan-Lösungen extrahierte man die wässrige Lösung viermal mit 500 mL MTBE. Die vereinigten MTBE-Phasen wurden zweimal mit jeweils 1 L Wasser gewaschen, getrocknet ($Na_2SO_4$) und anschließend unter reduziertem Druck eingedampft. Man erhielt 48.0 g der gewünschten Verbindung als klares, gelbes Öl; $^1$H-NMR ($CDCl_3$), $\delta$ = 1.29 (m, 1 H), 1.43-1.93 (m, 6 H), 2.06 (m, 1 H), 2.55 (s (br.), 1 H), 3.56 (m, 1 H), 3.74 (br, 1 H), 4.55 (dd, 2 H), 7.25-7.36 (m, 5 H).

Beispiel 28

Racematspaltung von 3-Benzyloxy-cyclohexan-1-ol

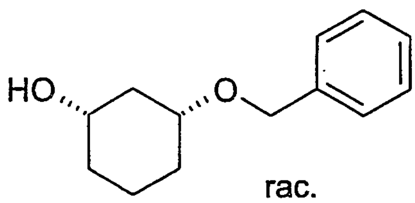

rac.

20.3 g cis-3-Benzyloxy-cyclohexan-1-ol werden in 35 mL Vinylacetat und 125 mL Methylenchlorid gelöst, mit 2.0 g Novozym 435 versetzt und bei 20-23 °C 6 h gerührt. Nach Stehenlassen über Nacht wurde das Enzym abfiltriert. Eine

Probe wurde entnommen und im Vakuum eingedampft. Der Enantiomeren-Überschusses von (1S,3R)-3-Benzyloxy-cyclohexan-1-ol betrug > 99 % (HPLC an Chiralpak AD-H 250 x 4.6; 1 mL/min, Heptan/EtOH/$CH_3CN$ 25:1:0.5 + 0.1 % TFA), der Enantiomeren-Überschuss des (1R,3S)-Acetats betrug 78 % (HPLC an Chiralcel OD 250 x 4.6; 1 mL/min, Heptan/EtOH/ $CH_3CN$ 100:1:0.5).

Beispiel 29

Racematspaltung von 3-Benzyloxy-cydohexan-1-ol

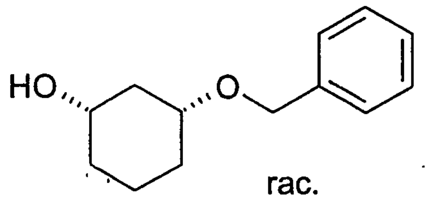

100.0 g cis-3-Benzyloxy-cyclohexan-1-ol werden in 170 mL Vinylacetat und 630 mL Methylenchlorid gelöst, mit 5.0 g Novozym 435 versetzt und bei 20-23 °C 26 h gerührt. Das Enzym wurde abfiltriert, eine Probe wurde entnommen und im Vakkum eingedampft. Der Enantiomeren-Überschusses von (1S,3R)-3-Benzyloxy-cyclohexan-1-ol betrug > 99 % (HPLC an Chiralpak AD-H 250 x 4.6; 1 mL/min, Heptan/EtOH/$CH_3CN$ 25:1:0.5 + 0.1 % TFA), der Enantiomeren-Überschuss des (1R,3S)-Acetats betrug 90 % (HPLC an Chiralcel OD 250 x 4.6; 1 mL/min, Heptan/EtOH/ $CH_3CN$ 100:1:0.5).

Beispiel 30

Isolierung von (1S,3R)-3-Benzyloxy-cyclohexan-1-ol, Trennung des Gemisches aus Acetat und Alkohol mit Pyridin-$SO_3$

1.9 g des Acetafi/Alkohol-Rohgemisches aus der stereoselektiven enzymatischen Acetylierung von 3-Benzyloxy-cyclohexan-1-ol (aus Beispiel 29) wurden in 10 mL Pyridin und 2 mL DMF mit 2 g Pyridin-$SO_3$ bei 20-22°C gerührt. Nach 4 h war die Umsetzung des Benzylcyclohexanols zum Pyridinsalzes des Schwefelsäureesters nahezu quantitativ. Die Reaktionsmischung wurde mit 40 mL Wasser verdünnt und zweimal mit ca. 20 mL MTBE extrahiert. Die MTBE-Phasen enthalten quantitativ das unveränderte (1 R,3S)-Acetat. Die verbleibende, Acetat-freie Wasserphase wurde im Vakuum eingedampft. Der Rückstand wurde mit MTBE versetzt, das Sulfatierungsprodukt wurde fest; Ausbeute: 2.7 g

2.7 g des Pyridinsalzes des Schwefelsäureesters von (1S,3R)-Benzylcyclohexan-1-ol wurden bei 55 °C 2 h in 45 mL THF, 4 mL Wasser und 1 mL konz. Schwefelsäure gerührt. Man verdünnte mit 40 mL Wasser, gab ca. 10 mL MTBE zu, trennte die Phasen und extrahierte die wässrige Phase einmal mit MTBE. Die vereinigten organischen Phasen wurden getrocknet (Na2SO4) und eingedampft; Ausbeute: 640 mg eines hellgelben Öls. Die NMR-Daten sind in Übereinstimmung mit den in Beispiel 16 genannten Daten; die Überprüfung der optischen Reinheit ergab > 99 % ee.

Beispiel 31

Isolierung von (1S,3R)-3-Benzyloxy-cyclohexan-1-ol, Trennung des Gemisches aus Acetat und Alkohol durch Extraktion

10 g des Acetat/Alkohol-Rohgemisches aus Beispiel 29 wurden in ca. 90 mL Methanol und ca. 70 mL Wasser aufgenommen und dreimal mit jeweils ca. 50 mL n-Heptan gewaschen. Die vereinigten Heptan-Phasen (enthalten überwiegend das Acetat) werden mit 50 mL Methanol/Wasser 1:1 extrahiert. Die vereinigten Wasserphasen wurden nochmals mit n-Heptan gewaschen. Nach Einengen der wässrigen Phase erhielt man 3.6 g des gewünschten (1S,3R)-3-Benzyloxy-cyclohexan-1-ols, das Einengen der vereinigten Heptan-Phasen ergab 5.5 g des (1R,3S)-Acetats.

Beispiel 32

Synthese von 4-lodomethyl-2-(4-Fluor-phenyl)-oxazol

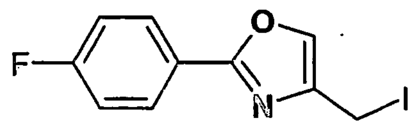

4.0 g (18.9 mmol) 4-Chloromethyl-2-(4-Fluor-phenyl)-oxazol wurden in 80 mL THF gelöst und mit 3.18 g (21.2 mmol) NaI versetzt. Man rührte 3 h bei 20-23 °C und etwa 12 h bei 50 °C, saugte die Salze ab und engte das Filtrat im Vakuum ein, Ausbeute: 6.1 g. Das Produkt kristallisierte; mp 100-102°C; $^1$H-NMR ($CDCl_3$): δ = 4.34 (s, 2 H), 6.97 (dd, 1 H), 7.14 (m, 2 H), 7.68 (s, 1 H), 8.03 (m, 2 H).

Beispiel 33

Synthese von (1S,3R)-4-(3-Benzyloxy-cyclohexyl-1-oxymethy)-2-(4-fluor-phenyl)-oxazol

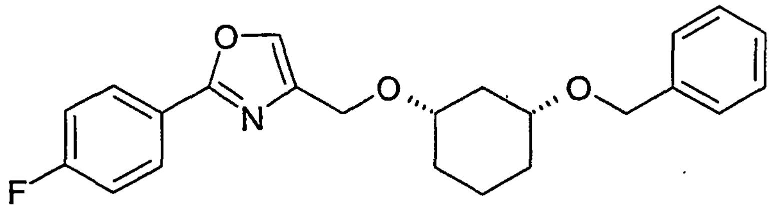

2.0 g (9.7 mmol) (1S,3R)-3-Benzyloxy-cyclohexan-1-ol wurden in 35 mL tBuOMe gelöst. Man gab 1.3 g (ca. 55 %, 43.7 mmol) NaH hinzu und rührte 60 Minuten bei 22 °C. 3.9 g (12.9 mmol) 4-Iodomethyl-2-(4-fluor-phenyl)-oxazol (Beispiel 32) wurden zugegeben und etwa 3 Stunden bei 22-23 °C gerührt. Nach Stehenlassen über Nacht wurde weitere 11 h bei 22-23 °C gerührt. Unter Kühlung gab man Wasser zu (ca. 30 mL) und trennte die organische Phase ab. Trocknen ($Na_2SO_4$), Einengen (Rohausbeute: 4.5 g) und Chromatographie an Kieselgel (Dichlormethan/ Aceton 19:1) ergab 2.4 g des gewünschten cis-konfigurierten, optisch reinen, dialkylierten 1,3-Cyctohexandiolderivates als weißen Feststoff; mp 61-62 °C; $^1$H-NMR ($CDCl_3$), δ = 1.11-1.39 (m, 4 H), 1.82 (m, 1 H), 2.07 (m, 2 H), 2.55 (m, 1 H), 3.38 (m, 2 H), 4.55 (s, 2 H), 4.57 (s, 2 H), 7.13 (m, 2 H), 7.25-7.35 (m, 5 H), 7.63 (s, 1 H), 8.02 (m, 2 H).

Beispiel 34

Synthese von (1 R,3S)-3-[2-(4-Fluor-phenyl)-oxazol-4-ylmethoxy]-cyclohexanol durch Hydrierung

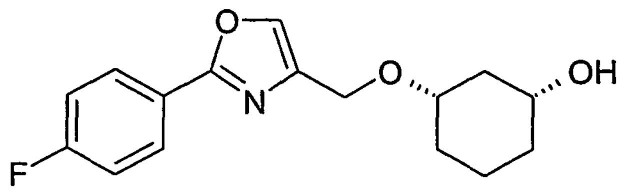

2.4 g (1S,3R)-4-(3-Benzyloxy-cyclohexyl-1-oxymethy)-2-(4-fluor-phenyl)-oxazol wurden in ca. 40 mL Methanol gelöst, mit einer Spatelspitze Pd/C (10%, mit 50 % Wasser) versetzt bei 20-23 °C unter Normaldruck ca. 8 Stunden hydriert. Abfiltrieren des Katalysators und Einengen der verbleibenden Lösung ergab 1.8 g des gewünschten cis-konfigurierten, monoalkylierten 1,3-Cyclohexandiolderivates als Öl, das bei Zugabe von DIPE kristallisierte; Ausbeute 1.6 g; mp 81-82 °C; $^1$H-NMR ($CDCl_3$), δ = 1.25-2.14 (m, 9 H), 3.63 (m, 1 H), 3.75 (m, 1H), 4.55 (dd, 2 H), 7.13 (m, 2 H), 7.64 (s, 1 H), 8.02 (m, 2 H); MS (DCI): 292.3 (100 %).

Beispiel 35

Synthese von (1R,3S)-2-{3-[2-(4-Fluor-phenyl)-oxazol-4-ylmethoxy]-cyclohexyl-1-oxymethyl}-6-methyl-ben-

zoesäuremethylester

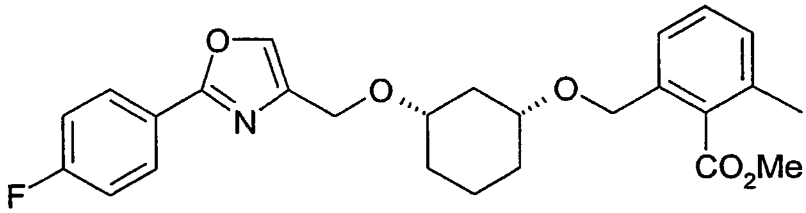

0.8 g (2.75 mmol) (1 R,3S)-3-[2-(4-Fluor-phenyl)-oxazol-4-ylmethoxy]-cyclohexanol (aus Beispiel 34) wurden in 10 mL tBuOMe aufgenommen, mit 0.78 g (6.95 mmol) KOtBu versetzt und ca. 30 Minuten bei 22-27 °C gerührt. Man kühlte auf 0-5 °C ab, gab 1.24 g (ca. 94 %ig, ca. 4.8 mmol) 2-Bromomethyl-6-methyl-benzoesäuremethylester tropfenweise zu, rührte zunächst 2 Stunden bei 3 °C und eine weitere Stunde bei 20 °C. Über Nacht lässt man bei 18 - 21 °C rühren, dann wird das Lösungsmittel abdestilliert. Der Rückstand wird zwischen Wasser und tBuOMe verteilt. Die organische Phase wird getrocknet ($Na_2SO_4$) und im Vakuum eingeengt; Ausbeute: 1.04 g (1R,3S)-2-{3-[2-(4-Fluor-phenyl)-oxazol-4-ylmethoxy]-cyclohexyl-1-oxymethyl}-6-methyl-benzoesäuremethylester als gelbliches Öl; $^1$H-NMR ($CDCl_3$), $\delta$ = 1.15-1.32 (m, 4 H), 1.82 (m, 1 H), 1.98- 2.1 (m, 2 H), 2.34 (s, 3 H), 2.50 (m, 1 H), 3.27 (m, 1 H), 3.39 (m, 1 H), 3.90 (s, 3 H), 4.54 (s, 2 H), 4.60 (s, 2 H), 7.11 - 7.30 (m, 5 H), 7.63 (s, 1 H), 8.02 (m, 2 H).

Beispiel 36

Synthese von (1S,3R)-4-(3-Benzyloxy-cyclohexyl-1-oxymethy)-2-(3-methoxy-phenyl)-5-methyl-oxazol

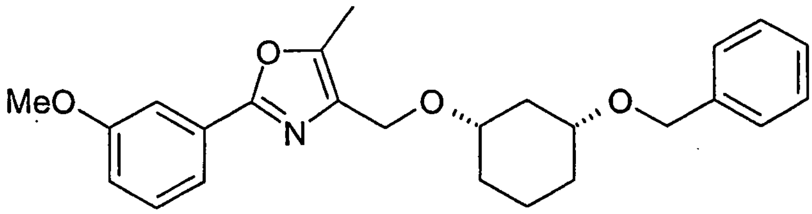

4.6 g (22.3 mmol) (1S,3R)-3-Benzyloxy-cyclohexan-1-ol wurden in 70 mL Chlorbenzol gelöst. Man gab 6.6 g (58.8 mmol) KOtBu hinzu, rührte 30 Minuten bei 22 °C und gab dann 10.3 g (31.3 mmol) 4-lodomethyl-2-(3-methoxy-phenyl)-5-methyl-oxazol zu. Die Temperatur stieg auf 35 °C an. Die Reaktion wurde leicht gekühlt und weitere 2 Stunden bei 22-23 °C gerührt. Nach Abdestillieren des Chlorbenzols im Vakuum wurde der Rückstand zwischen tBuOMe und Wasser verteilt. Die organische Phase wurde getrocknet ($Na_2SO_4$) und im Vakuum eingeengt; Rohausbeute: 10.6 g. Substanz wurde ohne weitere Reinigung in die nächste Reaktion (Hydrierung, s. Beispiel 37) eingesetzt.

Beispiel 37

Synthese von (1 R,3S)-3-[2-(3-Methoxy-phenyl)-oxazol-4-ylmethoxy]-cyclohexanol durch Hydrierung

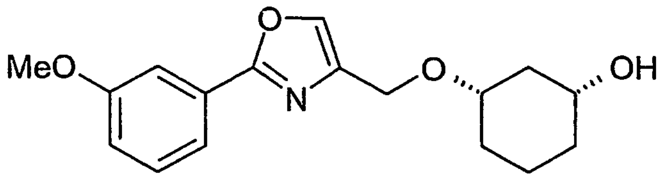

10.5 g (1S,3R)-4-(3-Benzyloxy-cyclohexyl-1-oxymethy)-2-(3-methoxy-phenyl)-oxazol wurden in ca. 120 mL Methanol gelöst, mit 2 g Pd/C (10%, mit 50 % Wasser) versetzt und bei 20-23 °C unter Normaldruck über Nacht hydriert. Abfiltrieren des Katalysators und Einengen der verbleibenden Lösung, Verteilen zwischen MTB-Ether und Wasser und Trocknen

der organischen Phase ergab 6.4 g des gewünschten cis-konfigurierten, monoalkylierten 1,3-Cyclohexandiolderivates als gelbes Öl. 1 g der Substanz wurde an Kieselgel chromatographiert (Essigester): Man erhielt 0.8 g eines farblosen Öls; $^1$H-NMR ($CDCl_3$), δ = 1.25-1.90 (m, 7 H), 2.12 (m, 1 H), 2.41 (s, 3 H), 3.61 (m, 1 H), 3.75 (m, 1 H), 3.87 (s, 3 H), 4.48 (dd, 2 H), 6.96 (d, 1 H), 7.33 (t, 1 H), 7.53 (s, 1 H), 7.58 (d, 1 H); MS (ES+): 318.27 (83%), 243.18 (100 %).

Beispiel 38

Synthese von (1 R,3S)-2-{3-[2-(3-Methoxy-phenyl)-5-methyl-oxazol-4-ylmethoxy]-cyclohexyl-1-oxymethyl}-6-methyl-benzoesäuremethylester

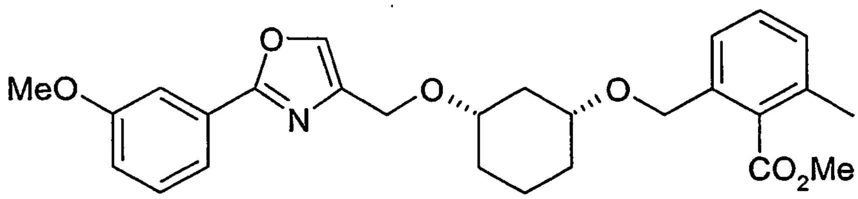

136 mg (0.4 mmol) (1 R,3S)-3-[2-(3-Methoxy-phenyl)-5-methyl-oxazol-4-ylmethoxy]-cyclohexanol (aus Beispiel 37, Hydrierung) wurden in 1 mL Chlorbenzol gelöst, bei 24-27 °C mit 120 mg (1.07 mmol) KOtBu versetzt und ca. 30 Minuten gerührt. Man kühlte auf 0-5 °C ab, gab 189 mg (ca. 94 %ig, ca. 0.78 mmol) 2-Bromomethyl-6-methyl-benzoesäuremethylester tropfenweise zu und rührte zunächst 30 Minuten bei 0-5 °C. Ohne weitere Kühlung hatte die Reaktionsmischung nach 1.5 Stunden eine Temperatur von ca. 20 °C. Nach Stehenlassen über Nacht und Zugabe von ca. 20 mg KOtBu war die Reaktion nach einer weiteren Stunde Rühren bei 22 °C beendet. Abdestillieren des Chlorbenzols im Vakuum, Verteilen des Rückstandes zwischen Wasser und tBuOMe und Trocknen der produkthaltigen organischen Phase ergab nach Einengen im Vakuum 160 mg (1R,3S)-2-{3-[2-(3-Methoxy-phenyl)-5-methyloxazol-4-ylmethoxy]-cyclohexyl-1-oxymethyl}-6-methyl-benzoesäuremethylester als gelbliches Öl; $^1$H-NMR ($CDCl_3$), δ = 1.15-1.32 (m, 4 H), 1.81 (m, 1 H), 2.00 (m, 1 H), 2.06 (m, 1 H), 2.34 (s, 3 H), 2.40 (s, 3 H), 2.51 (m, 1 H), 3.27 (m, 1 H), 3.36 (m, 1 H), 3.87 (s, 3 H), 3.90 (m, 3 H), 4.48 (s, 2 H), 4.60 (s, 2 H), 6.96 (m, 1 H), 7.12-7.35 (m, 4 H), 7.53-7.60 (m, 2 H).

**Zu Schema IIIb**

Beispiel 39

Synthese von Glutarsäure-mono-(3-benzyloxy-cyclohexyl)-ester

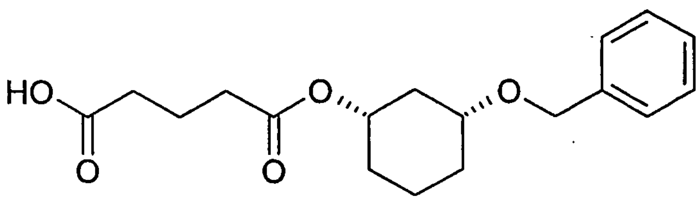

rac.

3.0 g 3-Benzyloxy-cyclohexan-1-ol, 2.15 g Glutarsäureanhydrid und 3.03 g Triethylamin wurden in 25 mL Methylenchlorid bei 21-23 °C gerührt. Nach vollständigem Umsatz gab man auf Wasser, schüttelte aus und trocknete mit $MgSO_4$. Nach Einengen im Vakuum erhielt man 4.3 g der gewünschten Verbindung; $^1$H-NMR ($CDCl_3$), δ = 1.20-1.28 (m, 4 H), 1.82 (m, 1 H), 1.90-1.97 (m, 3 H), 2.05 (m, 1 H), 2.32-2.42 (m, 5 H), 3.39 (m, 1 H), 4.55 (dd, 2 H), 4.69 (m, 1 H), 7.25-7.33 (m, 5 H), 8.7 (br., 1 H).

Beispiel 40

Stereoselektive Hydrolyse von Glutarsäure-mono-(3-benzyloxy-cyclohexyl)-ester, Herstellung von (1 R,3S)-3-Benzyloxy-cyclohexan-1-ol

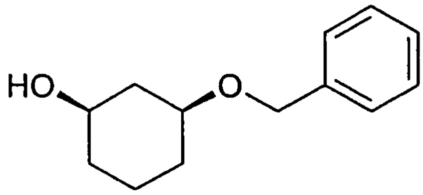

20 mg racemischer Glutarsäure-mono-(3-benzyloxy-cyclohexyl)-ester (aus Beispiel 39) wurden in 2 mL Phosphatpuffer, pH 8, und 3-5 Tropfen DME verteilt, mit 3-5 mg Novozym 435 versetzt und bei 21-23 °C gerührt. Nach ca. 50 % Umsatz wurde die Reaktionslösung zwischen gesättigter wässriger $NaHCO_3$-Lösung und Essigester verteilt. Die Essigester-Phase wurde getrocknet und eingeengt, Ausbeute: 5 mg (1 R,3S)-3-Benzyloxy-cyclohexan-1-ol, der Enantiomerenüberschuß betrug >95 % (HPLC an Chiralpak AD-H 250 x 4.6; 1 mL/min, Heptan/EtOH/$CH_3CN$ 25:1:0.5 + 0.1 % TFA).

Beispiel 41

Synthese von (1 R,3S)-4-(3-Benzyloxy-cyclohexyl-1-oxymethy)-2-(4-fluor-phenyl)-oxazol

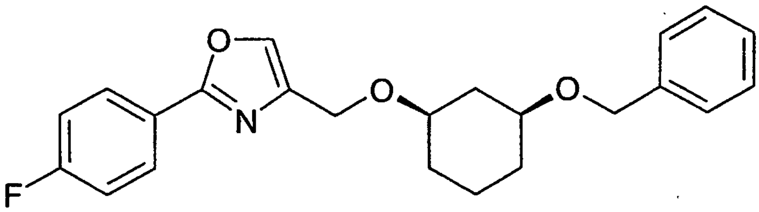

Ausgehend von (1R,3S)-3-Benzyloxy-cyclohexan-1-ol (s. Beispiel 40) gelangt man durch Alkylierung mit 4-Iodomethyl-2-(4-fluor-phenyl)-oxazol (s. Beispiel 32) zu (1 R,3S)-4-(3-Benzyloxy-cyclohexyl-1-oxymethy)-2-(4-fluor-phenyl)-oxazol vgl. Beispiel 33.

**Weitere Beispiele für die Alkylierung von cis-1,3-Cyclohexandiol**

Beispiel 42

Synthese von racemischem cis-2-(3-Hydroxy-cyclohexyloxymethyl)-6-methyl-benzoesäuremethylester

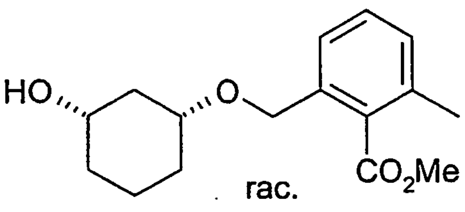

5 g (42.8 mmol) cis-1,3-Cyclohexandiol werden in 50 mL Dimethoxyethan (DME) gelöst, mit 3.36 g (30 mmol) Kalium-tert.-butylat (KOtBu) bei 20-23 °C versetzt und gerührt. Nach etwa 30 Minuten wird auf 5 °C gekühlt und 3.7 g (ca. 50 %ig) 2-Bromomethyl-6-methyl-benzoesäuremethylester, den man z. B. durch Methanolyse des Säurebromids (2-Bromomethyl-6-methyl-benzoesäurebromid) oder durch Bromierung von 2,6-Dimethylbenzoesäuremethylester herstellen kann, zugetropft. Man rührt 1 h bei 5-10 °C und dann über Nacht bei 20-23 °C. Man gibt Wasser und Methyl-tert.-butylether (MTBE) zu, rührt kräftig, trennt die Phasen, wäscht die wässrige Phase nochmals mit MTBE und engt die vereinigten organischen Phasen im Vakuum ein. Der Rückstand wird an Kieselgel chromatographiert (Essigsäure-ethylester/n-Heptan 1:1). Man erhält 600 mg der gewünschten Verbindung als leicht gelbes Öl, $^1H$-NMR ($CDCl_3$), $\delta$ = 1.27 (m, 1 H), 1.45 (m, 1 H), 1.55 (m, 1 H), 1.74 (m, 1 H), 1.83 (m, 1 H), 2.05 (m, 1 H), 2.34 (s, 3 H), 3.47 (m, 1 H), 3.72 (m, 1 H), 3.91 (s, 3 H), 4.58 (dd, 2 H), 7.15 (d, 1 H), 7.20 (d, 2 H), 7.27 (m, 1 H).

Beispiel 43

Synthese von racemischem cis-2-(3-Hydroxy-cyclohexyloxymethyl)-6-methyl-benzoesäuremethylester

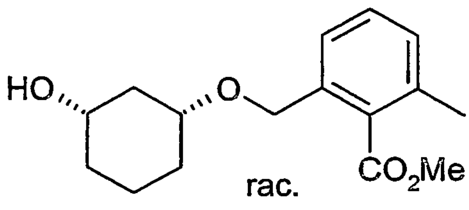

rac.

10.0 g (86 mmol) cis-1,3-Cyclohexandiol wurden in 150 mL Methyl-tert.-butylether (MTBE) aufgenommen, mit 6.72 g (59.9 mmol) Kalium-tert.-butylat (KOtBu) bei ca. 20°C versetzt und gerührt. Nach etwa 30 Minuten wurde die Suspension auf 5 °C abgekühlt und mit 7.4 g (ca. 50 %ig) 2-Bromomethyl-6-methyl-benzoesäuremethylester, den man z. B. durch Methanolyse des Säurebromids (2-Bromomethyl-6-methyl-benzoesäurebromid) oder durch Bromierung von 2,6-Dimethylbenzoesäuremethylester herstellen kann, tropfenweise versetzt. Man rührte 1 h bei 0-5 °C, erwärmte auf 20-23 °C und ließ über Nacht rühren. Man gab Wasser zu, rührte kräftig, trennte die Phasen, wusch die organische Phase nochmals mit Wasser und engte dann die organische Phase im Vakuum ein. Der Rückstand (4.6 g) wurde an Kieselgel chromatographiert (Essigsäureethylester/n-Heptan 1:1). Man erhielt 1.2 g der gewünschten Verbindung als leicht gelbes Öl, $^{1}$H-NMR ($CDCl_3$), $\delta$ = 1.27 (m, 1 H), 1.45 (m, 1 H), 1.55 (m, 1 H), 1.74 (m, 1 H), 1.82 (m, 1 H), 2.05 (m, 1 H), 2.34 (s, 3 H), 3.46 (m, 1 H), 3.72 (m, 1 H), 3.91 (s, 3 H), 4.58 (dd, 2 H), 7.15 (d, 1 H), 7.20 (d, 2 H), 7.27 (m, 1 H).

Beispiel 44

Synthese von racemischem cis-2-(3-Hydroxy-cyclohexyloxymethyl)-6-methyl-benzoesäuremethylester

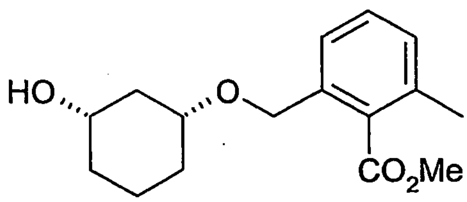

rac.

5 g (42.8 mmol) cis-1,3-Cyclohexandiol wurden in 40 mL Chlorbenzol und 10 mL 1,3-Dimethyl-3,4,5,6-tetrahydro-2 (1H)-pyrimidinon (DMPU, Dimethylpropylen-harnstoff) gelöst, mit 3.36 g (30 mmol) Kalium-tert.-butylat (KOtBu) bei 20-23 °C versetzt und gerührt. Nach 10-15 Minuten wurde auf 15-20°C gekühlt und 3.7 g (ca. 50 %ig) 2-Bromomethyl-6-methyl-benzoesäuremethylester zugetropft. Man rührte 1.5 h bei 20 °C und gab dann auf Wasser. Die organische Phase Wurde abgetrennt und unter vermindertem Druck eingeengt. Der Rückstand wurde in NMP/Wasser aufgenommen und zur Entfernung von Verunreinigungen zweimal mit n-Heptan gewaschen. Anschließend wurde zur Isolierung des Produktes zweimal mit MTBE extrahiert. Die vereinigten MTBE-Phasen wurden mit Wasser gewaschen, getrocknet ($Na_2SO_4$) und im Vakuum eingeengt. Der Rückstand (1.2 g) wurde an Kieselgel chromatographiert (Essigsäureethylester/n-Heptan 1:1). Man erhielt 580 mg der gewünschten Verbindung als leicht gelbes Öl; $^{1}$H-NMR ($CDCl_3$), $\delta$ = 1.27 (m, 1 H), 1.45 (m, 1 H), 1.55 (m, 1 H), 1.74 (m, 1 H), 1.83 (m, 1 H), 2.05 (m, 1 H), 2.34 (s, 3 H), 3.47 (m, 1 H), 3.72 (m, 1 H), 3.91 (s, 3 H), 4.58 (dd, 2 H), 7.15 (d, 1 H), 7.20 (d, 2 H), 7.27 (m, 1 H).

**Weitere Beispiele für die Racematspaltung durch stereoselektive enzymatische Esterbildung (EB)**

Beispiel 45

Racematspaltung von cis-2-(3-Hydroxy-cyclohexyloxymethyl)-6-methyl-benzoesäuremethylester

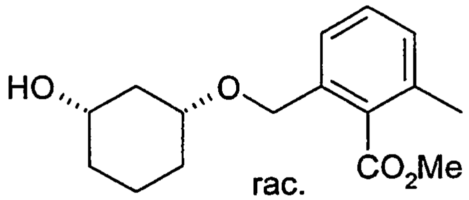

730 mg des racemischen cis 2-(3-Hydroxy-cyclohexyl-1-oxymethyl)-6-methylbenzoesäuremethylesters werden in 5 mL Methylenchlorid und 2 mL Vinylacetat gelöst, auf 38 °C erwärmt und mit 100 mg Novozym 435 versetzt.
Nach ca. 5 h wurde die Reaktion durch Abfiltrieren des Enzyms beendet und die optische Reinheit des gebildeten Acetats und des nicht umgesetzten Alkohols per HPLC ($HPLC_{Acetat}$: Chiralcel OD 250 x 4.6, 1 mL/min, Heptan/EtOH/$CH_3CN$ 100:1:0.5; $HPLC_{Alkohol}$: Chiralpak AD 250 x 4.6; 1 mL/min, Heptan/EtOH/$CH_3CN$ 25:1:0.5 + 0.1 % TFA) bestimmt. Die Bestimmung der optischen Reinheit ergab für (3S,1R)-2-(3-Hydroxy-cyclohexyl-1-oxymethyl)-6-methyl-benzoesäuremethylester 98 % ee und für das (3R,1S)-Acetat 86 % ee.

Beispiel 46

Racematspaltung von cis-2-(3-Hydroxy-cyclohexyl-1-oxymethyl)-6-methyl-benzoesäuremethylester

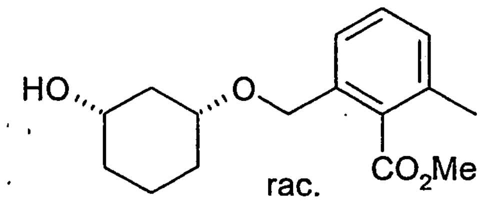

20 mg des racemischen cis-2-(3-Hydroxy-cyclohexyl-1-oxymethyl)-6-methylbenzoesäuremethylesters wurden in 2 mL Chlorbenzol und 1 mL Vinylacetat gelöst, bei 22-25 °C mit 8 mg Chirazyme L-2, lyo. (Roche), versetzt und gerührt. Nach ca. 6 h wurde die Reaktion durch Abfiltrieren des Enzyms beendet und die optische Reinheit des gebildeten Acetats und des nicht umgesetzten Alkohols per HPLC ($HPLC_{Acetat}$: Chiralcel OD 250 x 4.6, 1 mL/min, Heptan/EtOH/$CH_3CN$ 100:1:0.5; $HPLC_{Alkohol}$: Chiralpak AD 250 x 4.6; 1 mUmin, Heptan/EtOH/$CH_3CN$ 25:1:0.5 + 0.1 % TFA) bestimmt: (3S, 1R)-2-(3-Hydroxy-cyclohexyl-1-oxymethyl)-6-methyl-benzoesäuremethylester 84 % ee und das (3R,1S)-Acetat 95 % ee.

Beispiel 47

Racematspaltung von cis-2-(3-Hydroxy-cyclohexyl-1-oxymethyl)-6-methyl-benzoesäuremethylester

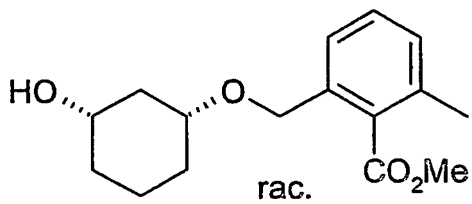

1.0 g cis-2-(3-Hydroxy-cyclohexyl-1-oxymethyl)-6-methyl-benzoesäuremethylester wurden in 10 mL 1,2-Dichlorethan und 2 mL Vinylpropionat gelöst, mit 25 mg Chirazyme L-2, lyo. (Roche) versetzt und 40 h bei 21-24 °C gerührt. Abfiltrieren des Enzyms, Einengen des Filtrats im Vakuum und Chromatographie des Rückstandes an Kieselgel (Essigsäureethylester/n-Heptan 1:1) ergab 0.49 g des (3R,1S)-Propionats mit 92 % ee (HPLC an Chiralcel OD 250 x 4.6, 1 mL/min, Heptan/EtOH/$CH_3CN$ 100:1:0.5), $^1$H-NMR ($CDCl_3$), $\delta$ = 1.13 (t, 3 H), 1.15-1.36 (m, 4 H), 1.79 (m, 1H), 1.91 (m, 1 H), 2.01 (m, 1 H), 2. 30 (q, 2 H), 2.34 (s, 3H), 2.35 (m, 1 H), 3.34 (m, 1 H), 3.90 (s, 3 H), 4.58 (dd, 2 H), 4.67 (m, 1 H), 7.14

(d, 1 H), 7.19 (d, 1 H) 7.26 (m, 1 H), sowie 0.3 g des nicht umgesetzten (3S,1R)-2-(3-Hydroxy-cyclohexyl-1-oxymethyl)-6-methylbenzoesäuremethylesters mit 98 % ee (HPLC an Chiralpak AD-H 250 x 4.6; 1 mL/min, Heptan/EtOH/$CH_3CN$ 25:1:0.5 + 0.1 % TFA), $^1H$-NMR ($CDCl_3$), δ = 1.27 (m, 1 H), 1.45 (m, 1 H), 1.55 (m, 1 H), 1.74 (m, 1 H), 1.83 (m, 1 H), 2.05 (m, 1H), 2.34 (s, 3 H), 3.47 (m, 1 H), 3.72 (m, 1 H), 3.91 (s, 3 H), 4.58 (dd, 2 H), 7.15 (d, 1 H), 7.20 (d, 2 H), 7.27 (m, 1 H).

Beispiel 48

Racematspaltung von cis-2-(3-Hydroxy-cyclohexyl-1-oxymethyl)-6-methyl-benzoesäuremethylester

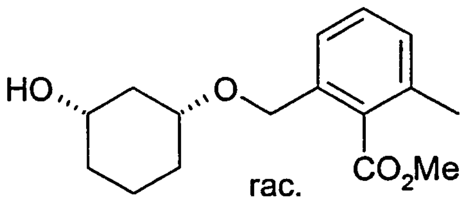

rac.

10 mg des racemischen cis 2-(3-Hydroxy-cyclohexyl-1-oxymethyl)-6-methylbenzoesäuremethylesters wurden in 1 mL Vinylacetat gelöst, mit ca. 4-6 mg Lipase TL (Pseud. stutzeri, Meito Sangyo) versetzt und bei 22-25 °C gerührt. Nach > 50 % Umsatz wurde die Reaktion durch Abfiltrieren des Enzyms beendet und die optische Reinheit des nicht umgesetzten (3S,1R)-2-(3-Hydroxy-cyclohexyl-1-oxymethyl)-6-methyl-benzoesäuremethylesters bestimmt: > 98 % ee (HPLC an Chiralpak AD 250 x 4.6; 1 mL/min, Heptan/EtOH/$CH_3CN$ 25:1:0.5 + 0.1 % TFA).

Beispiel 49

Racematspaltung von cis-2-(3-Hydroxy-cyclohexyloxymethyl)-6-methyl-benzoesäuremethylester

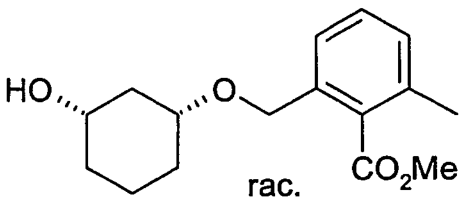

rac.

3.9 g des racemischen cis-2-(3-Hydroxy-cyclohexyl-1-oxymethyl)-6-methylbenzoesäuremethylesters wurden in 25 mL Methylenchlorid und 10 mL Vinylacetat gelöst, auf 45 °C erwärmt und mit 250 mg Novozym 435 versetzt.

Nach ca. 45 % Umsatz wurde die Reaktion durch Abfiltrieren des Enzyms beendet und die Reaktionsmischung eingeengt. Chromatographie des Rückstandes an Kieselgel (Essigsäureethylester/n-Heptan 1:1) ergab 1.9 g des (3R, 1S)-Acetats (> 95 % ee, HPLC an Chiralcel OD 250 x 4.6, 1 mL/min, Heptan/EtOH/$CH_3CN$ 100:1:0.5). und 1.9 g des nicht umgesetzten (3S,1R)-2-(3-Hydroxy-cyclohexyl-1-oxymethyl)-6-methyl-benzoesäuremethylesters (82 % ee, HPLC an Chiralpak AD 250 x 4.6; 1 mL/min, Heptan/EtOH/$CH_3CN$ 25:1:0.5 + 0.1 % TFA).

Beispiel 50

Racematspaltung von cis-2-(3-Hydroxy-cyclohexyloxymethyl)-6-methyl-benzoesäuremethylester

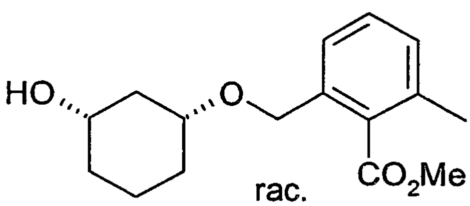

rac.

20 mg des racemischen cis-2-(3-Hydroxy-cyclohexyl-1-oxymethyl)-6-methylbenzoesäuremethylesters wurden in 2 mL Toluol und 1 mL Vinylacetat gelöst, bei 20-23 °C mit 6-8 mg Chirazyme L-2, lyo. (Roche), versetzt und gerührt. Nach ca. 45 % Umsatz wurde die Reaktion durch Abfiltrieren des Enzyms beendet und die optische Reinheit des gebildeten (3R,1S)-Acetats bestimmt: 94 % ee (HPLC an Chiralcel OD 250 x 4.6, 1 mUmin, Heptan/EtOH/$CH_3CN$ 100:1:0.5).

Beispiel 51

Racematspaltung von cis-2-(3-Hydroxy-cyclohexyloxymethyl)-6-methyl-benzoesäuremethylester

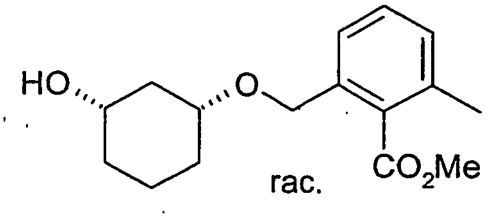

10 mg des racemischen cis-2-(3-Hydroxy-cyclohexyl-1-oxymethyl)-6-methylbenzoesäuremethylesters wurden in 1 mL Vinylacetat gelöst, mit ca. 4-6 mg Lipase QL(Alcaligenes spec., Meito Sangyo) versetzt und bei 20-23 °C gerührt. Nach ca. 52 % Umsatz wurde die Reaktion durch Abfiltrieren des Enzyms beendet und die optische Reinheit des gebildeten Acetats und des nicht umgesetzten Alkohols bestimmt, ee des Acetats: 91 % (HPLC an Chiralcel OD 250 x 4.6, 1 mL/min, Heptan/EtOH/$CH_3CN$ 100:1:0.5), ee des (3S,1R)-2-(3-Hydroxy-cyclohexyl-1-oxymethyl)-6-methyl-benzoesäuremethylesters: > 98 % ee (HPLC an Chiralpak AD 250 x 4.6; 1 mL/min, Heptan/EtOH/$CH_3CN$ 25:1:0.5 + 0.1 % TFA).

Beispiel 52

Racematspaltung von cis-2-(3-Hydroxy-cyclohexyloxymethyl)-6-methyl-benzoesäuremethylester

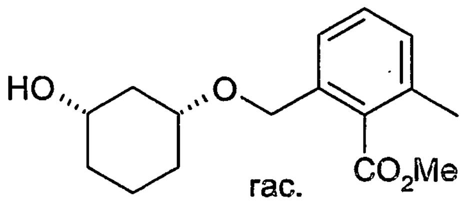

10 mg des racemischen cis-2-(3-Hydroxy-cyclohexyl-1-oxymethyl)-6-methylbenzoesäuremethylesters wurden in 1 mL Vinylacetat gelöst, mit ca. 4-6 mg Lipase SL (Pseud. cepacia, Meito Sangyo) versetzt und bei 20-23 °C gerührt. Nach ca. 52 % Umsatz wurde die Reaktion durch Abfiltrieren des Enzyms beendet und die optische Reinheit des gebildeten Acetats und des nicht umgesetzten Alkohols bestimmt, ee des Acetats: 90 % (HPLC an Chiralcel OD 250 x 4.6, 1 mL/min, Heptan/EtOH/$CH_3CN$ 100:1:0.5), ee (3S,1R)-2-(3-Hydroxy-cyclohexyl-1-oxymethyl)-6-methyl-benzoesäuremethylesters: > 95 % ee (HPLC an Chiralpak AD 250 x 4.6; 1 mL/min, Heptan/EtOH/$CH_3CN$ 25:1:0.5 + 0.1 % TFA).

Beispiel 53

Racematspaltung von cis-2-(3-Hydroxy-cyclohexyloxymethyl)-6-methyl-benzoesäuremethylester

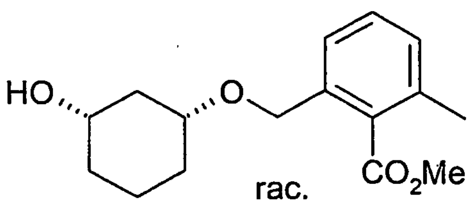

39 g cis-2-(3-Hydroxy-cyclohexyl-1-oxymethyl)-6-methyl-benzoesäuremethylester wurden in 250 mL Methylenchlorid und 50 mL Vinylacetat gelöst, auf 45 °C erwärmt und mit 1.0 g Novozym 435 versetzt. Nach 25 h wurden weitere 0.5 g Novozym 435 zugegeben. Nach weitere 6.5 h wurde das Enzym abfiltriert und die Reaktionsmischung eingeengt. Chromatographie des Rückstandes an 630 g Kieselgel (Essigsäureethylester/n-Heptan 1:1) ergab 18.2 g (3S,1R)-2-(3-Hydroxy-cyclohexyl-1-oxymethyl)-6-methyl-benzoesäuremethylester (> 98 % ee, HPLC an Chiralpak AD-H 250 x 4.6; 1 mL/min, Heptan/EtOH/$CH_3CN$ 25:1:0.5 + 0.1 % TFA), $^1$H-NMR ($CDCl_3$), $\delta$ = 1.27 (m, 1 H), 1.45 (m, 1 H), 1.55 (m, 1 H), 1.74 (m, 1 H), 1.83 (m, 1 H), 2.05 (m, 1 H), 2.34 (s, 3 H), 3.47 (m, 1 H), 3.72 (m, 1 H), 3.91 (s, 3 H), 4.58 (dd, 2 H), 7.15 (d, 1 H), 7.20 (d, 2 H), 7.27 (m, 1 H).

Beispiel 54

Racematspaltung von cis-2-(3-Hydroxy-cyclohexyloxymethyl)-6-methyl-benzoesäuremethylester

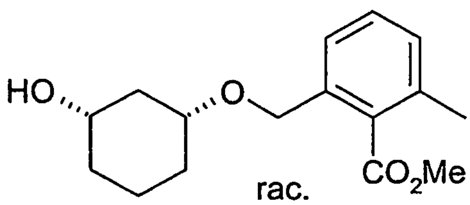

20 mg des racemischen cis-2-(3-Hydroxy-cyclohexyl-1-oxymethyl)-6-methylbenzoesäuremethylesters wurden in 2 mL THF und 1 mL Vinylacetat gelöst, bei 20-23 °C mit 6-8 mg Chirazyme L-2, lyo. (Roche), versetzt und gerührt. Nach ca. 6 h wurde die Reaktion durch Abfiltrieren des Enzyms beendet und die optische Reinheit des gebildeten Acetats und des nicht umgesetzten Alkohols per HPLC ($HPLC_{Acetat}$: Chiralcel OD 250x4.6, 1 mL/min, Heptan/EtOH/$CH_3CN$ 100:1: 0.5; $HPLC_{Alkohol}$: Chiralpak AD 250 x 4.6; 1 mUmin, Heptan/EtOH/$CH_3CN$ 25:1:0.5 + 0.1 % TFA) bestimmt: ee des (3S, 1R)-2-(3-Hydroxy-cyclohexyl-1-oxymethyl)-6-methylbenzoesäuremethylesters 89 % und ee des (3R,1S)-Acetats 95 % und

Beispiel 55

Racematspaltung von cis-2-(3-Hydroxy-cyclohexyloxymethyl)-6-methyl-benzoesäuremethylester

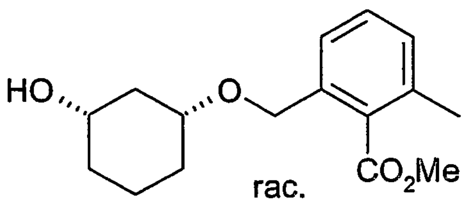

Ca. 15 mg des racemischen cis-2-(3-Hydroxy-cyclohexyl-1-oxymethyl)-6-methylbenzoesäuremethylesters wurden in 2 mL tert.-Butanol und 1 mL Vinylacetat gelöst, bei 20-23 °C mit ca. 6 mg Novozym 435 versetzt und gerührt. Nach ca. 24 h wurde die Reaktion durch Abfiltrieren des Enzyms beendet und die optische Reinheit des gebildeten Acetats und des nicht umgesetzten Alkohols per HPLC bestimmt: (3R,1S)-Acetat 91 % ee (HPLC: Chiralcel OD 250 x 4.6, 1 mL/min, Heptan/EtOH/$CH_3CN$ 100:1:0.5), (3S,1R)-2-(3-Hydroxy-cyclohexyl-1-oxymethyl)-6-methyl-benzoesäuremethylester 96 % ee (HPLC: Chiralpak AD 250 x 4.6; 1 mL/min, Heptan/EtOH/$CH_3CN$ 25:1:0.5 + 0.1 % TFA).

Beispiel 56

Racematspaltung von cis-2-(3-Hydroxy-cyclohexyloxymethyl)-6-methyl-benzoesäuremethylester

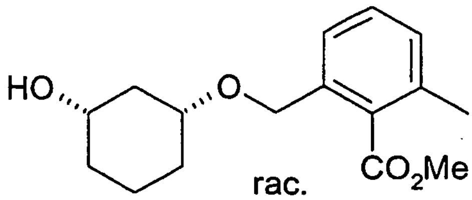

rac.

10 mg des racemischen cis-2-(3-Hydroxy-cyclohexyl-1-oxymethyl)-6-methylbenzoesäuremethylesters wurden in 1 mL Vinylacetat gelöst, mit ca. 4-6 mg Lipase TL (Pseud. stutzeri, Meito Sangyo) versetzt und bei 20-23 °C gerührt. Nach > 50 % Umsatz wurde die Reaktion durch Abfiltrieren des Enzyms beendet und die optische Reinheit des nicht umgesetzten Alkohols bestimmt: (3S,1R)-2-(3-Hydroxy-cyclohexyl-1-oxymethyl)-6-methyl-benzoesäuremethylester > 98 % ee (HPLC an Chiralpak AD 250 x 4.6; 1 mL/min, Heptan/EtOH/$CH_3CN$ 25:1:0.5 + 0.1 % TFA).

Beispiel 57

Racematspaltung von cis-3-Benzyloxy-cyclohexan-1-ol

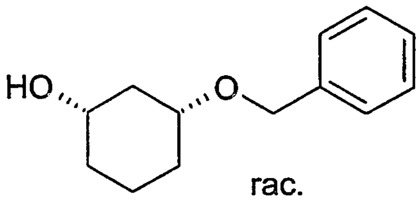

rac.

35-40 mg racemisches cis-3-Benzyloxy-cyclohexan-1-ol wurden in 0.5-1 mL Vinylacetat und 2-3 mL Methylenchlorid gelöst, mit ca. 8-10 mg Novozym 435 versetzt und bei 22-25 °C gerührt. Nach 4 Tagen wurde die Reaktion durch Abfiltrieren des Enzyms beendet. Die optische Reinheit des Alkohols (1S,3R)-3-Benzyloxycyclohexan-1-ol betrug > 98 % ee (HPLC an Chiralpak AD-H 250 x 4.6; 1 mL/min, Heptan/EtOH/$CH_3CN$ 25:1:0.5 + 0.1 % TFA), der ee des (1R, 3S)-Acetats war 82 % (HPLC an Chiralcel OD 250 x 4.6; 1 mL/min, Heptan/EtOH/$CH_3CN$ 100:1:0.5).

Beispiel 58

Racematspaltung von cis-3-Benzyloxy-cyclohexan-1-ol

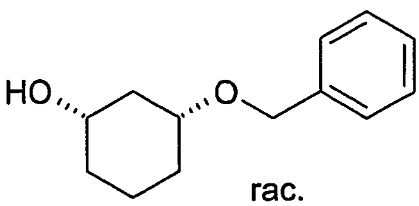

rac.

10 mg des racemischen cis-3-Benzyloxy-cyclohexan-1-ols wurden in 1 mL Vinylacetat und 3 mL THF gelöst, mit ca. 5 mg Lipase L-10 versetzt und bei 22-25 °C gerührt. Nach ≥50 % Umsatz wurde die Reaktion durch Abfiltrieren des Enzyms beendet. Die optische Reinheit von (1S,3R)-3-Benzyloxy-cyclohexan-1-ol betrug ≥90 % ee (HPLC an Chiralpak AD-H 250 x 4.6; 1 mL/min, Heptan/EtOH/$CH_3CN$ 25:1:0.5 + 0.1 % TFA).

Beispiel 59

Racematspaltung von cis-3-Benzyloxy-cyclohexan-1-ol

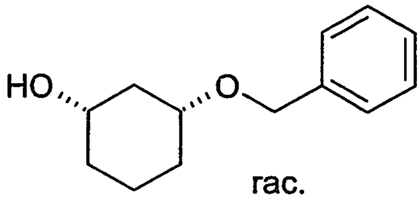

rac.

10 mg racemisches cis-3-Benzyloxy-cyclohexan-1-ol wurden in 1 mL Vinylacetat und 3 mL Chlorbenzol gelöst, mit 10 mg Novozym 435 versetzt und bei 22-25 °C gerührt. Nach 4 Stunden wurde die Reaktion durch Abfiltrieren des Enzyms beendet. Die optische Reinheit des Alkohols (1S,3R)-3-Benzyloxy-cyclohexanol betrug 68 % ee (HPLC an Chiralpak AD-H 250 x 4.6; 1 mL/min, Heptan/EtOH/$CH_3CN$ 25:1:0.5 + 0.1 % TFA), der ee des enantiomeren Acetats war 95 % (HPLC an Chiralcel OD 250 x 4.6; 1 mL/min, Heptan/EtOH/$CH_3CN$ 100:1:0.5).

Beispiel 60

Racematspaltung von cis-3-Benzyloxy-cyclohexan-1-ol

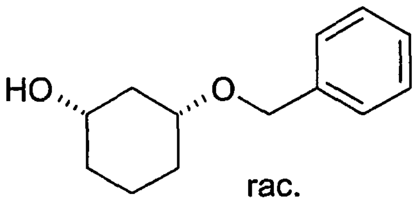

rac.

10 mg racemisches cis-3-Benzyloxy-cyclohexan-1-ol wurden in 1 mL Vinylacetat und 3 mL Cyclohexan gelöst, mit ca. 5 mg Lipase QL versetzt und bei 22-25 °C gerührt. Nach 24 Stunden wurde die Reaktion durch Abfiltrieren des Enzyms beendet. Die optische Reinheit von (1S,3R)-3-Benzyloxy-cyclohexan-1-ol betrug 94 % ee (HPLC an Chiralpak AD-H 250 x 4.6; 1 mUmin, Heptan/EtOH/$CH_3CN$ 25:1:0.5 + 0.1 % TFA).

Beispiel 61

Racematspaltung von cis-3-Benzyloxy-cyclohexan-1-ol

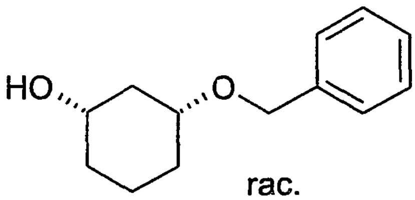

rac.

10 mg des racemischen cis-3-Benzyloxy-cyclohexan-1-ols wurden in 1 mL Vinylacetat und 3 mL Toluol gelöst, mit 10 mg Novozym 435 versetzt und bei 22-25 °C gerührt. Nach 4 Stunden wurde die Reaktion durch Abfiltrieren des Enzyms beendet. Die optische Reinheit von (1S,3R)-3-Benzyloxy-cyclohexan-1-ol betrug 70 % ee (HPLC an Chiralpak AD-H 250 x 4.6; 1 mUmin, Heptan/EtOH/$CH_3CN$ 25:1:0.5 + 0.1 % TFA), der ee des (1R,3S)-Acetats war 95 % (HPLC an Chiralcel OD 250 x 4.6; 1 mL/min, Heptan/EtOH/$CH_3CN$ 100:1:0.5).

Beispiel 62

Racematspaltung von cis-3-Benzyloxy-cyclohexan-1-ol

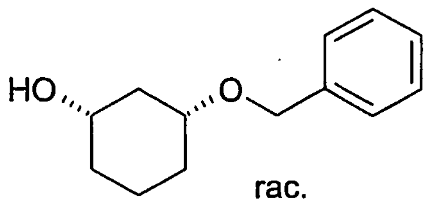

10 mg des racemischen cis-3-Benzyloxy-cyclohexan-1-ols wurden in 1 mL Vinylacetat und 3 mL Cyclohexan gelöst, mit ca. 10 mg Novozym 435 versetzt und bei 22-25 °C gerührt. Nach ca. 4 Stunden wurde die Reaktion durch Abfiltrieren des Enzyms beendet. Die optische Reinheit von (1S,3R)-3-Benzyloxy-cyclohexan-1-ol betrug 95 % ee (HPLC an Chiralpak AD-H 250 x 4.6; 1 mL/min, Heptan/EtOH/$CH_3CN$ 25:1:0.5 + 0.1 % TFA), der ee des (1R,3S)-Acetats war 90 % (HPLC an Chiralcel OD 250 x 4.6; 1 mL/min, Heptan/EtOH/$CH_3CN$ 100:1:0.5).

Beispiel 63

Racematspaltung von cis-3-Benzyloxy-cyclohexan-1-ol

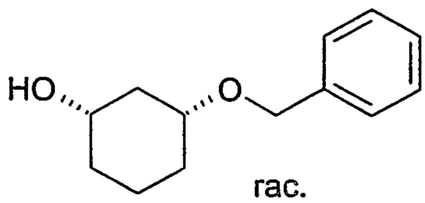

10 mg racemisches cis-3-Benzyloxy-cyclohexan-1-ol wurden in 1 mL Vinylacetat und 3 mL Cyclohexan gelöst, mit ca. 5 mg Lipase L-10 versetzt und bei 22-25 °C gerührt. Nach 24 Stunden wurde die Reaktion durch Abfiltrieren des Enzyms beendet. Die optische Reinheit von (1S,3R)-3-Benzyloxy-cyclohexan-1-ol betrug
> 95 % ee (HPLC an Chiralpak AD-H 250 x 4.6; 1 mL/min, Heptan/EtOH/$CH_3CN$ 25:1:0.5 + 0.1 % TFA).

Beispiel 64

Racematspaltung von cis-3-Benzyloxy-cyclohexan-1-ol

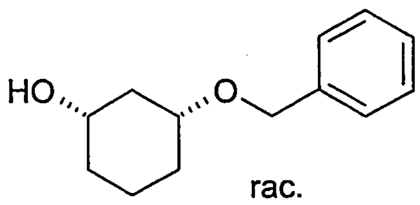

Ca. 10 mg des racemischen cis-3-Benzyloxy-cyclohexan-1-ols wurden in 1 mL Vinylacetat und 3 mL THF gelöst, mit 10 mg Novozym 435 versetzt und bei 22-25 °C gerührt. Nach 4 Stunden wurde die Reaktion durch Abfiltrieren des Enzyms beendet. Die optische Reinheit von (1S,3R)-3-Benzyloxy-cyclohexanol betrug 73 % ee (HPLC an Chiralpak AD-H 250 x 4.6; 1 mL/min, Heptan/EtOH/ $CH_3CN$ 25:1:0.5 + 0.1 % TFA), der ee des (1R, 3S)-Acetats war 94 % (HPLC an Chiralcel OD 250 x 4.6; 1 mL/min, Heptan/EtOH/$CH_3CN$ 100:1:0.5).

Beispiel 65

Racematspaltung von cis-3-Benzyloxy-cyclohexan-1-ol

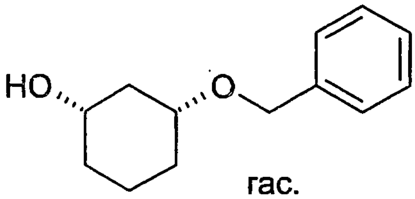

rac.

10 mg racemisches cis-3-Benzyloxy-cyclohexan-1-ol wurden in 1 mL Vinylacetat und 3 mL Chlorbenzol gelöst, mit ca. 5 mg Lipase L-10 versetzt und bei 22-25 °C gerührt. Nach ≥50 % Umsatz wurde die Reaktion durch Abfiltrieren des Enzyms beendet. Die optische Reinheit von (1S,3R)-3-Benzyloxy-cyclohexan-1-ol betrug ≥92 % ee (HPLC an Chiralpak AD-H 250 x 4.6; 1 mL/min, Heptan/EtOH/$CH_3CN$ 25:1:0.5 + 0.1 % TFA).

Beispiel 66

Racematspaltung von cis-3-Benzyloxy-cyclohexan-1-ol

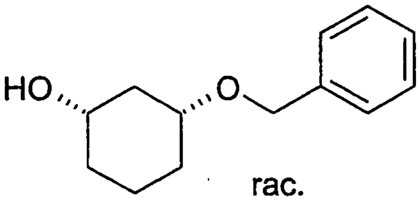

rac.

Ca. 10 mg des racemischen cis-3-Benzyloxy-cyclohexan-1-ols wurden in 1 mL Vinylacetat und 3 mL Essigsäureethylester gelöst, mit ca. 10 mg Novozym 435 versetzt und bei 22-25 °C gerührt. Nach 4 Stunden wurde die Reaktion durch Abfiltrieren des Enzyms beendet. Die optische Reinheit von (1S,3R)-3-Benzyloxycyclohexan-1-ol betrug 77 % ee (HPLC an Chiralpak AD-H 250 x 4.6; 1 mL/min, Heptan/EtOH/$CH_3CN$ 25:1:0.5 + 0.1 % TFA), der ee des (1R,3S)-Acetats war 93 % (HPLC an Chiralcel OD 250 x 4.6; 1 mL/min, Heptan/EtOH/$CH_3CN$ 100:1:0.5).

Beispiel 67

Racematspaltung von cis-3-Benzyloxy-cydohexan-1-ol

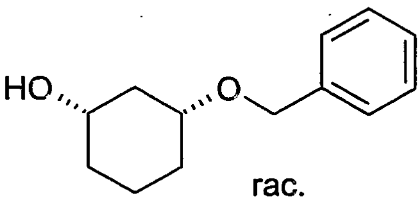

rac.

10 mg racemisches cis-3-Benzyloxy-cyclohexan-1-ol wurden in 1 mL Vinylacetat und 3 mL Chlorbenzol gelöst, mit ca. 5 mg Lipase SL versetzt und bei 22-25 °C gerührt. Nach ≥50 % Umsatz wurde die Reaktion durch Abfiltrieren des Enzyms beendet. Die optische Reinheit von (1S,3R)-3-Benzyloxy-cyclohexan-1-ol betrug ≥87 % ee (HPLC an Chiralpak AD-H 250 x 4.6; 1 mL/min, Heptan/EtOH/$CH_3CN$ 25:1:0.5 + 0.1 % TFA).

Beispiel 68

Racematspaltung von cis-3-Benzyloxy-cyclohexan-1-ol

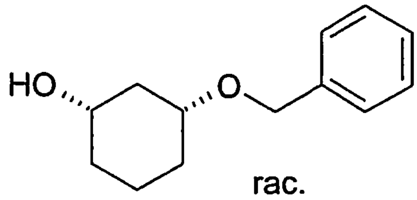

10 mg racemisches cis-3-Benzyloxy-cyclohexan-1-ol wurden in 1 mL Vinylacetat und 3 mL Diisopropylether gelöst, mit 10 mg Novozym 435 versetzt und bei 22-25 °C gerührt. Nach 4 Stunden wurde die Reaktion durch Abfiltrieren des Enzyms beendet. Die optische Reinheit von (1S,3R)-3-Benzyloxy-cyclohexan-1-ol betrug 90 % ee (HPLC an Chiralpak AD-H 250 x 4.6; 1 mL/min, Heptan/EtOH/ $CH_3CN$ 25:1:0.5 + 0.1 % TFA), der ee des (1R,3S)-Acetats war 90 % (HPLC an Chiralcel OD 250 x 4.6; 1 mL/min, Heptan/EtOH/$CH_3CN$ 100:1:0.5).

Beispiel 69

Racematspaltung von cis-3-Benzyloxy cyclohexan-1-ol

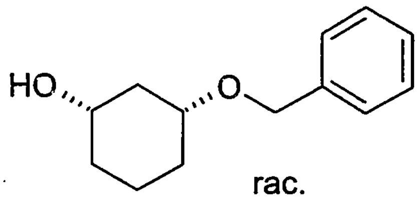

10 mg des racemischen cis-3-Benzyloxy-cyclohexan-1-ol wurden in 1 mL Vinylacetat und 3 mL MTBE gelöst, mit 10 mg Novozym 435 versetzt und bei 22-25 °C gerührt. Nach 4 Stunden wurde die Reaktion durch Abfiltrieren des Enzyms beendet. Die optische Reinheit von (1S,3R)-3-Benzyloxy-cyclohexan-1-ol betrug 93 % ee (HPLC an Chiralpak AD-H 250 x 4.6; 1 mUmin, Heptan/EtOH/$CH_3CN$ 25:1:0.5 + 0.1 % TFA), der ee des (1R,3S)-Acetats war 89 % (HPLC an Chiralcel OD 250 x 4.6; 1 mL/min, Heptan/EtOH/$CH_3CN$ 100:1:0.5).

Beispiel 70

Racematspaltung von cis-3-Benzyloxy-cyclohexan-1-ol

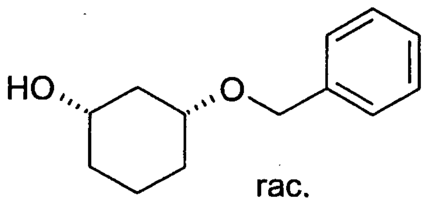

10 mg des racemischen cis-3-Benzyloxy-cyclohexan-1-ols wurden in 1mL Vinylacetat und 3 mL Cyclohexan gelöst, mit ca. 5 mg Lipase SL versetzt und bei 22-25 °C gerührt. Nach 24 Stunden wurde die Reaktion durch Abfiltrieren des Enzyms beendet. Die optische Reinheit von (1S,3R)-3-Benzyloxy-cyclohexan-1-ol betrug > 90 % ee (HPLC an Chiralpak AD-H 250 x 4.6; 1 mL/min, Heptan/EtOH/$CH_3CN$ 25:1:0.5 + 0.1 % TFA).

Beispiel 71

Racematspaltung von cis-3-Benzyloxy-cyclohexan-1-ol

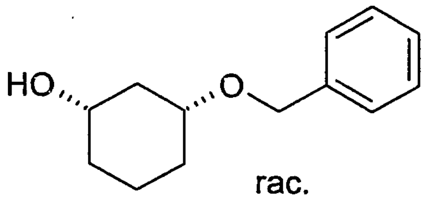

rac.

27 mg racemisches cis-3-Benzyloxy-cyclohexan-1-ol wurden in 3 mL Methylenchlorid gelöst, mit 65 mg iso-Valeriansäureanhydrid und mit 11 mg Novozym 435 versetzt und bei 22-25 °C gerührt. Nach 45-50 % Umsatz wurde die Reaktion durch Abfiltrieren des Enzyms beendet. Die optische Reinheit von (1S,3R)-3-Benzyloxycyclohexan-1-ol betrug 87 % ee (HPLC an Chiralpak AD-H 250 x 4.6; 1 mL/min, Heptan/EtOH/ $CH_3CN$ 25:1:0.5 + 0.1 % TFA), der Enantiomerenüberschuss des (1R,3S)-iso-Valeriansäurederivats war > 95 % (HPLC an Chiralcel OD 250 x 4.6;1 mL/min, Heptan/EtOH/$CH_3CN$ 100:1:0.5).

Beispiel 72

Racematspaltung von cis-3-Benzyloxy-cyclohexan-1-ol

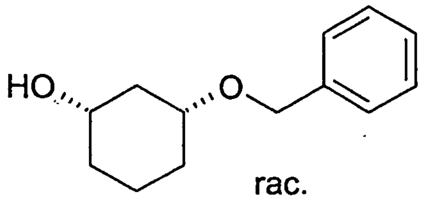

rac.

200 mg des racemischen cis-3-Benzyloxy-cyclohexan-1-ols wurden in 3 mL Chlorbenzol gelöst, mit 100 mg Bernsteinsäureanhydrid und 10 mg Chirazyme L-2, lyo. versetzt und bei 25-27 °C gerührt. Nach 29 Stunden wurde die Reaktion durch Abfiltrieren des Enzyms beendet. Aus einer eingedampften Probe wurde die optische Reinheit sowohl des nicht umgesetzten Substrates als auch des gebildeten Acylierungsprodukts bestimmt. Die optische Reinheit von (1S,3R)-3-Benzyloxycyclohexan-1-ol betrug > 98 % ee (HPLC an Chiralpak AD-H 250 x 4.6; 1 mL/min, Heptan/EtOH/$CH_3CN$ 25:1:0.5 + 0.1 % TFA), die optische Reinheit des Bernsteinsäurederivates betrug 94 % ee (HPLC an Chiralcel OD 250 x 4.6; 1 mL/min, Heptan/EtOH/$CH_3CN$ 25:1:0.5 + 0.1 % TFA).

Beispiel 73

Racematspaltung von cis-3-Benzyloxy-cyclohexan-1-ol

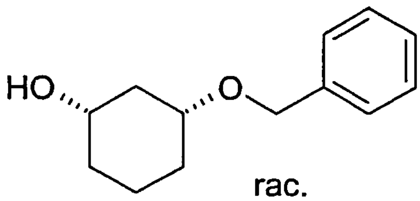

rac.

200 mg des racemischen cis-3-Benzyloxy-cyclohexan-1-ols wurden in 3 mL DME gelöst, mit 100 mg Bernsteinsäureanhydrid und 10 mg Chirazyme L-2, lyo. versetzt und bei 25-27 °C gerührt. Nach 29 Stunden wurde die Reaktion durch Abfiltrieren des Enzyms beendet. Aus einer eingedampften Probe wurde die optische Reinheit sowohl des nicht umgesetzten Substrates als auch des gebildeten Acylierungsprodukts bestimmt. Die optische Reinheit von (1S,3R)-3-Benzyloxy-cyclohexan-1-ol betrug > 95 % ee (HPLC an Chiralpak AD-H 250 x 4.6; 1 mL/min, Heptan/EtOH/$CH_3CN$ 25:1:0.5 + 0.1 % TFA), die optische Reinheit des Bernsteinsäurederivates betrug > 97 % ee (HPLC an Chiralcel OD 250 x 4.6; 1 mL/min, Heptan/EtOH/$CH_3CN$ 25:1:0.5 + 0.1 % TFA).

Beispiel 74

Racematspaltung von cis-3-Benzyloxy-cyclohexan-1-ol

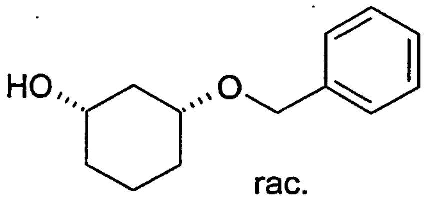

rac.

200 mg des racemischen cis-3-Benzyloxy-cyclohexan-l-ols wurden in 3 mL THF gelöst, mit 100 mg Bernsteinsäureanhydrid und 10 mg Chirazyme L-2, lyo. versetzt und bei 25-27 °C gerührt. Nach 29 h wurde die Reaktion durch Abfiltrieren des Enzyms beendet. Aus einer eingedampften Probe wurde die optische Reinheit sowohl des nicht umgesetzten Substrates als auch des gebildeten Acylierungsprodukts bestimmt. Die optische Reinheit von (1S,3R)-3-Benzyloxy-cyclohexan-1-ol betrug 84 % ee (HPLC an Chiralpak AD-H 250 x 4.6; 1 mL/min, Heptan/EtOH/$CH_3CN$ 25:1:0.5 + 0.1 % TFA), die optische Reinheit des Bernsteinsäurederivates betrug > 95 % ee (HPLC an Chiralcel OD 250 x 4.6; 1 mL/min, Heptan/EtOH/$CH_3CN$ 25:1:0.5 + 0.1 % TFA).

Beispiel 75

Racematspaltung von cis-3-Benzyloxy-cyclohexan-1-ol

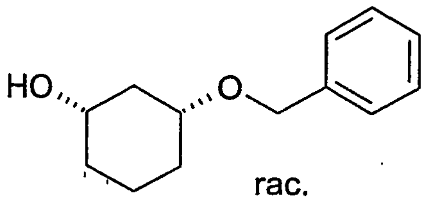

rac.

200 mg des racemischen cis-3-Benzyloxy-cyclohexan-1-ols wurden in 3 mL Methylenchlorid gelöst, mit 100 mg Bernsteinsäureanhydrid und 10 mg Chirazyme L-2, lyo., versetzt und bei 25-27 °C gerührt. Nach 29 h wurde die Reaktion durch Abfiltrieren des Enzyms beendet. Aus einer eingedampften Probe wurde die optische Reinheit sowohl des nicht umgesetzten Substrates als auch des gebildeten Acylierungsprodukts bestimmt. Die optische Reinheit von (1S,3R)-3-Benzyloxycyclohexan-1-ol betrug > 98 % ee (HPLC an Chiralpak AD-H 250 x 4.6; 1 mL/min, Heptan/EtOH/$CH_3CN$ 25: 1:0.5 + 0.1 % TFA), die optische Reinheit des Bernsteinsäurederivates betrug 88 % ee (HPLC an Chiralcel OD 250 x 4.6; 1 mL/min, Heptan/Et0H/$CH_3CN$ 25:1:0.5 + 0.1 % TFA).

Beispiel 76

Racematspaltung von cis-3-Benzyloxy-cyclohexan-1-ol

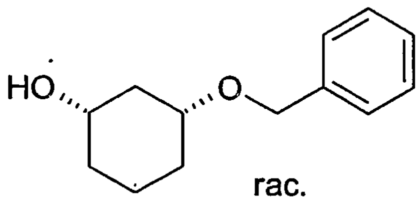

rac.

200 mg des racemischen cis-3-Benzyloxy-cyclohexan-1-ols wurden in 3 mL Aceton gelöst, mit 100 mg Bernsteinsäureanhydrid und 10 mg Chirazyme L-2, lyo. versetzt und bei 25-27 °C gerührt. Nach 29 h wurde die Reaktion durch Abfiltrieren des Enzyms beendet. Aus einer eingedampften Probe wurde die optische Reinheit sowohl des nicht umgesetzten Substrates als auch des gebildeten Acylierungsprodukts bestimmt. Die optische Reinheit von (1S,3R)-3-Benzyloxy-cyclohexan-1-ol betrug > 99 % ee (HPLC an Chiralpak AD-H 250 x 4.6; 1 mL/min, Heptan/EtOH/$CH_3CN$ 25:1: 0.5 + 0.1 % TFA), die optische Reinheit des Bernsteinsäurederivates betrug 78 % ee (HPLC an Chiralcel OD 250 x 4.6; 1 mL/min, Heptan/EtOH/$CH_3CN$ 25:1:0.5 + 0.1 % TFA).

Beispiel 77

Racematspaltung von cis-3-Benzyloxy-cyclohexan-1-ol, Trennung von Alkohol und Bernsteinsäurederivat

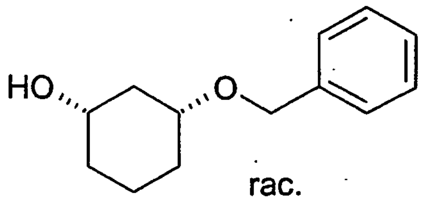

rac.

8.15 g (39.5 mmol)des racemischen cis-3-Benzyloxy-cyclohexan-1-ols wurden in 120mL THF gelöst, mit 3.9 g (39.0 mmol) Bernsteinsäureanhydrid und 390 mg Chirazyme L-2, lyo. versetzt und bei 22-25 °C gerührt. Nach ca. 40 % Umsatz wurde die Reaktion durch Abfiltrieren des Enzyms beendet. Das Filtrat wurde im Vakuum eingeengt. Der Rückstand wurde mit tBuOMe aufgenommen und dreimal mit jeweils 100 mL ges. wässriger $NaHCO_3$-Lösung intensiv extrahiert. Die organische Phase wurde getrocknet ($MgSO_4$) und im Vakuum eingeengt; Ausbeute: 4.4 g; die optische Reinheit von (1S,3R)-3-Benzyloxy-cyclohexan-1-ol betrug 70 % ee (HPLC an Chiralpak AD-H 250 x 4.6; 1 mL/min, Heptan/EtOH/$CH_3CN$ 25:1:0.5 + 0.1 % TFA). Die optische Reinheit des in den vereinigten Wasserphasen gelösten Bernsteinsäurederivates betrug > 99 % ee (HPLC an Chiralcel OD 250 x 4.6; 1 mL/min, Heptan/EtOH/$CH_3CN$ 25:1:0.5 + 0.1 % TFA).

Durch Behandeln mit konz. Natronlauge wurde die wässrige Lösung des Bernsteinsäurederivats chemisch hydrolysiert. Das gebildete (1R,3S)-3-Benzyloxycyclohexan-1-ol wurde mit tBuOMe extrahiert;Ausbeute: 2.9 g.

Beispiel 78

Racematspaltung von cis-3-Benzyloxy-cyclohexan-1-ol, Trennung von Alkohol und Bernsteinsäurederivat

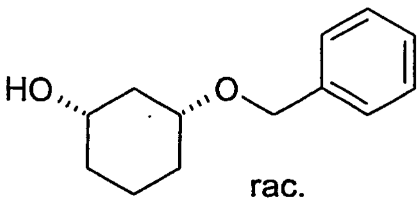

rac.

5.06 g (24.5 mmol) des racemischen cis-3-Benzyloxy-cyclohexan-1-ols wurden in 75 mL THF gelöst, mit 2.52 g (25.2 mmol) Bernsteinsäureanhydrid und 3.1 g Novozym 435 versetzt und bei 22-25 °C gerührt. Nach 28.5 h wurde die Reaktion durch Abfiltrieren des Enzyms beendet. Das Filtrat wurde im Vakuum bis auf ca. 15 mL eingeengt. Der Rückstand wurde mit 30 mL Wasser versetzt und das restliche THF im Vakuum abdestilliert. 15 mL gesättigte wässriger $NaHCO_3$-Lösung, 15 mL Wasser und 30 mL Methylenchlorid wurden zugegeben und die Mischung 15-30 min intensiv gerührt. Nach Phasentrennung wurde die organische Phase erst mit 90 mL gesättigter wässriger $NaHCO_3$-Lösung und 150 mL Wasser, anschließend noch mit 15 mL gesättigter wässriger $NaHCO_3$-Lösung und 30 mL Wasser extrahiert, zweimal mit 30 mL Wasser gewaschen. Die organische Phase wurde getrocknet ($MgSO_4$) und im Vakuum eingeengt; Ausbeute: 2.52 g (50 %), $[\alpha]_D^{20}$ +12.1° (c=1.0, MeOH); die optische Reinheit von (1S,3R)-3-Benzyloxy-cyclohexan-1-ol betrug > 99% ee (HPLC an Chiralpak AD-H 250 x 4.6; 1 mUmin, Heptan/EtOH/$CH_3CN$ 25:1:0.5 + 0.1 % TFA). Die vereinigten Wasserphasen wurden mit 20 mL Essigsäure (Eisessig) versetzt und zweimal mit
20 mL Methylenchlorid extrahiert. Die organische Phase wurde getrocknet ($MgSO_4$) und eingeengt; Ausbeute: 3.55 g (47 %) des Bernsteinsäurederivates; die optische Reinheit betrug > 99 % ee (HPLC an Chiralcel OD 250 x 4.6; 1 mL/min,

Heptan/EtOH/$CH_3CN$ 25:1:0.5 + 0.1 % TFA); $^1H$ NMR ($CDCl_3$) δ: 1.2-1.43 (m, 4H), 1.82 (m, 1 H), 1.93 (m, 1 H), 2.04 (m, 1 H), 2.40 (m, 1 H), 2.58-2.70 (m, 4H), 3.39 (m, 1 H), 4.54 (m, 2H), 4.71 (m, 1 H); 7.23-7.36 (m, 5H).

Beispiel 79

Herstellung von racemischem cis-3-(tert-Butyl-dimethyl-silanyloxy)-cyclohexanol

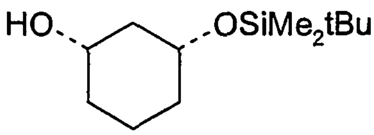

rac

Zu einer auf 10 °C gekühlten Lösung von 1,3-Cyclohexanediol (20.05 g, 0.173 mol), $Et_3N$ (28.79 ml, 1.2 eq) und DMAP (0.844 g, 0.04 eq) in $CH_2Cl_2$ (600 ml) wurde TBDMSCI (28.62 g, 1.1 eq) langsam zugegeben. Nach 18stündigem Rühren bei 20-23 °C wurde die Reaktionsmischung mit $H_2O$ (2x100 ml) gewaschen. Die organische Phase wurde mit ges. $NH_4Cl$ (2x100 ml) gewaschen, über $MgSO_4$ getrocknet und im Vakuum eingeengt. Chromatographie an Kieselgel (n-Heptan/EE 20:1) ergab 18.77 g (47%) des gewünschten Monosilylethers; $^1H$-NMR ($CDCl_3$) δ: 0.0-0.1 (m, 6H), 0.8-0.9 (m, 9H), 1.2-2.0 (m, 8H), 3.2 (s, br., 1 H), 3.8 (m, 1 H), 3.95 (m, 1 H).

Beispiel 80

Herstellung von cis-(1S, 3R)-3-(tert-Butyl-dimethyl-silanyloxy)-cyclohexanol

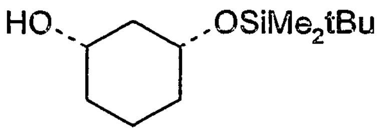

770 mg racemisches cis-3-(tert-Butyl-dimethyl-silanyloxy)-cyclohexanol wurden in 10 mL Aceton gelöst, mit 0.36 mL Vinylacetat und 400 mg Novozym 435 versetzt und bei 21-24 °C gerührt. Nach 47 h (ca. 50 % Umsatz) wurde die Reaktion durch Abfiltrieren des Enzyms beendet und die Lösung im Vakuum eingedampft. Chromatographie einer Teilmenge an Kieselgel (n-Heptan/EE 3:1) ergab (1S, 3R)-3-(tert-Butyl-dimethyl-silanyloxy)-cyclohexanol mit $[\alpha]_D^{20}$ +12.8° (c=1.0, MeOH).

**Patentansprüche**

1. Verfahren zur Herstellung einer chiralen, nicht racemischen Verbindung der Formel I

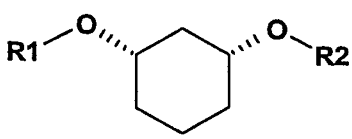

**(I)**

mit:
R1

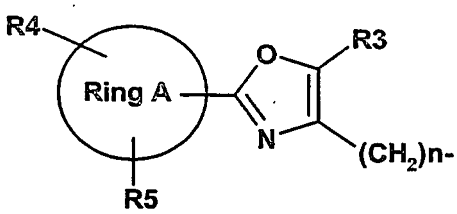

worin bedeuten:

Ring A Phenyl, 5 - 12 gliedriger heteroaromatischer Ring, der ein bis vier Heteroatome aus der Gruppe N, O oder S enthalten kann, 8 bis 14 gliedriger aromatischer Ring, $(C_3-C_8)$-Cycloalkyl;
R3 H, F, Cl, Br, OH, $NO_2$ $CF_3$, $OCF_3$, $(C_1-C_6)$-Alkyl, $(C_3-C_8)$-Cycloalkyl, Phenyl;
R4, R5 H, F, Cl, Br, OH, $NO_2$, $CF_3$, $OCF_3$, $OCF_2H$, $OCF_2-CF_3$, $OCF_2-CHF_2$, $SCF_3$, O-Phenyl, $(C_1-C_6)$-Alkyl, O-$(C_1-C_6)$-Alkyl, O-$(C_1-C_6)$-Alkyl-O-$(C_1-C_3)$-Alkyl;
n 1 bis 3;

und

$R^2$ $(C_1-C_8)$-Alkyl, wobei in den Alkylgruppen ein oder mehrere $CH_2$-Gruppen durch O, CO, S, SO oder $SO_2$ ersetzt sein können, und Alkyl ein bis dreifach substituiert sein kann durch F, Cl, Br, $CF_3$, CN, $NO_2$ NHAc, NHBoc, NH-CO-$C(CH_3)_3$, Hydroxyl, $OCF_3$, O-$(C_1-C_6)$-Alkyl, COOH, CO-Benzoxy, CO-O$(C_1-C_6)$-Alkyl, Tetrazol, Thiazolidin-2,4-dion, Indol und $(C_6-C_{10})$-Aryl, wobei Thiazolidin-2,4-dion und Aryl wiederum durch F, Cl, Br, $CF_3$, CN, $NO_2$, NHAc, NHTs, NHBoc, NHCbz, NH-CO-$C(CH_3)_3$, Hydroxyl, $OCF_3$, O-$(C_1-C_6)$-Alkyl, COOH, CO-Benzoxy, CO-O$(C_1-C_6)$-Alkyl, $(C_1-C_6)$-Alkyl, O-$(C_1-C_6)$-Alkyl oder Tetrazol substituiert sein können, oder;
$R^2$ eine OH-Schutzgruppe (SG), wie z.B. Benzyloxymethyl, Benzyl, para-Methoxybenzyl oder tert.-Butyl-dimethylsilyl;

**dadurch gekennzeichnet, dass** man
A)

a) Alkylierung (Alk-R2 / Alk-SG)
cis-1,3-Cyclohexandiol der Formel (II)

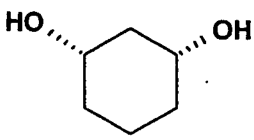

(II)

mit einer Verbindung der Formel (III)

$X^1$-$R^2$ (III)

worin R2 wie oben definiert ist und

$X^1$ für Cl, Br, I, OMs, OTs, OTf steht;

in Gegenwart von Basen in einem geeigneten Lösungsmittel umsetzt zu einer racemischen Verbindung der Formel (IV),

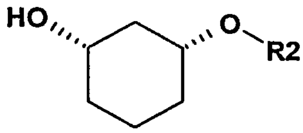

(IV)

worin R2 wie oben definiert ist;
b1) Enzymatische Esterbildung (EB) + Trennung (T)
die erhaltene Verbindung der Formel (IV) einer stereoselektiven enzymatischen Esterbildung (EB) unterwirft, wobei die Alkohole in einem organischen Lösungsmittel mit einem Acyldonor und dem Enzym versetzt und die resultierende Mischung bei -20 bis 80 °C gerührt wird und nach Ablauf der Reaktion das eine Stereoisomere als Ester der Formel (V)

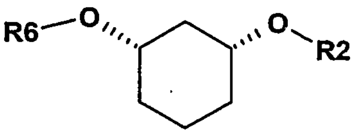

(V)

worin

$R^6$ C(=O)-($C_1$-$C_{16}$)-Alkyl, C(=O)-($C_2$-$C_{16}$)-Alkenyl, C(=O)-($C_3$-$C_{16}$)-Alkinyl, C(=O)-($C_3$-$C_{16}$)-Cycloalkyl, wobei ein oder mehrere Kohlenstoffatome durch Sauerstoffatome ersetzt sein und substituiert sein können mit 1-3 Substituenten aus der Gruppe F, Cl, Br, $CF_3$, CN, $NO_2$, Hydroxy, Methoxy, Ethoxy, Phenyl und CO-O($C_1$-$C_4$)-Alkyl, CO-O($C_2$-$C_4$)-Alkenyl, die wiederum substituiert sein können mit 1-3 Substituenten aus der Gruppe F, Cl, Br, $CF_3$ bedeutet, und
R2 wie oben definiert sind,

und das andere Stereoisomere unverändert als Alkohol der Formel (IV) vorliegt, und daher durch Ausnutzung ihrer unterschiedlichen chemischen bzw. physikochemischen Eigenschaften voneinander getrennt wird (Trennung T)
oder
b2) Enzymatische Esterspaltung [= Chemische Veresterung (CV) + Enzymatische Spaltung (ES)] + Trennung (T)
die erhalte Verbindung der Formel (IV) einer stereoselektiven enzymatischen Esterspaltung unterwirft, wobei der racemische Alkohol zunächst durch chemische Veresterung (CV), z. B. mittels Säurechlorid R6-Cl oder Säureanhydrid R6-O-R6 in Gegenwart von Basen in den racemischen Ester der Formel (V)

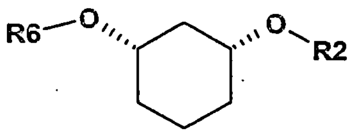

(V)

worin R6 und R2 wie oben definiert sind,
überführt wird, der dann zur Durchführung der stereoselektiven enzymatischen Esterspaltung (ES) in homogenem oder heterogenem, wässrigem, wässrigorganischem oder organischem Medium aufgenommen und in Gegenwart eines Enzyms im Falle der Hydrolyse mit Wasser und im Falle der Alkoholyse mit einem Alkohol bei einer Temperatur von 10 - 80 °C zur Reaktion gebracht wird, nach deren Ablauf das eine Stereoisomere als Alkohol der Formel (IV) und das andere unverändert als Ester der Formel (V) vorliegt und somit wie unter b1) beschreiben voneinander getrennt werden kann, wobei
das als Alkohol anfallende Enantiomere der Formel (IV) wie unter d) beschrieben weiter verarbeitet wird, oder
c) Chemische Hydrolyse (CH)
das als Ester anfallende Enantiomere der Formel (V) zum chemisch enantiomeren Alkohol nach bekannten

Verfahren verseift und
d) Alkylierung (Alk-R1)
weiter mit einer Verbindung der Formel (VI)

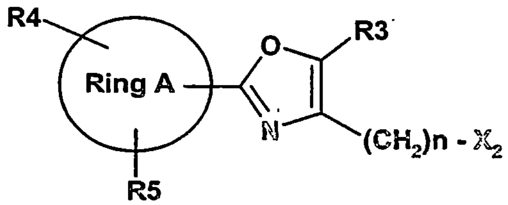

(VI)

worin
Ring A, R3, R4, R5 und n wie oben definiert sind und

$x^2$ Cl, Br, I, OTs, OMs, OTf bedeutet;

in Gegenwart von Basen in einem geeigneten Lösungsmittel zu der Verbindung der Formel (I) umgesetzt wird, und
e) Abspaltung der Schutzgruppe SG (AbSG)
falls R2 für eine OH-Schutzgruppe (SG) steht wie oben unter R2 definiert, die Verbindung der Formel (Ia)

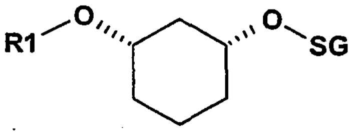

(Ia)

worin R1 und SG wie oben definiert sind,
durch Abspaltung der Schutzgruppe nach bekannten Verfahren in eine Verbindung der Formel (VII)

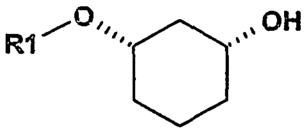

(VII)

worin R1 wie oben definiert ist, überführt,
f) Alkylierung (Alk-R2)
die anschließend mit einer Verbindung der Formel (III),

$X^1$-$R^2$ (III)

worin X1 und R2 wie oben definiert sind,
in Gegenwart von Basen in einem geeigneten Lösungsmittel zu einer Verbindung der Formel (I), dem Produkt bzw. der enantiomeren Form, umgesetzt wird,
wobei es auch möglich ist, die Reihenfolge der einzelnen Reaktionsschritte wie vorstehend unter A) beschrieben:

A) Alk-R2 → EB+T / CV + ES + T [→ CH] → Alk-R1 [→ AbSG → Alk-R2] → Produkt/enantiomere Form
zu ändern in:
B) Alk-R1 → EB+T / CV+ES+T [→ CH] → Alk-R2 [→ AbSG → Alk-R2] → Produkt/enantiomere Form
oder
C) Alk-SG → EB+T / CV+ES+T → CH → Alk-R2 → AbSG → Alk-R1 → Produkt/enantiomere Form

oder
D) Alk-SG → EB+T / CV+ES+T → Alk-R1 → AbSG → Alk-R2 → Produkt/enantiomere Form.

2. Verfahren gemäß Anspruch 1, **dadurch gekennzeichnet, dass** die Verfahren C) und D) angewendet werden.

3. Verfahren gemäß den Ansprüchen 1 oder 2, **dadurch gekennzeichnet, dass** Verbindungen der Formel (III)

$X^1 - R^2$ (III)

verwendet werden, worin

$X^1$ Cl, Br, I, OMs oder OTs bedeutet.

4. Verfahren gemäß den Ansprüchen 1 bis 3, **dadurch gekennzeichnet, dass** Verbindungen der Formel (III)

$X^1 - R^2$ (III)

verwendet werden, worin

$X^1$ Cl, Br oder 1 bedeutet.

5. Verfahren gemäß den Ansprüchen 1 bis 4, **dadurch gekennzeichnet, dass** eine Verbindung der Formel (I)

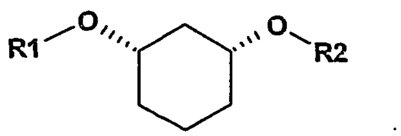

**(I)**

hergestellt wird, worin stehen:

R1 für

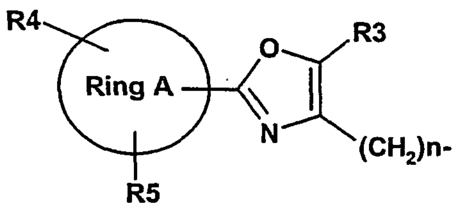

mit

Ring A Phenyl, 5 -12 gliedriger heteroaromatischer Ring, der ein oder mehrere Heteroatome aus der Gruppe N, O oder S enthalten kann, annellierter/bicyclischer 8 bis 14 gliedriger aromatischer Ring, ($C_3$-$C_8$)-Cycloalkyl;
R3 H, $CF_3$, ($C_1$-$C_6$)-Alkyl, ($C_3$-$C_8$)-Cycloalkyl, Phenyl;
R4, R5 H, F, Br, $CF_3$, $OCF_3$, ($C_1$-$C_6$)-Alkyl, O-($C_1$-$C_6$)-Alkyl;
n 1 bis 2 und
R2 ($C_1$-$C_8$)-Alkyl, wobei in den Alkylgruppen ein oder mehrere $CH_2$-Gruppen durch O, CO, S, SO oder $SO_2$ ersetzt sein können, und Alkyl ein bis dreifach substituiert sein kann durch F, Cl, Br, $CF_3$, CN, $NO_2$, NHAc, NHBoc, NH-CO-C($CH_3$)$_3$, Hydroxyl, $OCF_3$, O-($C_1$-$C_6$)-Alkyl, COOH, CO-Benzoxy, CO-O($C_1$-$C_6$)-Alkyl, Tetrazol, Thiazolidin-2,4-dion, Indol und ($C_6$-$C_{10}$)-Aryl, wobei Thiazolidin-2,4-dion und Aryl wiederum durch F, Cl, Br, $CF_3$, CN, $NO_2$, NHAc, NHTs, NHBoc, NHCbz, NH-CO-C($CH_3$)$_3$, Hydroxyl, $OCF_3$, O-($C_1$-$C_6$)-Alkyl, COOH, CO-Benzoxy, CO-O($C_1$-$C_6$)-Alkyl, ($C_1$-$C_6$)-Alkyl, O-($C_1$-$C_6$)-Alkyl oder Tetrazol substituiert sein können.

6. Verfahren gemäß den Ansprüchen 1 bis 5, **dadurch gekennzeichnet, dass** eine Verbindung der Formel (I)

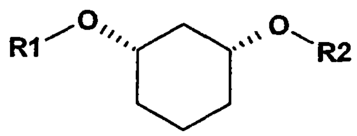

(I)

hergestellt wird, worin stehen:

R1 für

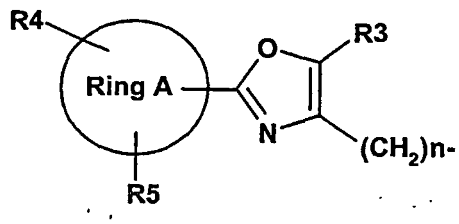

mit

Ring A Phenyl;
R3 $(C_1-C_4)$-Alkyl;
R4, R5 H, $(C_1-C_4)$-Alkyl, O-$(C_1-C_4)$-Alkyl;
n 1 und
R2 $(C_1-C_8)$-Alkyl, wobei in den Alkylgruppen ein oder mehrere $CH_2$-Gruppen durch O, CO, S, SO oder $SO_2$ ersetzt sein können, und Alkyl ein bis dreifach substituiert sein kann durch F, Cl, Br, $CF_3$, CN, $NO_2$, NHAc, NHBoc, NH-CO-$C(CH_3)_3$, Hydroxyl, $OCF_3$, O-$(C_1-C_6)$-Alkyl, COOH, CO-Benzoxy, CO-O$(C_1-C_6)$-Alkyl, Tetrazol, Thiazolidin-2,4-dion, Indol und $(C_6-C_{10})$-Aryl, wobei Thiazolidin-2,4-dion und Aryl wiederum durch F, Cl, Br, $CF_3$, CN, $NO_2$, NHAc, NHTs, NHBoc, NHCbz, NH-CO-$C(CH_3)_3$, Hydroxyl, $OCF_3$, O-$(C_1-C_6)$-Alkyl, COOH, CO-Benzoxy, CO-O$(C_1-C_6)$-Alkyl, $(C_1-C_6)$-Alkyl, O-$(C_1-C_6)$-Alkyl oder Tetrazol substituiert sein können.

## Claims

**1.** A process for preparing a chiral, nonracemic compound of the formula I

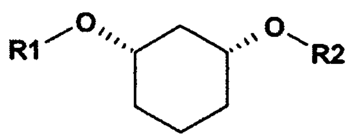

(I)

where:

R1 is

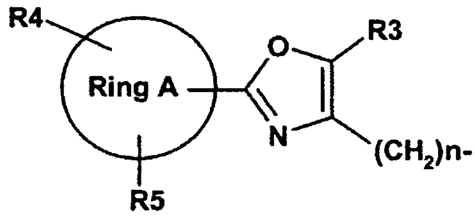

where:

ring A is phenyl, 5-12 membered heteroaromatic ring which may contain from one to four heteroatoms from the group of N, O and S, 8 to 14 membered aromatic ring, $(C_3-C_8)$-cycloalkyl;
R3 is H, F, Cl, Br, OH, $NO_2$, $CF_3$, $OCF_3$, $(C_1-C_6)$-alkyl, $(C_3-C_8)$-cycloalkyl, phenyl;
R4, R5 are H, F, Cl, Br, OH, $NO_2$, $CF_3$, $OCF_3$, $OCF_2H$, $OCF_2-CF_3$, $OCF_2-CHF_2$, $SCF_3$, O-phenyl, $(C_1-C_6)$-alkyl, O-$(C_1-C_6)$-alkyl, O-$(C_1-C_6)$-alkyl-O-$(C_1-C_3)$-alkyl;
n is from 1 to 3;

and

$R^2$ is $(C_1-C_8)$ -alkyl where one or more $CH_2$ groups in the alkyl groups may be replaced by O, CO, S, SO or $SO_2$, and alkyl may be one to trisubstituted by F, Cl, Br, $CF_3$, CN, $NO_2$, NHAc, NHBoc, NH-CO-$C(CH_3)_3$, hydroxyl, $OCF_3$, O- $(C_1-C_6)$ -alkyl, COOH, CO-benzoxy, CO-O$(C_1-C_6)$-alkyl, tetrazole, thiazolidine-2,4-dione, indole and $(C_6-C_{10})$-aryl, where thiazolidine-2,4-dione and aryl may in turn be substituted by F, Cl, Br, $CF_3$, CN, $NO_2$, NHAc, NHTs, NHBoc, NHCbz, NH-CO-$C(CH_3)_3$, hydroxyl, $OCF_3$, O-$(C_1-C_6)$-alkyl, COOH, CO-benzoxy, CO-O $(C_1-C_6)$ -alkyl, $(C_1-C_6)$ -alkyl, O-$(C_1-C_6)$-alkyl or tetrazole, or;
$R^2$ is an OH protecting group (PG), for example benzyloxymethyl, benzyl, para-methoxybenzyl or tert-butyldimethylsilyl;

which comprises
A)

a) alkylation (alk-R2/alk-PG)
reacting cis-1,3-cyclohexanediol of the formula (II)

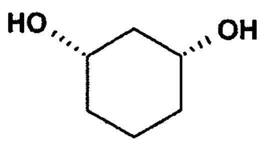

(II)

with a compound of the formula (III)

$X^1-R^2$ (III)

where R2 is as defined above and

$X^1$ is Cl, Br, I, OMs, OTs, OTf;

in the presence of bases in a suitable solvent to give a racemic compound of the formula (IV)

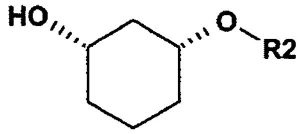

(IV)

where R2 is as defined above;
b1) enzymatic ester formation (EF) + separation (S)
subjecting the resulting compound of the formula (IV) to stereoselective enzymatic ester formation (EF), in which the alcohols are admixed with an acyl donor and the enzyme in an organic solvent and the resulting mixture is stirred at -20 to 80°C and, after the reaction has ended, one stereoisomer is present as an ester of the formula (V)

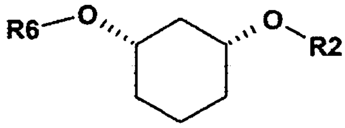

(V)

where

$R^6$ is C(=O)-($C_1$-$C_{16}$)-alkyl, C(=O)-($C_2$-$C_{16}$)-alkenyl, C (=O) - ($C_3$-$C_{16}$)-alkynyl, C (=O)-($C_3$-$C_{16}$) -cycloalkyl, where one or more carbon atoms may be replaced by oxygen atoms and be substituted by 1-3 substituents from the group of F, Cl, Br, $CF_3$, CN, $NO_2$, hydroxyl, methoxy, ethoxy, phenyl and CO-O($C_1$-$C_4$)-alkyl, CO-O($C_2$-$C_4$)-alkenyl, which may in turn be substituted by 1-3 substituents from the group of F, Cl, Br, $CF_3$, and R2 is as defined above,

and the other stereoisomer is present unchanged as the alcohol of the formula (IV), and are therefore separated from each other by utilizing their different chemical or physicochemical properties (separation S)
or
b2) enzymatic ester hydrolysis [=chemical esterification (CE) + enzymatic hydrolysis (EH)] + separation (S)
subjecting the resulting compound of the formula (IV) to a stereoselective enzymatic ester hydrolysis, in which the racemic alcohol is initially converted by chemical esterification (CE), for example by means of acid chloride R6-Cl or acid anhydride R6-O- R6, in the presence of bases, to the racemic ester of the formula (V)

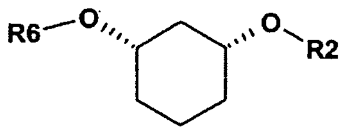

(V)

where R6 and R2 are each as defined above,
which, to carry out the stereoselective enzymatic ester hydrolysis (EH), is then taken up in homogeneous or heterogeneous, aqueous, aqueous-organic or organic medium, and reacted, in the presence of an enzyme in the case of hydrolysis with water and in the case of alcoholysis with an alcohol, at a temperature of 10-80°C, and after the reaction has ended, one stereoisomer is present as the alcohol of the formula (IV) and the other is present unchanged as the ester of the formula (V) and can thus be separated from each other as described under b1), and
the enantiomer of the formula (IV) occurring as an alcohol is further processed as described under d), or
c) chemical hydrolysis (CH)
hydrolyzing the enantiomer of the formula (V) occurring as an ester to the chemically enantiomeric alcohol by known methods and
d) alkylation (alk- R1)
reacting further with a compound of the formula (VI)

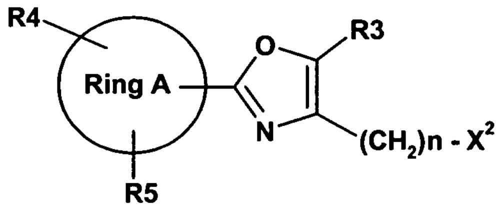

(VI)

where
ring A, R3, R4, R5 and n are each as defined above and

$X^2$ is Cl, Br, I, OTs, OMs, OTf;

in the presence of bases in a suitable solvent to give the compound of the formula (I), and
e) detachment of the protecting group PG (detPG)
if R2 is an OH protecting group (PG) as defined above under R2, converting the compound of the formula (Ia)

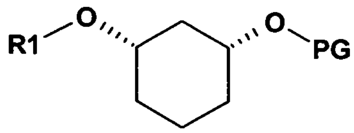

(Ia)

where R1 and PG are each as defined above,
by detaching the protecting group by known methods to a compound of the formula (VII)

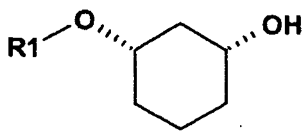

(VII)

where R1 is as defined above,
f) alkylation (alk- R2)
then reacting it with a compound of the formula (III)

$X^1$-$R^2$ (III)

where $X^1$ and R2 are each as defined above,
in the presence of bases in a suitable solvent to give a compound of the formula (I), the product or the enantiomeric form,
it being also possible to change the sequence of individual reaction steps as described above under A):
A) alk-R2 → EF + S/CE + EH + S [→ CH] → alk- R1 [→ DetPG → alk- R2] → product/enantiomeric form
to:
B) alk- R1 → EF + S/CE + EH + S [→ CH] → alk- R2 [→ DetPG → alk- R2] → product/enantiomeric form
or
C) alk-PG → EF + S/CE + EH + S → CH → alk- R2 → DetPG → alk- R1 → product/enantiomeric form
or
D) alk-PG → EF + S/CE + EH + S → alk- R1 → DetPG → alk- R2 → product/enantiomeric form.

**2.** The process as claimed in claim 1, wherein the processes C) and D) are employed.

**3.** The process as claimed in claim 1 or 2, wherein compounds of the formula (III)

$X^1 - R^2$ (III)

are used where

$X^1$ is Cl, Br, I, OMs or OTs.

**4.** The process as claimed in any of claims 1 to 3, wherein compounds of the formula (III)

$X^1 - R^2$ (III)

are used where

$x^1$ is Cl, Br or I.

**5.** The process as claimed in claims 1 to 4, wherein a compound of the formula (I)

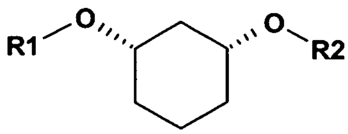

**(I)**

is prepared where:

R1 is

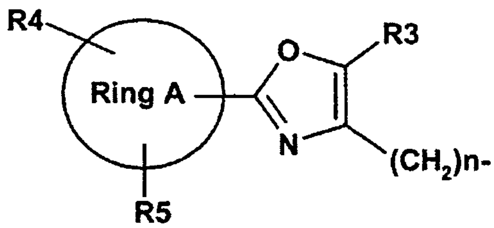

where

ring A is phenyl, 5-12 membered heteroaromatic ring which may contain from one or more heteroatoms from the group of N, O and S, fused/bicyclic 8 to 14 membered aromatic ring, $(C_3-C_8)$-cycloalkyl;
R3 is H, $CF_3$, $(C_1-C_6)$ -alkyl, $(C_3-C_8)$ -cycloalkyl, phenyl;
R4, R5 are H, F, Br, $CF_3$, $OCF_3$, $(C_1-C_6)$-alkyl, O-$(C_1-C_6)$-alkyl;
n is from 1 to 2 and
R2 is $(C_1-C_8)$-alkyl where one or more $CH_2$ groups in the alkyl groups may be replaced by O, CO, S, SO or $SO_2$, and alkyl may be one to trisubstituted by F, Cl, Br, $CF_3$, CN, $NO_2$, NHAc, NHBoc, NH-CO-$C(CH_3)_3$, hydroxyl, $OCF_3$, O-$(C_1-C_6)$-alkyl, COOH, CO-benzoxy, CO-O$(C_1-C_6)$-alkyl, tetrazole, thiazolidine-2,4-dione, indole and $(C_6-C_{10})$-aryl, where thiazolidine-2,4-dione and aryl may in turn be substituted by F, Cl, Br, $CF_3$, CN, $NO_2$, NHAc, NHTs, NHBoc, NHCbz, NH-CO-$C(CH_3)_3$, hydroxyl, $OCF_3$, O-$(C_1-C_6)$ -alkyl, COOH, CO-benzoxy, CO-O $(C_1-C_6)$ -alkyl, $(C_1-C_6)$ -alkyl, O-$(C_1-C_6)$ -alkyl or tetrazole.

**6.** The process as claimed in any of claims 1 to 5, wherein a compound of the formula (I)

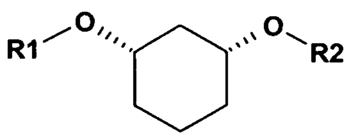

**(I)**

is prepared where:

R1 is

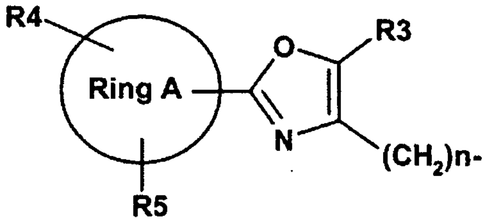

where

ring A is phenyl;
R3 is $(C_1-C_4)$ -alkyl;
R4, R5 are H, $(C_1-C_4)$ -alkyl, O- $(C_1-C_4)$ -alkyl;
n is 1 and
R2 is $(C_1-C_8)$-alkyl where one or more $CH_2$ groups in the alkyl groups may be replaced by O, CO, S, SO or $SO_2$ and alkyl may be one to trisubstituted by F, Cl, Br, $CF_3$, CN, $NO_2$, NHAc, NHBoc, NH-CO-$C(CH_3)_3$, hydroxyl, $OCF_3$, O-$(C_1-C_6)$-alkyl, COOH, CO-benzoxy, CO-O$(C_1-C_6)$-alkyl, tetrazole, thiazolidine-2,4-dione, indole and $(C_6-C_{10})$-aryl, where thiazolidine-2-4-dione and aryl may in turn be substituted by F, Cl, Br, $CF_3$, CN, $NO_2$, NHAc, NHTs, NHBoc, NHCbz, NH-CO-$C(CH_3)_3$, hydroxyl, $OCF_3$, O-$(C_1-C_6)$-alkyl, COOH, CO-benzoxy, CO-O$(C_1-C_6)$-alkyl, $(C_1-C_6)$-alkyl, O-$(C_1-C_6)$-alkyl or tetrazole.

## Revendications

**1.** Procédé de préparation d'un composé chiral, non racémique de formule I

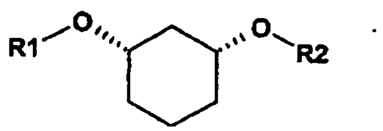

(I) .

avec
R1

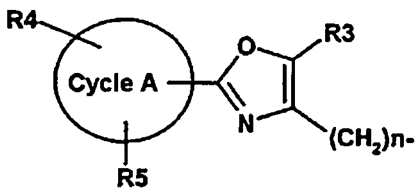

dans laquelle
le cycle A signifie phényle, un cycle hétéroaromatique de 5 à 12 chaînons, qui peut contenir un à quatre hétéroatomes du groupe N, O ou S, un cycle aromatique de 8 à 14 chaînons, $(C_3-C_8)$-cycloalkyle ;
R3 signifie H, F, Cl, Br, OH, $NO_2$, $CF_3$, $OCF_3$, $(C_1-C_6)$-alkyle, $(C_3-C_8)$-cycloalkyle, phényle ;
R4, R5 signifient H, F, Cl, Br, OH, $NO_2$, $CF_3$, $OCF_3$, $OCF_2H$, $OCF_2$-$CF_3$, $OCF_2$-$CHF_2$, $SCF_3$, O-phényle, $(C_1-C_6)$-alkyle, O- $(C_1-C_6)$-alkyle, O- $(C_1-C_6)$ -alkyl-O- $(C_1-C_3)$-alkyle ;
n signifie 1 à 3 ;
et

R2 signifie ($C_1$-$C_8$)-alkyle, un ou plusieurs groupes $CH_2$ dans les groupes alkyle pouvant être remplacés par 0, CO, S, SO ou $SO_2$, et alkyle pouvant être monosubstitué à trisubstitué par F, Cl, Br, $CF_3$, CN, $NO_2$, NHAc, NHBoc, NH-CO-C($CH_3$)$_3$, hydroxyle, $OCF_3$, O- ($C_1$-$C_6$)-alkyle, COOH, CO-benzoxy, CO-O($C_1$-$C_6$)-alkyle, tétrazole, thiazolidine-2,4-dione, indole et ($C_6$-$C_{10}$)-aryle, où thiazolidine-2,4-dione et aryle peuvent à nouveau être substitués par F, Cl, Br, $CF_3$, CN, $NO_2$, NHAc, NHTs, NHBoc, NHCbz, NH-CO-C($CH_3$)$_3$, hydroxyle, $OCF_3$, O-($C_1$-$C_6$)-alkyle, COOH, CO-benzoxy, CO-O ($C_1$-$C_6$) -alkyle, ($C_1$-$C_6$) -alkyle, 0- ($C_1$-$C_6$)-alkyle ou tétrazole ; ou
R2 représente un groupe de protection de la fonction OH (GP), tel que par exemple un groupe benzyloxyméthyle, benzyle, para-méthoxybenzyle ou tert-butyldiméthylsilyle ;
**caractérisé en ce que**
A)

a) alkylation (alk-R2 / alk-GP)
on transforme du cis-1,3-cyclohexanediol de formule (II)

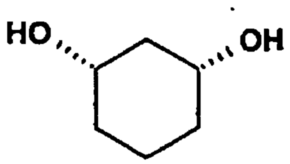

(II)

avec un composé de formule (III)

$X^1$-R2 (III)

dans laquelle R2 est défini comme ci-dessus et
$X^1$ représente Cl, Br, I, OMs, OTs, OTf ;
en présence de bases dans un solvant approprié en un composé racémique de formule (IV),

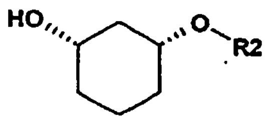

(IV)

dans laquelle R2 est défini comme ci-dessus ;
b1) formation enzymatique d'un ester (FE) et séparation (S)
on soumet le composé obtenu de formule (IV) à une formation d'ester (FE) enzymatique stéréosélective, les alcools étant mélangés dans un solvant organique avec un donneur d'acyle et l'enzyme et le mélange obtenu étant agité à -20 à 80°C et, après la réaction, un stéréo-isomère se trouve sous forme d'ester de formule (V)

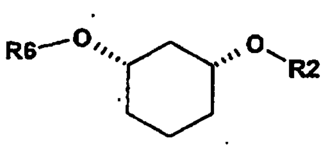

(V)

dans laquelle
R6 signifie C (=O)-($C_1$-$C_{16}$)-alkyle, C (=O)-($C_2$-$C_{16}$)-alcényle, C (=O)-($C_3$-$C_{16}$)-alcynyle, C (=O)-($C_3$-$C_{16}$)-cycloalkyle, où un ou plusieurs atomes de carbone peuvent être replacés par des atomes d'oxygène et peuvent être substitués par 1 à 3 substituants du groupe F, Cl, Br, $CF_3$, CN, $NO_2$, hydroxy, méthoxy, éthoxy, phényle et CO-O ($C_1$-$C_4$)-alkyle, CO-O($C_2$-$C_4$)-alcényle, qui peuvent à leur tour être substitués par 1 à 3 substituants du groupe F, Cl, Br, $CF_3$, et
R2 est défini comme ci-dessus,
et l'autre stéréo-isomère se trouve sous forme inchangée sous forme de l'alcool de formule (IV) et ils sont dès lors séparés l'un de l'autre en exploitant leurs propriétés chimiques ou physico-chimiques différentes (séparation S)

ou

b2) dissociation enzymatique de l'ester [= estérification chimique (EC) + dissociation enzymatique (DE)] + séparation (S)

on soumet le composé obtenu de formule (IV) à une dissociation d'ester enzymatique stéréosélective, l'alcool racémique étant d'abord transformé par estérification chimique (EC), par exemple au moyen du chlorure d'acide R6-C1 ou de l'anhydride d'acide R6-OR6 en présence de bases en ester racémique de formule (V)

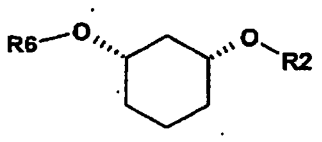

(V)

dans laquelle R6 et R2 sont définis comme ci-dessus, qui est alors repris, pour réaliser la dissociation d'ester (DE) enzymatique stéréosélective, dans un milieu homogène ou hétérogène, aqueux, aqueux-organique ou organique et on le fait réagir en présence d'une enzyme, dans le cas d'une hydrolyse avec de l'eau et dans le cas d'une alcoolyse avec un alcool, à une température de 10 à 80°C et après la réaction, un stéréo-isomère se trouve sous forme d'alcool de formule (IV) et l'autre se trouve sous forme inchangée sous forme d'ester de formule (V) qui peuvent être séparés l'un de l'autre comme décrit au point b1), où

l'énantiomère produit sous forme d'alcool de formule (IV) étant transformé davantage comme décrit ci-dessous au point d), ou

c) hydrolyse chimique (HC)

on saponifie l'énantiomère produit sous forme d'ester de formule (V) en alcool chimiquement énantiomère selon des procédés connus et

d) alkylation (a1k-R1)

on transforme ensuite avec un composé de formule (VI)

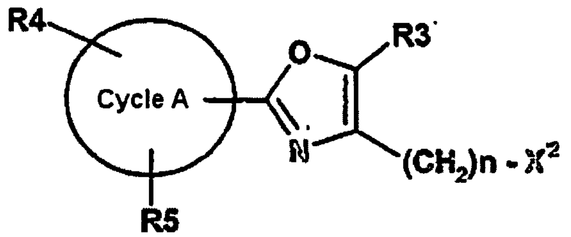

(VI)

dans laquelle

le cycle A, R3, R4, R5 et n sont définis comme ci-dessus et

$X^2$ signifie Cl, Br, I, OTs, OMs, OTf ;

en présence de bases dans un solvant approprié en composé de formule (I), et

e) dissociation du groupe de protection GP (diGP)

lorsque R2 représente un groupe de protection de la fonction OH (GP) tel que défini ci-dessus pour R2, on transforme le composé de formule (Ia)

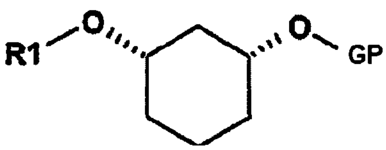

(Ia)

dans laquelle R1 et GP sont définis comme ci-dessus,

par dissociation du groupe de protection selon des procédés connus en un composé de formule (VII)

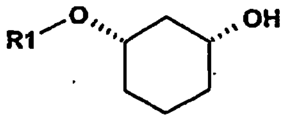

(VII)

dans laquelle R1 est défini comme ci-dessus,
f) alkylation (alk-R2)
qui est ensuite transformé avec un composé de formule (III),

$X^1$-R2 (III)

dans laquelle $X^1$ et R2 sont tels que définis ci-dessus en présence de bases dans un solvant approprié en un composé de formule (I), le produit ou la forme énantiomère,
où il est également possible de modifier l'ordre des différentes étapes de réaction tel que décrit ci-dessus au point A) :

A) alk-R2 → FE+S / EC+SE+S [→ CH] → alk-R1 [→ DiGP → alk-R2] → produit/forme énantiomère en
B) alk-R1 → FE+S / EC+SE+S [→ CH] → alk-R2 [→ DiGP → alk-R2] → produit/forme énantiomère
ou
C) alk-GP → FE+S / EC+SE+S → CH → alk-R2 → DiGP → alk-R1 → produit/forme énantiomère
ou
D) alk-SG → FE+S / EC+SE+S → alk-R1 → DiGP → alk-R2 → produit/forme énantiomère.

**2.** Procédé selon la revendication 1, **caractérisé en ce qu'**on utilise les procédés C) et D).

**3.** Procédé selon les revendications 1 ou 2, **caractérisé en ce qu'**on utilise des composés de formule (III)

$X^1$-R2 (III)

dans laquelle
$X^1$ signifie Cl, Br, I, OMs ou OTf.

**4.** Procédé selon les revendications 1 à 3, **caractérisé en ce qu'**on utilise des composés de formule (III)

$X^1$- R2 (III)

dans laquelle
$X^1$ signifie Cl, Br ou I.

**5.** Procédé selon les revendications 1 à 4, **caractérisé en ce qu'**on prépare un composé de formule (I)

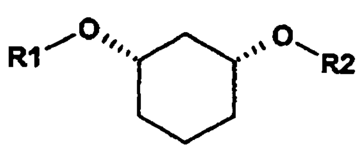

(I) .

dans laquelle :
R1 représente

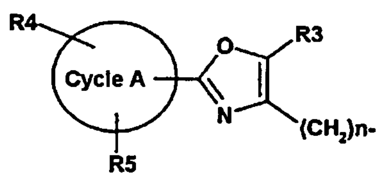

avec
le cycle A représentant phényle, un cycle hétéroaromatique de 5 à 12 chaînons, qui peut contenir un ou plusieurs hétéroatomes du groupe N, O ou S, un cycle aromatique annelé/bicyclique de 8 à 14 chaînons, $(C_3\text{-}C_8)$ -cycloalkyle ;
R3 représentant H, $CF_3$, $(C_1\text{-}C_6)$-alkyle, $(C_3\text{-}C_8)$-cycloalkyle, phényle ;
R4, R5 signifiant H, F, Br, $CF_3$, $OCF_3$, $(C_1\text{-}C_6)$-alkyle, 0-$(C_1\text{-}C_6)$-alkyle ;
n signifiant 1 à 2 et
R2 signifiant $(C_1\text{-}C_8)$-alkyle, un ou plusieurs groupes $CH_2$ dans les groupes alkyle pouvant être remplacés par O, CO, S, SO ou $SO_2$, et alkyle pouvant être monosubstitué à trisubstitué par F, Cl, Br, $CF_3$, CN, $NO_2$, NHAc, NHBoc, NH-CO-$C(CH_3)_3$, hydroxyle, $OCF_3$, O-$(C_1\text{-}C_6)$ -alkyle, COOH, CO-benzoxy, CO-O$(C_1\text{-}C_6)$ -alkyle, tétrazole, thiazolidine-2,4-dione, indole et $(C_6\text{-}C_{10})$-aryle, où thiazolidine-2,4-dione et aryle peuvent à nouveau être substitués par F, Cl, Br, $CF_3$, CN, $NO_2$, NHAc, NHTs, NHBoc, NHCbz, NH-CO-$C(CH_3)_3$, hydroxyle, $OCF_3$, O-$(C_1\text{-}C_6)$-alkyle, COOH, CO-benzoxy, CO-O$(C_1\text{-}C_6)$-alkyle, $(C_1\text{-}C_6)$-alkyle, O-$(C_1\text{-}C_6)$-alkyle ou tétrazole.

**6.** Procédé selon les revendications 1 à 5, **caractérisé en ce qu'**on prépare un composé de formule (I)

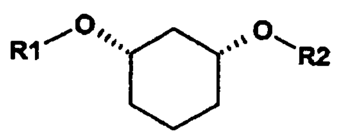

(I) .

dans laquelle :
R1 représente

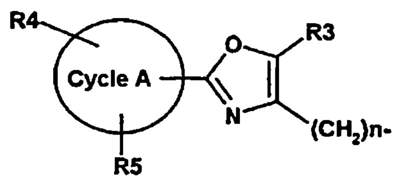

avec
le cycle A signifiant phényle ;
R3 signifiant $(C_1\text{-}C_4)$-alkyle ;
R4, R5 signifiant H, $(C_1\text{-}C_4)$-alkyle, O-$(C_1\text{-}C_4)$-alkyle; n signifiant 1 et
R2 signifiant $(C_1\text{-}C_8)$-alkyle, un ou plusieurs groupes $CH_2$ dans les groupes alkyle pouvant être remplacés par O, CO, S, SO ou $SO_2$, et alkyle pouvant monosubstitué à trisubstitué par F, Cl, Br, $CF_3$, CN, $NO_2$, NHAc, NHBoc, NH-CO-$C(CH_3)_3$, hydroxyle, $OCF_3$, O-$(C_1\text{-}C_6)$ -alkyle, COOH, CO-benzoxy, CO-O$(C_1\text{-}C_6)$-alkyle, tétrazole, thiazolidine-2,4-dione, indole et $(C_6\text{-}C_{10})$-aryle, où thiazolidine-2,4-dione et aryle peuvent à nouveau être substitués par F, Cl, Br, $CF_3$, CN, $NO_2$, NHAc, NHTs, NHBoc, NHCbz, NH-CO-C $(CH_3)_3$, hydroxyle, $OCF_3$, 0- $(C_1\text{-}C_6)$ -alkyle, COOH, CO-benzoxy, CO-O$(C_1\text{-}C_6)$ -alkyle, $(C_1_C_6)$ -alkyle, O-$(C_1\text{-}C_6)$-alkyle ou tétrazole.